(19) **Europäisches Patentamt / European Patent Office / Office européen des brevets**

(11) **EP 3 259 288 B1**

(12) **EUROPEAN PATENT SPECIFICATION**

(45) Date of publication and mention
of the grant of the patent:
**22.07.2026 Bulletin 2026/30**

(51) International Patent Classification (IPC):
**C07K 16/28** (2006.01)     **C07K 16/40** (2006.01)
**C07K 16/30** (2006.01)

(21) Application number: **16709299.8**

(22) Date of filing: **19.02.2016**

(52) Cooperative Patent Classification (CPC):
**C07K 16/2896; C07K 16/30; C07K 16/40;**
C07K 2317/24; C07K 2317/33; C07K 2317/34;
C07K 2317/41; C07K 2317/71; C07K 2317/76;
C07K 2317/92

(86) International application number:
**PCT/EP2016/053539**

(87) International publication number:
**WO 2016/131950 (25.08.2016 Gazette 2016/34)**

(54) **CD73 BLOCKADE**

CD73-BLOCKADE

BLOCAGE DE CD73

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.02.2015 US 201562118549 P**
**16.03.2015 US 201562133597 P**
**06.07.2015 US 201562188881 P**
**09.10.2015 PCT/EP2015/073370**
**22.01.2016 US 201662281841 P**

(43) Date of publication of application:
**27.12.2017 Bulletin 2017/52**

(73) Proprietor: **Innate Pharma**
**13009 Marseille (FR)**

(72) Inventors:
• **CHANTEUX, Stéphanie**
**13009 Marseille (FR)**
• **PATUREL, Carine**
**69280 Marcy l'Etoile (FR)**
• **PERROT, Ivan**
**13830 Roquefort la Bedoule (FR)**
• **GAUTHIER, Laurent**
**13008 Marseille (FR)**

(74) Representative: **Cabinet Becker et Associés**
**25, rue Louis le Grand**
**75002 Paris (FR)**

(56) References cited:
**EP-A1- 2 078 732     WO-A1-2016/055609**

• **J. STAGG ET AL: "CD73-Deficient Mice Have
Increased Antitumor Immunity and Are Resistant
to Experimental Metastasis", CANCER
RESEARCH, vol. 71, no. 8, 15 April 2011
(2011-04-15), US, pages 2892 - 2900, XP55252662,
ISSN: 0008-5472, DOI: 10.1158/
0008-5472.CAN-10-4246**
• **ANONYMOUS: "Purified Rat Anti-Mouse CD73
clone TY/23 (RUO)",
WWW.BDBIOSCIENCES.COM, 1 August 2019
(2019-08-01), pages 1 - 3, XP055610489,
Retrieved from the Internet <URL:http://www.
bdbiosciences.com/us/applications/research/
stem-cell-research/
mesenchymal-stem-cell-markers-bone-marrow/
mouse/positive-markers/
purified-rat-anti-mouse-cd73-ty23/p/550738>
[retrieved on 20190801]**

Processed by Luminess, 75001 PARIS (FR)

**(Cont. next page)**

- KURELLA VINODH B ET AL: "Structure guided homology model based design and engineering of mouse antibodies for humanization.", BIOINFORMATION 2014, vol. 10, no. 4, 2014, pages 180 - 186, ISSN: 0973-2063
- SEBASTIAN FM HÄUSLER ET AL: "Anti-CD39 and anti-CD73 antibodies A1 and 7G2 improve targeted therapy in ovarian cancer by blocking adenosine-dependent immune evasion", AM J TRANSL RES, vol. 6, no. 2, 15 January 2014 (2014-01-15), pages 129 - 139, XP055240896
- L. AIRAS: "Differential Regulation and Function of CD73, a Glycosyl-Phosphatidylinositol-linked 70-kD Adhesion Molecule, on Lymphocytes and Endothelial Cells", THE JOURNAL OF CELL BIOLOGY : JCB, vol. 136, no. 2, 27 January 1997 (1997-01-27), US, pages 421 - 431, XP055242069, ISSN: 0021-9525, DOI: 10.1083/jcb.136.2.421
- FLOCKE K ET AL: "MONOCLONAL ANTIBODIES AGAINST 5'-NUCLEOTIDASE FROM A HUMAN PANCREATIC TUMOR CELL LINE: THEIR CHARACTERIZATION AND INHIBITORY CAPACITY ON TUMOR CELL ADHESION TO FIBRONECTIN SUBSTRATUM", EUROPEAN JOURNAL OF CELL BIOLOGY, WISSENSCHAFLICHE VERLAGSGESELLSCHAFT, STUTTGART, DE, vol. 58, no. 1, 1 June 1992 (1992-06-01), pages 62 - 70, XP009033209, ISSN: 0171-9335
- GURD J W ET AL: "Distribution of liver plasma membrane 5' nucleotidase as indicated by its reaction with anti-plasma membrane serum", ARCHIVES OF BIOCHEMISTRY AND BIOPHYSICS, ACADEMIC PRESS, US, vol. 164, no. 1, 1 September 1974 (1974-09-01), pages 305 - 311, XP024757974, ISSN: 0003-9861, [retrieved on 19740901], DOI: 10.1016/0003-9861(74) 90035-6
- JAMES C. GEOGHEGAN ET AL: "Inhibition of CD73 AMP hydrolysis by a therapeutic antibody with a dual, non-competitive mechanism of action", MABS, vol. 8, no. 3, 8 February 2016 (2016-02-08), US, pages 454 - 467, XP055266812, ISSN: 1942-0862, DOI: 10.1080/ 19420862.2016.1143182

Remarks:
- The complete document including Reference Table(s) and the Sequence Listing(s) can be downloaded from the EPO website
- The file contains technical information submitted after the application was filed and not included in this specification

**Description**

**FIELD OF THE INVENTION**

The present invention relates to antibodies that inhibit CD73. The invention also relates to pharmaceutical compositions comprising the same; and uses thereof for the treatment of a cancer.

**BACKGROUND**

CD73 (ecto-5'-nucleotidase) is a 70-kDa glycosylphosphatidylinositol (GPI)-anchored protein normally expressed on endothelial cells and subsets of hematopoietic cells. CD73, together with CD39, regulates adenosine triphosphate (ATP) metabolism. CD39 (NTPDase-1) converts ATP into AMP, with only trace amounts of ADP being released, while CD73 catalyzes the conversion of AMP to adenosine.

Adenosine triphosphate (ATP) and its metabolites AMP and adenosine, have important roles in cellular metabolism, signalling and immune homeostasis. The release of extracellular adenosine triphosphates (ATP) in response to cell death or cellular stress acts to activate immune responses. However, its metabolite adenosine has immunosuppressive activity. Extracellular adenosine accumulates in cancerous tissues and constitutes an important mechanism of tumor immune escape. Among other effects, tumor-derived adenosine profoundly inhibits infiltrating effector T cells through adenylyl cyclase-activating A2A receptors.

CD73 expression has been reported in a range of tumor cells, including leukemia, bladder cancer, glioma, glioblastoma, ovarian cancer, melanoma, prostate cancer, thyroid cancer, esophageal cancer and breast cancer. CD73 expression has also been associated with a prometastatic phenotype in melanoma and breast cancer. It has been shown that therapy with an antibody that binds murine CD73 can inhibit breast tumor growth and metastasis in mice (Stagg, et al. (2010) Proc. Natl. Acad. Sci. USA 104:1547-1552). Antibodies however generally do not cross react with human and mouse CD73, complicating the study of the antibodies and the biological functions of CD73. It has been shown that genetic deletion of A2A receptors can induce T cell-dependent tumor rejection (Ohta, et al., (2006) Proc Natl Acad Sci USA 103:13132-13137). Knock-down using siRNA or overexpression of CD73 on tumor cells can modulate tumor growth and metastasis (Beavis et al (2013 Proc. Natl. Acad. Sci. USA 110:14711-716; Stagg et al. (2010), supra; Jin et al. (2010) Cancer Res. 70: 2245-55). CD73-/- mice are protected from transplanted and spontaneous tumors (Stagg et al. (2010) Cancer Res. 71: 2892-2900). In humans, high CD73 expression had been shown to be a negative prognostic for triple negative breast cancer (Loi et al (2013 Proc. Natl. Acad. Sci. USA 110: 11091-11096).

Häusler et al (2014, Am L Transl Res, 6, 129-139), Airas (1997, Journal of Cell Biology, 136, 421-431), and Flocke et al (1992, European Journal of Cell Biology, 58, 62-70) disclose anti-CD73 antibodies inhibiting 5'-ectonucleotidase activity.

Despite the long-standing interest in CD73 as a therapeutic target, the activity required of an agent to target CD73 in vivo has not been fully elucidated. While CD73 is expressed on tumor cells, it is also expressed on different cells of the immune system, notably CD4 and CD8 T cells, as well as B cells. While some antibodies have been reported to bind human CD73 and increase the activity or proliferation of T cells or modify the migration of tumor cells, it remains to be clarified how such antibodies function since such T cell modulation and CD73-mediated transmission of co-stimulatory signals have been reported to be possible without dependence on the ecto-5'nucleotidase activity of CD73 (Gutensohn et al. 1995 Cell Immunol. 161:213-217). Consequently, antibodies generically referred to as CD73 inhibitors may not act by modulating the ecto-5'nucleotidase activity of CD73. One antibody, 7G2 (mIgG2 isotype, Life Technologies), has been reported to inhibit CD73, however this antibody does not bind cell surface CD73 in flow cytometry, or at best only with very low affinity. Another antibody that binds CD73, clone AD2 (mouse IgG1 isotype), has been reported to cause receptor clustering and internalization but have minimal effect on enzymatic activity. Yet another agent, 1E9 (mouse IgG3 isotype, Santa Cruz Biotechnology, Inc.), is reported to promote T cell signaling independently of enzymatic inhibition. A further mAb, 4G4 (IgG1 isotype, Novus Biologicals), is reported to induce CD73 shedding from the T cell surface. Only one agent, although not further characterized, was reported to have partial ability to block enzymatic in an assay using recombinant CD73 (Sachsenmeier et al. ((2012) J. Biomed. Screening 17:993-998), and was later described as an antibody that induces intracellular internalization (Rust et al. (2013) Mol. Cancer 12:11). Additionally, one further complicating factor is that the antibodies described in the literature have generally been of murine isotypes that are capable of being bound by Fcγ receptors, making it difficult to separate any potential blocking effect from Fc-mediated effects. Anti-CD73 antibodies that are bound by Fcγ receptors can for example mediate depletion (e.g. by ADCC) of CD73-expressing tumor cells (and possibly CD73-expressing immune suppressor cells), and/or may elicit the production of pro-inflammatory cytokines rather than any true blocking effect. Consequently, the mode of action of antibodies remains elusive.

Thus, despite the interest in targeting CD73, the characteristics of the most effective anti-CD73 antibodies remains to be determined. No antibodies have been reported that bind the CD73 active site. CD73 expression on different cell types, including immune cells and tumor cells, combined with use of antibodies that either do not actually block CD73 or are not pure blockers, create a complex setting for evaluation of the underlying activity of antibodies. New assays and antibodies are needed.

## SUMMARY OF THE INVENTION

The invention is as defined in claims 1-12.

## BRIEF DESCRIPTION OF THE DRAWINGS

Figure 1 shows ability of anti-CD73 antibodies to block enzymatic activity of CD73, assessed by measuring ability of test mAbs to affect CD73's ability to cleaves AMP into adenosine + inorganic phosphate that restores luciferase activity and light emission. Results are expressed as residual enzyme activity (%). Antibodies 11E1, 8C7, 6E1 and 3C12 (not shown) cause a strong decrease in enzyme activity, and continue to reduce residual enzyme activity even when provided at excess, an immune-complex-independent setting.

Figure 2 shows the results of titration of antibodies by ELISA on soluble recombinant human CD73 polypeptide.

Figure 3 shows results of titration of antibodies by flow cytometry on human-, cynomolgus- and mouse-CD73-expressing recombinant host cell lines. 11E1, 8C7, 3C12 and 6E1, but not 7G2 or 1E9, bind to recombinant host cells expressing human and cynomolgus (but not mouse) CD73 with excellent affinity.

Figure 4 shows results of titration of antibodies by flow cytometry on human MDA-MB-231 breast adenocarcinoma cells that endogenously expresses CD73. 11E1, 8C7, 3C12 and 6E1, but not 7G2 or 1E9, bind to MDA-MB-231 cells with excellent affinity.

Figure 5 shows antibodies 11E1, 8C7, 3C12 and 6E1 neutralize the enzymatic activity of cellular CD73.

Figure 6 shows the ability of various antibodies to cause down-modulation of CD73 expression on cells. Each of AD2, 7G2 and 1E9 caused down-modulation of CD73, however none of antibodies 11E1, 8C7, 3C12, or 6E1 caused a decrease in cell surface CD73.

Figure 7 shows titration of antibodies by flow cytometry on cell expressing mutants of human CD73. Antibody 3C12 binds to wild type CD73 and mutant 2 but not to mutant 3, while antibody AD2 binds to wild type CD73 and mutant 3, but not to mutant 2.

Figure 8A shows the molecular structure of the CD73 dimer, with amino acids mutated in mutant 2 (loss of binding by AD2) indicated (white circles) in both "open" or "closed" configurations. Figure 8B shows the molecular structure of the CD73 dimer, with amino acids mutated in mutant 3 (loss of binding by 11E1, 8C7, 3C12 or 6E1) indicated in both "open" or "closed" configurations. The active site is indicated by the box (dashed lines).

Figure 9 shows the molecular structure of the CD73 dimer, with amino acids mutated in mutant 16 (loss of binding by 11E1, 3C12 and 6E1) indicated in both "open" or "closed" configurations.

Figure 10 shows the molecular structure of the CD73 dimerin open configuration, showing (circled) the amino acids mutated in mutants 3 and its surroundings (residues substituted in mutants 16, 21 and 22) which resulted in loss of binding by antibodies.

## DETAILED DESCRIPTION OF THE INVENTION

The inventors have discovered antibodies that bind an epitope present on CD73 expressed at the surface of cells, including tumor cells, and that inhibit the enzymatic (ecto-5' nucleotidase) activity of the CD73 enzyme. The antibodies can inhibit the enzymatic activity of membrane-bound CD73 protein expressed at the surface of cells. Advantageously, these antibodies can be used as pure CD73 blocking antibodies, e.g., they inhibit the enzymatic activity of membrane-bound CD73 protein expressed at the surface of cells without substantially binding Fcγ receptors and/or without substantially directing ADCC toward a CD73-expressing cell. Optionally, the antibodies retain an Fc domain and retain binding to human FcRn.

Optionally, in contrast to some antibodies capable of depleting CD73-expressing tumor cells (which, e.g., can provide full efficacy at concentrations equal or substantially lower than that which provides receptor saturation), the antibodies can advantageously be used as pure blockers and administered in an amount effective to neutralize the enzymatic activity of CD73 for a desired period of time, e.g. 1 week, 2 weeks, a month, until the next successive administration of anti-CD73 antibody.

Optionally, the antibodies are pure blockers and directed to neutralize the enzymatic activity of CD73 in the tumor environment. Optionally, the antibodies comprise a modified Fc domain, e.g. to decrease protease sensitivity (e.g. toward proteases such as MMPs in the tumor environment) and/or to decrease binding to human Fcγ receptors (e.g., CD16).

The disclosure provides assays that can be used to identify true CD73 function blocking antibodies. As shown herein, in previous soluble enzyme blocking assays, the vast majority of antibodies found to block as bivalent antibodies are false positives, while monovalent binding antibodies may have no or little blocking activity, possibly due the inability to block the active site or act as allosteric inhibitors when not binding to each CD73 polypeptide within the CD73 dimer. Previous cellular assays could not distinguish between mechanisms of action, and the antibodies reported to decrease CD73 activity may combine multiple mechanisms of action, for example induction of receptor internalization, receptor shedding and/or Fcγ receptor-mediated effects. Because residual CD73 enzymatic activity can result in sufficient adenosine generation to mediate immunosuppressive effects, high levels of antibody-mediated enzyme blockade are advantageous in order to mediate a therapeutic effect.

The disclosure provides antibodies that bind an epitope present on human CD73 polypeptide expressed at the surface of cells, including but limited to tumor cells, and that inhibit the enzymatic (ecto-5' nucleotidase) activity of the CD73 enzyme.

The disclosure provides antibodies that can inhibit the enzymatic activity of soluble recombinant CD73 protein.

The antibodies of the disclosure do not cause intracellular internalization of, or more generally down-modulation of, cell surface-expressed CD73 and/or do not depend thereupon for their CD73 inhibitory activity. The antibodies of the disclosure can provide greater inhibitory potency (the ability to substantially neutralize CD73 enzymatic activity) than antibodies that inhibit CD73 by causing CD73 internalization. As opposed to antibodies that inhibit soluble CD73 by other mechanisms (e.g. causing CD73-antibody oligomer formation), the antibodies of the disclosure are capable of inhibiting the enzymatic activity of CD73 at all concentrations, including at higher (e.g. 10 fold) excess of antibody:enzyme. Furthermore, unlike antibodies that bind an epitope on recombinant CD73 that may be modified or absent on cell surface CD73 (e.g. antibody 7G2) or with affinity that is too low to translate into efficacy in CD73-expressing cells, the present antibodies bind with high affinity to an epitope that is present and/or remains intact on cell surface CD73, providing the antibodies with the ability to potently neutralize of the enzymatic activity of cellular CD73. The present antibodies inhibit CD73 enzymatic activity in cells but can optionally also inhibit the ecto-5'nucleotidase activity of soluble recombinant CD73 (as observed in a cell-free assay using soluble dimeric CD73 polypeptide).

Furthermore, exemplary antibodies (see, e.g., antibodies 11E1, 6E1, 3C12 and 8C7) are disclosed herein which are believe to be capable of acting as allosteric inhibitors of CD73 expressed by cells, e.g. they inhibit the activity of the human CD73 polypeptide without binding to the enzymatic active site of the CD73 polypeptide, and/or that they are non-competitive inhibitors of CD73, e.g., they inhibit the activity of the human CD73 polypeptide without detectably reducing binding between the CD73 polypeptide and a natural substrate thereof. The exemplary antibodies lose binding to CD73 mutants having a substitution at residue K136. The exemplary antibodies lose binding to CD73 mutants having a substitution at residues K136, and also lose binding to mutants having substitutions at residues A99, E129, K133, E134 and A135, as well as to mutants having a substitution at residues K97, E125, Q153 and K330. In view of binding of the exemplary antibodies to CD73 both in the presence and absence of the CD73 active site inhibitor APCP, their epitope on CD73 appears to be present on CD73 not only in the "open" conformation when not bound to substrate but also in the "closed" conformation when bound to a substrate (e.g. a natural substrate such as AMP or an inhibitor or other compound that binds the active site such as an AMP analogue adenosine 5'-(α,β-methylene)diphosphate (APCP)).

Accordingly, the disclosure provides an allosteric inhibitor of the CD73 polypeptide. In one aspect, the allosteric inhibitor is an antibody. The disclosure provides an antibody that binds human CD73 polypeptide expressed at the surface of a cell, including but limited to tumor cells, and that inhibit the enzymatic (ecto-5' nucleotidase) activity CD73 polypeptide, wherein the antibody is an allosteric inhibitor of the CD73 polypeptide.

Moreover, exemplary antibodies are described herein that bind to an epitope on CD73 that is present on the same face when CD73 is present as a CD73 dimer, e.g., potentially permitting an antibody to bind bivalently to one CD73 dimer, notably in "closed" position where the binding sites are spatially further apart. In view of binding to ligand-bound CD73, the antibodies described herein may be useful for binding to CD73 when bound to AMP, e.g., in the tumor environment where upstream ADP and/or AMP are present at significant levels prior to treatment). The tumor microenvironment can be characterized by any appropriate parameter, for example high levels of ADP (e.g. generated by dying cells), taken up by CD39 on stromal and cellular infiltrate (e.g. TReg cells) to yield high levels of AMP, as well as more generally by AMP, adenosine, by presence or levels of CD39 expression or CD39-expressing cells, by presence or levels of CD73 expression or CD73-expressing cells, by presence or levels of adenosine receptor expression or adenosine-receptor expressing cells. Thus, CD73 molecules in the tumor environment may be in the substrate-bound conformation, and the ability to bind and inhibit substrate-bound cellular CD73 (e.g. cells expressing CD73 pre-incubated with substrate such as AMP) in addition to non-substrate bound CD73 may provide greater ability to inhibit CD73 in vivo. Optionally, levels of ADP or AMP (and/or ATP or adenosine) can be assessed in the tumor environment prior to treatment. The antibodies may have a particular advantage for treatment in an individual having significant levels (e.g. high levels, compared to a reference) ADP, AMP, ATP or adenosine in the tumor sample.

Accordingly, in one aspect, the disclosure provides an antibody that binds human CD73 polypeptide expressed at the surface of cells and that inhibits the enzymatic (ecto-5' nucleotidase) activity of the CD73 polypeptide, wherein the antibody is capable of binding bivalently to a single CD73 polypeptide dimer (a soluble CD73 polypeptide dimer or a CD73 polypeptide dimer expressed by a cell). Optionally, the antibody binds with a first antigen binding domain to a first CD73 polypeptide within the dimer and with a second antigen binding domain to a second CD73 polypeptide. In one aspect the antibody is an allosteric inhibitor of the CD73 polypeptide.

Accordingly, in another aspect, the disclosure provides an antibody that binds human CD73 polypeptide expressed at the surface of cells and that inhibits the enzymatic (ecto-5' nucleotidase) activity of the CD73 polypeptide, wherein the antibody is capable of binding the CD73 polypeptide in the substrate-bound conformation.

Despite efforts in the literature to screen for CD73-inhibition antibodies, existing antibodies do not neutralize cellular CD73, or at best cause CD73 down-modulation. The inventors provide herein an explanation why these antibodies no longer inhibit CD73 in cells: antibodies that are capable of binding CD73 homodimers in bivalent manner and that inhibit recombinant CD73 in solution may be causing oligomerization of the CD73 polypeptides and anti-CD73 antibodies into complexes (e.g., structures containing more than two or more antibodies and two or more CD73 dimers), presenting difficulties to distinguish an true inhibitor from a false positive.

Through design of improved assay methods conducted at higher excess of antibody:enzyme, we present herein antibodies with bivalent binding to CD73 that have no dependence upon oligomerization. In particular, the anti-CD73 antibodies provided herein are capable of inhibiting the enzymatic activity of soluble human dimeric CD73 polypeptide when the antibodies are in a setting/configuration where they not capable of forming oligomers, e.g. when they are provided at a substantial molar excess (e.g. at least 10-fold, 20-fold, 100-fold, etc.) to the CD73 polypeptide dimers. Antibodies that function by causing oligomerization fail to inhibit CD73 when the antibodies provided at a substantial molar excess to the CD73 polypeptide dimers. The antibodies furthermore bind an epitope on CD73 that is maintained when CD73 is expressed at the cell surface. Through use of this assay, antibodies can also be identified that bind bivalently to a single CD73 dimer; such antibodies may have improved CD73-binding and CD73 blocking activity in vitro and vivo in CD73-expressing cells. The antibodies identified by these methods were then tested in cellular enzymatic activity assays using purified antibody, and found to neutralize the enzymatic activity of cellular CD73. Antibodies that inhibit CD73 by inducing internalization or that lose significant binding to cellular CD73 were less potent and were not able to neutralize enzymatic activity, providing at best only partial inhibition of the enzymatic activity of CD73 in cells.

The epitope on CD73 bound by these antibodies is present on CD73 polypeptides as expressed by a range of cells, e.g. cancer cells, CD4 T cells, CD8 T cells, B cells, transfected cells, and binds with high affinity as determined by flow cytometry. For example, an antibody can be characterized by an $EC_{50}$, as determined by flow cytometry, of no more than 5 $\mu$g/ml, optionally no more than 2 $\mu$g/ml, no more than 1 $\mu$g/ml, no more than 0.5 $\mu$g/ml, no more than 0.1 $\mu$g/ml or no more than 0.05 $\mu$g/ml, for binding to cells that express at their surface a CD73 polypeptide. In one aspect, the cells are cells that are made to express CD73 at their surface. In one aspect, the cells are cells that endogenously express CD73 at their surface, e.g. cancer cells, leukemia cells, bladder cancer cells, glioma cells, glioblastoma cells, ovarian cancer cells, melanoma cells, prostate cancer cells, thyroid cancer cells, esophageal cancer cells or breast cancer cells.

In one aspect, the CD73 neutralizing antibodies can be characterized by being capable of causing a decrease in cells' 5'-ectonucletidase activity of CD73 by at least 60%, 75% or 80%. In one aspect, the CD73-neutralizing antibodies can be characterized by an $EC_{50}$ for inhibition of 5'-ectonucletidase activity of CD73 expressed by a cell of no more than 1 $\mu$g/ml, optionally no more than 0.5 $\mu$g/ml, optionally no more than 0.2 $\mu$g/ml.

Inhibition of 5'-ectonucletidase activity of CD73 expressed by a cell is determined by assessing neutralization of 5' ectonucleotidase activity in MDA-MB-231 cells by quantifying hydrolysis of AMP to adenosine (see, e.g., Example 5).

The epitope on CD73 bound by the neutralizing antibodies disclosed herein does not result in the down-modulation of CD73 expression on cells (and, e.g., does not cause clustering and internalization of the antibody-CD73 complex), including when full length antibodies are used that bind CD73 in bivalent manner. The anti-CD73 antibody thus remains bound, together with CD73, at the cell surface. In view of the broad tissue expression of CD73, antibodies that do not trigger CD73 down-modulation and/or internalization may provide improved pharmacological properties and greater amounts of antibody in the tumor microenvironment.

In one aspect, provided is an isolated antibody that specifically binds human CD73 (e.g. a polypeptide comprising the amino acid sequence of SEQ ID NOS: 1 or 2) and which neutralizes the 5'-ectonucleotidase activity of a homodimeric human CD73 polypeptide in solution. In one aspect, provided is an antibody that binds and inhibits the enzymatic activity of a soluble human CD73 polypeptide, notably an antibody that neutralizes the CD73-mediated catabolism of AMP to adenosine. In one aspect, the antibody binds CD73 in bivalent manner. In one aspect, the antibody is a non-depleting antibody e.g., an Fc silent antibody. In one aspect, the antibody neutralizes CD73 in solution without reliance on induction of CD73 polypeptide : anti-CD73 antibody oligomers.

In one aspect, provided is an isolated antibody that specifically binds human CD73 at the surface of a cell and that is capable of neutralizing the 5'-ectonucletidase activity of a soluble human CD73 polypeptide. In one aspect, the antibody does not induce the oligomerization of the soluble CD73.

Provided is an isolated antibody that specifically binds human CD73 at the surface of a cell and that is capable of neutralizing the 5'-ectonucletidase activity of cellular CD73 (CD73 expressed by cells). In one aspect, provided is an isolated antibody that specifically binds and neutralizing the 5'-ectonucletidase activity of a human CD73 at the surface of a cell and that is not internalized into CD73-expressing cells upon binding to CD73. The antibody does not cause multimerization and subsequence internalization of CD73. In one aspect, provided is an antibody that binds and is capable of inhibiting the enzymatic activity of a recombinant human CD73 polypeptide in solution, wherein said antibody is not internalized into CD73-expressing cells. In one aspect, the non-internalizing antibody binds CD73 in bivalent manner. In one aspect, the antibody is a non-depleting antibody, e.g., an Fc silent antibody. The antibody is capable of neutralizing the 5'-ectonucleotidase activity of a dimeric human CD73 polypeptide in solution, moreover without reliance on induction of CD73 polypeptides : anti-CD73 antibodies oligomers.

In one aspect, provided is an antibody that specifically binds bivalently to human CD73 polypeptides and inhibits the enzymatic activity of cellular human CD73 (and optionally further recombinant soluble human CD73), wherein said antibody is not internalized into CD73-expressing cells. Preferably, the antibody substantially lacks Fcy receptor binding (e.g. via its Fc domain).

In one aspect, provided is an isolated antibody that specifically binds human CD73 at the surface of a cell pre-incubated with AMP, and that is capable of neutralizing the 5'-ectonucletidase activity thereof. Neutralizing the 5'-ectonucletidase activity is determined by assessing neutralization of 5' ectonucleotidase activity in MDA-MB-231 cells by quantifying hydrolysis of AMP to adenosine (see, e.g., Example 5).

In any of the aspects herein, the antibody can be characterized by being capable of binding a human CD73 polypeptide whose active site is occupied by a substrate, e.g. AMP, APCP. In any of the aspects herein, the antibody can be characterized by being capable of inhibiting the 5'-ectonucletidase activity of a cell expressing CD73 when such cell has been pre-incubated with AMP. The invention also results, *inter alia,* from the discovery of an epitope on human CD73 that permits highly-effective targeting for neutralizing CD73 activity in cells and individuals suffering from cancer. The antibodies compete for one another for binding to CD73, (but did not compete with reference soluble CD73 blocking antibodies) suggesting a region on CD73 which is particularly suitable for inhibition of enzymatic activity of CD73 and that remains present on the CD73 polypeptide when expressed at the cell surface. Advantageously, the epitope is present on each of human and non-human primate CD73, as expressed on the cell surface, as well as on CD73 as expressed by cancer cells.

In one aspect, provided is an anti-CD73 antibody that binds a common antigenic determinant present on both soluble CD73 and CD73 expressed at the cell surface.

In one aspect, provided is an anti-CD73 antibody that binds a common antigenic determinant present on CD73 when it is "open" conformation (when CD73 active site is not occupied by/bound to a substrate, e.g. AMP, APCP) and "closed" CD73 when it is "closed" conformation (when CD73 active site is occupied by/bound to a substrate, e.g. AMP, APCP).

In one aspect, provided is an anti-CD73 antibody that binds an antigenic determinant within each CD73 polypeptide chain within a CD73 dimer, e.g., wherein the antigenic determinants are present on a common face of the CD73 dimer.

In one aspect, provided is an anti-CD73 antibody that binds that bind an epitope on CD73 comprising residue K136 (with reference to SEQ ID NO: 1).

In one aspect, provided is an anti-CD73 antibody that binds that bind an epitope on CD73 comprising one, two, three or four of the residues selected from the group consisting of K97, E125, Q153 and K330 (with reference to SEQ ID NO: 1).

In one aspect, provided is an anti-CD73 antibody that binds that bind an epitope on CD73 comprising one, two, three, four or five of the residues selected from the group consisting of A99, E129, K133, E134, and A135 (with reference to SEQ ID NO: 1).

In one aspect, provided is an anti-CD73 antibody that binds at least partly within a domain or segment of amino acid residues on a human CD73 protein (e.g. a CD73 homodimer protein) comprising the amino acid residues K97, A99, E125, E129, K133, E134, A135, K136, Q153 and K330 (with reference to SEQ ID NO: 1). In one aspect, provided is an anti-CD73 antibody that binds that bind an epitope on CD73 comprising at least one, two, three, four or five, or more, of the residues selected from the group consisting of K97, A99, E125, E129, K133, E134, A135, K136, Q153 and K330 (with reference to SEQ ID NO: 1).

In one aspect, provided is an anti-CD73 antibody that has reduced binding to a CD73 polypeptide having a mutation at a residue K136 (with reference to SEQ ID NO: 1); optionally, the mutant CD73 polypeptide has the mutation: K136A.

In one aspect, provided is an anti-CD73 antibody that has reduced binding to a CD73 polypeptide having a mutation at a residue selected from the group consisting of: K97, E125, Q153 and K330 (with reference to SEQ ID NO: 1); optionally, the mutant CD73 polypeptide has the mutations: K97A, E125A, Q153A and/or K330A (e.g., K97A, E125A and K330A; K97A, E125A and/or Q153A).

In one aspect, provided is an anti-CD73 antibody that has reduced binding to a CD73 polypeptide having a mutation at a residue selected from the group consisting of: A99, E129, K133, E134, and A135 (with reference to SEQ ID NO: 1); optionally, the mutant CD73 polypeptide has the mutations: A99S, E129A, K133A, E134N, and A135S.

Provided in one aspect provided is an anti-CD73 antibody that competes for binding to an epitope on CD73 bound by 11E1, 8C7, 3C12 and/or 6E1, (e.g., that competes for binding to an epitope on a CD73 polypeptide with an antibody having the heavy and light chain CDRs or variable regions of any of 11E1, 8C7, 3C12 or 6E1).

In one aspect, provided is an antigen-binding compound that binds the same epitope and/or competes for binding to a CD73 polypeptide with monoclonal antibodies 11E1, 8C7, 3C12 and/or 6E1 (e.g., that competes for binding to a CD73 polypeptide with an antibody having the heavy and light chain CDRs or variable regions of any of 11E1, 8C7, 3C12 or 6E1). In one aspect, provided is antigen-binding compound binds the same epitope and/or competes for binding to a CD73 polypeptide with an antibody selected from the group consisting of:

(a) an antibody having respectively a VH and VL region of SEQ ID NOS: 3 and 4 (11E1);
(b) an antibody having respectively a VH and VL region of SEQ ID NOS: 21 and 22 (6E1)
(c) an antibody having respectively a VH and VL region of SEQ ID NOS: 28 and 29 (8C7) ; and
(d) an antibody having respectively a VH and VL region of SEQ ID NOS: 36 and 37 (3C12).

In any aspect herein, an antibody can be an antibody other than antibodies 11E1 and/or 6E1. Optionally, In any aspect herein, antibodies 11E1 and/or 6E1 can be specifically excluded.

In one aspect, an anti-CD73 antibody binds an epitope comprising one, two or three amino acid residues selected from the group consisting of the amino acid residues on CD73 bound by 11E1, 6E1, 3C12 or 8C7. In one aspect, the amino acid residues on CD73 are selected from the group consisting of the residues listed in Table 1.

In one aspect, the antibody may have a heavy and/or light chain having one, two or three CDRs of the respective heavy and/or light chain of an antibody selected from the group consisting of antibody 11E1, 6E1, 3C12 and 8C7.

In any of the aspects herein, the anti-CD73 antibodies can be characterized by binding to human CD73 polypeptides expressed on the surface of a cell (e.g. a tumor cell, a cell made to express CD73, e.g. an MDA-MB-231 tumor cell line, or a recombinant host cell made to express CD73, as shown in the Examples), and optionally further wherein the antibody binds with high affinity as determined by flow cytometry. For example, an antibody can be characterized by an $EC_{50}$, as determined by flow cytometry, of no more than 5 $\mu$g/ml, optionally no more than 1 $\mu$g/ml, no more than 0.5 $\mu$g/ml, no more than 0.1 $\mu$g/ml or no more than 0.05 $\mu$g/ml, for binding to cells that express at their surface a CD73 polypeptide, e.g. tumor cells expressing CD73, cells expressing at their surface a CD73 polypeptide, lymphocytes expressing CD73, etc. Optionally, an antigen-binding compound has an $EC_{50}$ of no more than 1 $\mu$g/ml, optionally no more than 0.5 $\mu$g/ml, no more than 0.1 $\mu$g/ml, or no more than 0.05 $\mu$g/ml for binding to (i) cells expressing at their surface human CD73 (e.g. a polypeptide having the amino acid sequence of SEQ ID NO: 1) and/or (ii) cells expressing at their surface human non-human primate CD73 (e.g. a cynomolgus monkey CD73).

In one aspect, the anti-CD73 antibody is a tetrameric antibody comprising two heavy and two light chains, the heavy chains comprising Fc regions of human isotype and which substantially lack binding to human Fc$\gamma$ receptors (e.g. CD16A, CD16B, CD32A, CD32B and/or CD64).

In one aspect, the antibodies are to be administered to an individual having a cancer in an amount and frequency sufficient to neutralize the activity of CD73 in the tumor microenvironment. In one aspect, the antibodies are to be administered in an amount and frequency sufficient to decrease the generation and/or concentration of adenosine in the tumor microenvironment. In one aspect, the antibodies are to be administered in an amount and frequency sufficient to increase the generation and/or concentration of ATP in the tumor microenvironment. In one aspect, the antibodies are to be administered in an amount and frequency sufficient to neutralize the activity of CD73 expressed by tumor cells. In one aspect, the antibodies are administered in an amount and frequency sufficient to neutralize the activity of CD73 expressed by CD4 T cells, CD8 T cells and/or B cells.

The antibodies will be useful in inhibiting CD73-mediated catabolism of AMP to adenosine, e.g. decreasing the concentration of adenosine in the tumor microenvironment. These antibodies will therefore be useful in reversing the immunosuppressive effect of CD73 and/or adenosine on T cells, B cells and other cells that express adenosine receptors, for example in the treatment of cancer. In one aspect, the anti-CD73 antibody neutralizes adenosine-mediated inhibition of proliferation, cytokine production, cytotoxicity and/or NF$\kappa$B activity in T cells.

Because the CD73-mediated catabolism of AMP to adenosine is irreversible, whereas the catabolism of ATP to ADP and ADP to AMP by CD39 is reversible (by NDK kinase and adenylate kinase, respectively), the antibodies that block the irreversible CD73-mediated catabolism will increase the pool of AMP, thereby being of use in increasing the concentrations of ADP and ATP, e.g.in the tumor microenvironment. The antibodies can be useful to increase the formation of ADP from AMP and the formation of ATP from ADP. Since ATP has immune activating roles, the anti-CD73 antibodies can be useful in activating T cells, for example in the treatment of cancer.

The antibodies will be useful in inhibiting the production, amounts and/or concentrations of adenosine into the tumor microenvironment.

The antibodies that neutralize the activity of a soluble human CD73 polypeptide dimer can further neutralize CD73 in any other suitable context, e.g. in a reporter cell made to express CD73, in a T cell, etc.

Provided is an anti-CD73 antibody described herein for use for treating an individual, wherein the anti-CD73 antibody is to be administered to an individual (e.g. an individual having a disease, a tumor, etc.) in a therapeutically active amount. Preferably the compound is a non-depleting antibody (an antibody that does not deplete cells to which it binds, e.g., an Fc silent antibody). Optionally, the compound binds to CD73 in bivalent manner. Optionally, the antibody comprises a heavy chain constant region of IgG4 isotype.

In one aspect provided is an anti-CD73 antibody that inhibits the enzymatic (ecto-5' nucleotidase) activity of the CD73 polypeptide for use for decreasing adenosine present in the tumor environment (e.g. in an individual). In one aspect, the individual has a cancer.

In one aspect, the active amount of an antigen binding compound that inhibits a CD73 polypeptide is an amount effective to achieve and/or maintain (e.g. until the subsequent administration of antigen binding compound) a blood concentration of at least the $EC_{50}$, optionally the $EC_{70}$, optionally substantially the $EC_{100}$, for inhibition of CD73-mediated catabolism of AMP to adenosine in an individual. In one aspect, the active amount of an antigen binding compound that inhibits a CD73 polypeptide is an amount effective to achieve the $EC_{50}$, optionally the $EC_{70}$, optionally substantially the $EC_{100}$, for inhibition of CD73-mediated catabolism of AMP to adenosine in an extravascular tissue of an individual. In one aspect, the active amount an antigen binding compound that inhibits a CD73 polypeptide is an amount effective to achieve the $EC_{50}$, optionally the $EC_{70}$, optionally substantially the $EC_{100}$, for inhibition of CD73-mediated catabolism of AMP to adenosine in an individual. In one aspect, the active amount of an antigen binding compound that inhibits a CD73 polypeptide is between 1 and 20 mg/kg body weight. In one aspect, the active amount is administered to an individual weekly, every two weeks, monthly or every two months.

Optionally the individual is human having or who is susceptible to having a cancer.

The antibodies are optionally characterized by binding affinity ($K_D$) for a human CD73 polypeptide of less than (better than) $10^{-9}$ M, preferably less than $10^{-10}$ M, or preferably less than $10^{-11}$M, and/or by binding human CD73 with an EC50 lower than (better binding than) 1 $\mu$g/ml, preferably wherein the antibody has an $EC_{50}$ of no more than 0.5 $\mu$g/ml, optionally no more than 0.2 $\mu$g/ml, optionally no more than 0.1 $\mu$g/ml, for binding to cells (e.g. tumor cells) expressing human CD73 at the cell surface.

The antibodies are humanized antibodies.

The antibodies are optionally characterized by an $EC_{50}$ for neutralization of the enzymatic activity of CD73 in CD73-expressing cells of less than (better than) 1 $\mu$g/ml, optionally less than 0.5 $\mu$g/ml.

In one aspect, the antibody is a monoclonal antibody or a fragment thereof that retains binding specificity and ability to neutralize the enzymatic activity of CD73. In one aspect, the antibody is an IgG1, IgG2, IgG3, or IgG4 antibody. For example, the antibody may be an antibody comprising an Fc domain of human IgG4 isotype, or an antibody comprising an Fc domain of any human IgG isotype (e.g. IgG1, IgG2, IgG3, or IgG4) modified to reduce binding between the Fc domain and an Fcγ receptor (e.g. CD16). Preferably, the antigen-binding compound does not comprise an Fc domain capable of inducing antibody mediated cellular cytotoxicity (ADCC) and/or CDC; optionally the antigen-binding compound does not comprise an Fc domain capable of substantially binding to a FcγRIIIA (CD16) polypeptide (e.g., comprises an Fc domain not capable of substantially binding to a FcγRIIIA (CD16) polypeptide; lacks an Fc domain (e.g. lacks a CH2 and/or CH3 domain; comprises an Fc domain of IgG4 isotype). In one aspect, the Fc domain (e.g. of human IgG1, IgG2, IgG3 or IgG4 isotype) comprises an amino acid modification (e.g. substitution) compared to a wild-type Fc domain, wherein the substitution reduces the ability of the Fc domain (or antibodies containing it) to bind to an Fcγ receptor (e.g. CD16) and/or to bind complement. Optionally, if an Fc domain of IgG4 isotype is present, such Fc domain may comprise a stabilizing mutation to decrease formation of half-antibodies such as a mutation in the hinge, e.g. a S241P (S228P) mutation. Optionally the antigen-binding compound consists of or comprises a Fab, Fab', Fab'-SH, F (ab') 2, Fv, a diabody, single-chain antibody fragment, or a multispecific antibody comprising multiple different antibody fragments. In one embodiment, the antigen-binding compound is not linked to a toxic moiety.

Also provided are nucleic acids encoding the human or humanized antibody or antibody fragment having any of the foregoing properties, a vector comprising such a nucleic acid, a cell comprising such a vector, and a method of producing a human anti-CD73 antibody, comprising culturing such a cell under conditions suitable for expression of the anti- CD73 antibody. The disclosure also relates to compositions, such as pharmaceutically acceptable compositions and kits, comprising such proteins, nucleic acids, vectors, and/or cells and typically one or more additional ingredients that can be active ingredients or inactive ingredients that promote formulation, delivery, stability, or other characteristics of the composition (e.g., various carriers). The disclosure further relates various new and useful methods making and using such antibodies, nucleic acids, vectors, cells, organisms, and/or compositions, such as in the modulation of CD73-mediated biological activities, for example in the treatment of diseases related thereto, notably cancers.

The disclosure also provides methods of producing or testing an antibody which binds and neutralizes the enzymatic activity of CD73, said method comprising the steps of:

(a) providing a plurality of antibodies that bind a CD73 polypeptide,
(b) bringing each of said antibodies into contact (e.g., separately from one another) with a soluble CD73 polypeptide (e.g., in a cell-free assay, e.g. in the presence of AMP), and

(c) selecting an antibody (e.g. those of step (b)) that neutralizes the enzymatic activity of said soluble CD73 polypeptide. In one aspect, the antibodies are capable of binding CD73 in bivalent manner, e.g. the antibodies are full length IgG antibodies. Optionally, step (b) comprises bringing each of said antibodies into contact with a soluble CD73 polypeptide in a cell-free assay, wherein antibodies are provided in a molar excess of antibody (compared to CD73 polypeptide). Optionally, the CD73 polypeptide is a soluble CD73 dimer. Optionally step (c) comprises selecting an antibody that neutralizes the enzymatic activity of said soluble CD73 polypeptide when antibodies are provided at a molar excess of antibody to CD73 dimers (e.g., an at least 2-fold, 5-fold, 10-fold, or 100-fold molar excess).

The disclosure also provides methods of producing or testing an antibody which binds and neutralizes the enzymatic activity of CD73, said method comprising the steps of:

(a) providing a plurality of antibodies that bind a CD73 polypeptide,

(b) bringing each of said antibodies into contact (e.g., separately from one another) with a CD73 polypeptide in "open" conformation when not bound to substrate,

(c) bringing each of said antibodies into contact (e.g., separately from one another) with a CD73 polypeptide in "closed" conformation when bound to a substrate, and

(d) selecting an antibody (e.g. those obtained from steps (b) and (c) that binds, and optionally further neutralizes the enzymatic activity of, the CD73 polypeptide of step (b) and the CD73 polypeptide of step (c). In one aspect, the antibodies are capable of binding CD73 in bivalent manner, e.g. the antibodies are full length IgG antibodies. In one aspect, the substrate is a natural substrate of CD73 such as AMP or an inhibitor or other compound that binds the active site such as an AMP analogue adenosine 5'-($\alpha,\beta$-methylene)diphosphate (APCP)). Optionally, the method further comprises a step of further tested for activity (in vitro and/or in vivo) of the selected antibody. Optionally, the method further comprises producing a quantity of the selected antibody.

In one aspect, the step of providing a plurality of antibodies comprises immunizing a non-human mammal with an immunogen comprising a CD73 polypeptide.

The disclosure also provides an anti-CD73 antibody as described before for use for potentiating the activity of lymphocytes (e.g., T cells) in a subject in need thereof, or for restoring the activity of lymphocytes (e.g., T cells), or for relieving the adenosine-mediated inhibition of lymphocytes (e.g., T cells). In one aspect, the subject is a patient suffering from cancer. For example, the patient may be suffering from a solid tumor, e.g. colorectal cancer, renal cancer, ovarian cancer, lung cancer, breast cancer or malignant melanoma. Alternatively, the patient may be suffering from a hematopoietic cancer, e.g., acute myeloid leukaemia, chronic myeloid leukaemia, multiple myeloma, or non-Hodgkin's lymphoma.

The disclosure also provides an anti-CD73 antibody for use for the treatment of disease in an individual, wherein the anti-CD73 antibody is to be administered to the individual so that the anti-CD73 antibody neutralizes the enzymatic activity of CD73 for at least one administration cycle in which the anti-CD73 antibody is to be administered at least once, optionally at least twice, in an amount effective to achieve, and/or to maintain between two successive administrations of the anti-CD73 antibody, a concentration in blood (serum) or an extravascular tissue (e.g. tumor environment) that corresponds to at least the $EC_{50}$ (e.g. an $EC_{50}$ between 0.01 and 0.5 $\mu$g/ml), optionally the $EC_{70}$ or optionally the $EC_{100}$, for neutralization of the enzymatic activity of CD73 (e.g. an $EC_{100}$ between 0.05 and 1 $\mu$g/ml, between 0.1 and 1 $\mu$g/ml) . The antibody can for example be administered in an amount to achieve and/or maintained a concentration in circulation or in an extravascular tissue (e.g. tumor environment) of at least about 0.1 $\mu$g/ml, 0.5 $\mu$g/ml, 1 $\mu$g/ml or 2 $\mu$g/ml). For example, to achieve a concentration in an extravascular tissue of between 0.05 and 1 $\mu$g/ml, or between 0.1 and 1 $\mu$g/ml, the anti-CD73 antibody is to be administered in amounts effective to achieve a concentration in circulation of the anti-CD73 antibody of between 0.5 and 10 $\mu$g/ml, or between 1 and 10 $\mu$g/ml. Optionally, the anti-CD73 antibody is to be administered at least twice and in amounts effective to maintain the concentration of the anti-CD73 antibody at least the aforementioned concentration for at least 1 week, 2 weeks, 3 weeks, 4 weeks, between two successive administrations of the anti-CD73 antibody and/or throughout the administration cycle.

The disclosure also provides an anti-CD73 antibody for use for treatment of disease in an individual, wherein the anti-CD73 antibody is to be administered to the individual so that the anti-CD73 antibody neutralizes the enzymatic activity of CD73 for at least one administration cycle in which the anti-CD73 antibody is administered at least once, optionally at least twice, in an amount effective to achieve, and/or to maintain between two successive administrations of the anti-CD73 antibody, a blood or tissue concentration of anti-CD73 antibody of at least 1 $\mu$g/ml, optionally at least 10 $\mu$g/ml, optionally between 1 and 100 $\mu$g/ml. Optionally, the anti-CD73 antibody is to be administered at least twice and in amounts effective to maintain a continuous blood or tissue concentration of the anti-CD73 antibody of at least 1 $\mu$g/ml, optionally at least 10 $\mu$g/ml, optionally between 1 and 100 $\mu$g/ml, for at least 1 week, 2 weeks, 3 weeks, 4 weeks, between two successive administrations of the anti-Cd73 antibody and/or throughout the administration cycle.

**Definitions**

As used in the specification, "a" or "an" may mean one or more. As used in the claim(s), when used in conjunction with the word "comprising", the words "a" or "an" may mean one or more than one. As used herein "another" may mean at least a second or more.

Where "comprising" is used, this can optionally be replaced by "consisting essentially of" or by "consisting of".

Human CD73, also known as ecto-5'-nucleotidase and as 5-prime-ribonucleotide phosphohydrolase, EC 3.1.3.5, encoded by the *NT5E* gene, exhibits 5'-nucleotidase, notably AMP-, NAD-, and NMN-nucleosidase, activities. CD73 catalyzes the conversion at neutral pH of purine 5-prime mononucleotides to nucleosides, the preferred substrate being AMP. The enzyme consists of a dimer of 2 identical 70-kD subunits bound by a glycosyl phosphatidyl inositol linkage to the external face of the plasma membrane The amino acid sequence of Human CD73 preprotein (monomer), including a signal sequence at amino acids 1-26, is shown in Genbank under accession number NP_002517:

```
MCPRAARAPA  TLLLALGAVL  WPAAGAWELT  ILHTNDVHSR  LEQTSEDSSK  CVNASRCMGG
VARLFTKVQQ  IRRAEPNVLL  LDAGDQYQGT  IWFTVYKGAE  VAHFMNALRY  DAMALGNHEF
DNGVEGLIEP  LLKEAKFPIL  SANIKAKGPL  ASQISGLYLP  YKVLPVGDEV  VGIVGYTSKE
TPFLSNPGTN  LVFEDEITAL  QPEVDKLKTL  NVNKIIALGH  SGFEMDKLIA  QKVRGVDVVV
GGHSNTFLYT  GNPPSKEVPA  GKYPFIVTSD  DGRKVPVVQA  YAFGKYLGYL  KIEFDERGNV
ISSHGNPILL  NSSIPEDPSI  KADINKWRIK  LDNYSTQELG  KTIVYLDGSS  QSCRFRECNM
GNLICDAMIN  NNLRHTDEMF  WNHVSMCILN  GGGIRSPIDE  RNNGTITWEN  LAAVLPFGGT
FDLVQLKGST  LKKAFEHSVH  RYGQSTGEFL  QVGGIHVVYD  LSRKPGDRVV  KLDVLCTKCR
VPSYDPLKMD  EVYKVILPNF  LANGGDGFQM  IKDELLRHDS  GDQDINVVST  YISKMKVIYP
AVEGRIKFST  GSHCHGSFSL  IFLSLWAVIF  VLYQ
```
(SEQ ID NO: 1).

In the context herein, "neutralize the enzymatic activity of CD73", refers to a process in which the 5'-nucleotidase (5'-ectonucleotidase) activity of CD73 is inhibited. This comprises, notably the inhibition of CD73-mediated generation of adenosine, i.e. the inhibition of CD73-mediated catabolism of AMP to adenosine. This can be measured for example in a cell-free assay that measures the capacity of a test compound to inhibit the conversion of AMP to adenosine, either directly or indirectly. In one aspect, an antibody preparation causes at least a 50% decrease in the conversion of AMP to adenosine, at least a 70% decrease in the conversion of AMP to adenosine, or at least a 80% decrease in the conversion of AMP to adenosine, referring, for example, to the assays described herein.

Whenever within this whole specification "treatment of cancer" or the like is mentioned with reference to anti-CD73 binding agent (e.g. antibody), there is meant: (a) method of treatment of cancer, said method comprising the step of administering (for at least one treatment) an anti-CD73 binding agent, (preferably in a pharmaceutically acceptable carrier material) to an individual, a mammal, especially a human, in need of such treatment, in a dose that allows for the treatment of cancer, (a therapeutically effective amount), preferably in a dose (amount) as specified herein; (b) the use of an anti-CD73 binding agent for the treatment of cancer, or an anti-CD73 binding agent, for use in said treatment (especially in a human); (c) the use of an anti-CD73 binding agent for the manufacture of a pharmaceutical preparation for the treatment of cancer, a method of using an anti-CD73 binding agent for the manufacture of a pharmaceutical preparation for the treatment of cancer, comprising admixing an anti-CD73 binding agent with a pharmaceutically acceptable carrier, or a pharmaceutical preparation comprising an effective dose of an anti-CD73 binding agent that is appropriate for the treatment of cancer; or (d) any combination of a), b), and c). References to methods of treatment in this description are to be interpreted as references to the compounds, pharmaceutical compositions and medicaments of the present invention for use in a method for treatment of the human (o animal) body by therapy (of for diagnosis).

The term "antibody," as used herein, refers to polyclonal and monoclonal antibodies. Depending on the type of constant domain in the heavy chains, antibodies are assigned to one of five major classes: IgA, IgD, IgE, IgG, and IgM. Several of these are further divided into subclasses or isotypes, such as IgG1, IgG2, IgG3, IgG4, and the like. An exemplary immunoglobulin (antibody) structural unit comprises a tetramer. Each tetramer is composed of two identical pairs of polypeptide chains, each pair having one "light" (about 25 kDa) and one "heavy" chain (about 50-70 kDa). The N-terminus of each chain defines a variable region of about 100 to 110 or more amino acids that is primarily responsible for antigen recognition. The terms variable light chain ($V_L$) and variable heavy chain ($V_H$) refer to these light and heavy chains respectively. The heavy-chain constant domains that correspond to the different classes of immunoglobulins are termed "alpha," "delta," "epsilon," "gamma" and "mu," respectively. The subunit structures and three-dimensional configurations of different classes of immunoglobulins are well known. IgG are the exemplary classes of antibodies employed herein because they are the most common antibodies in the physiological situation and because they are most easily made in a laboratory setting. Optionally the antibody is a monoclonal antibody. Particular examples of antibodies are humanized, chimeric, human, or otherwise-human-suitable antibodies. "Antibodies" also includes any fragment or derivative of any of the herein described antibodies.

The term "specifically binds to" means that an antibody can bind preferably in a competitive binding assay to the binding partner, e.g. CD73, as assessed using either recombinant forms of the proteins, epitopes therein, or native proteins present on the surface of isolated target cells. Competitive binding assays and other methods for determining specific binding are further described below and are well known in the art.

When an antibody is said to "compete with" a particular monoclonal antibody, it means that the antibody competes with the monoclonal antibody in a binding assay using either recombinant CD73 molecules or surface expressed CD73 molecules. For example, if a test antibody reduces the binding of a reference antibody to a CD73 polypeptide or CD73-expressing cell in a binding assay, the antibody is said to "compete" respectively with the reference antibody.

The term "affinity", as used herein, means the strength of the binding of an antibody to an epitope. The affinity of an antibody is given by the dissociation constant Kd, defined as [Ab] x [Ag] / [Ab-Ag], where [Ab-Ag] is the molar concentration of the antibody-antigen complex, [Ab] is the molar concentration of the unbound antibody and [Ag] is the molar concentration of the unbound antigen. The affinity constant $K_a$ is defined by 1/Kd. Methods for determining the affinity of mAbs can be found in Harlow, et al., Antibodies: A Laboratory Manual, Cold Spring Harbor Laboratory Press, Cold Spring Harbor, N.Y., 1988), Coligan et al., eds., Current Protocols in Immunology, Greene Publishing Assoc. and Wiley Interscience, N.Y., (1992, 1993), and Muller, Meth. Enzymol. 92:589-601 (1983). One standard method well known in the art for determining the affinity of mAbs is the use of surface plasmon resonance (SPR) screening (such as by analysis with a BIAcore™ SPR analytical device).

Within the context herein a "determinant" designates a site of interaction or binding on a polypeptide.

The term "epitope" refers to an antigenic determinant, and is the area or region on an antigen to which an antibody binds. A protein epitope may comprise amino acid residues directly involved in the binding as well as amino acid residues which are effectively blocked by the specific antigen binding antibody or peptide, i.e., amino acid residues within the "footprint" of the antibody. It is the simplest form or smallest structural area on a complex antigen molecule that can combine with e.g., an antibody or a receptor. Epitopes can be linear or conformational/structural. The term "linear epitope" is defined as an epitope composed of amino acid residues that are contiguous on the linear sequence of amino acids (primary structure). The term "conformational or structural epitope" is defined as an epitope composed of amino acid residues that are not all contiguous and thus represent separated parts of the linear sequence of amino acids that are brought into proximity to one another by folding of the molecule (secondary, tertiary and/or quaternary structures). A conformational epitope is dependent on the 3-dimensional structure. The term 'conformational' is therefore often used interchangeably with 'structural'.

The term "deplete" or "depleting", with respect to CD73-expressing cells, means a process, method, or compound that results in killing, elimination, lysis or induction of such killing, elimination or lysis, so as to negatively affect the number of such CD73-expressing cells present in a sample or in a subject.

The term "internalization", used interchangeably with "intracellular internalization", refers to the molecular, biochemical and cellular events associated with the process of translocating a molecule from the extracellular surface of a cell to the intracellular surface of a cell. The processes responsible for intracellular internalization of molecules are well-known and can involve, *inter alia,* the internalization of extracellular molecules (such as hormones, antibodies, and small organic molecules); membrane-associated molecules (such as cell-surface receptors); and complexes of membrane-associated molecules bound to extracellular molecules (for example, a ligand bound to a transmembrane receptor or an antibody bound to a membrane-associated molecule). Thus, "inducing and/or increasing internalization" comprises events wherein intracellular internalization is initiated and/or the rate and/or extent of intracellular internalization is increased.

The term "agent" is used herein to denote a chemical compound, a mixture of chemical compounds, a biological macromolecule, or an extract made from biological materials. The term "therapeutic agent" refers to an agent that has biological activity.

For the purposes herein, a "humanized" or "human" antibody refers to an antibody in which the constant and variable framework region of one or more human immunoglobulins is fused with the binding region, e.g. the CDR, of an animal immunoglobulin. Such antibodies are designed to maintain the binding specificity of the non-human antibody from which the binding regions are derived, but to avoid an immune reaction against the non-human antibody. Such antibodies can be obtained from transgenic mice or other non-human animals that have been "engineered" to produce specific human antibodies in response to antigenic challenge (see, e.g., Green et al. (1994) Nature Genet 7:13; Lonberg et al. (1994) Nature 368:856; Taylor et al. (1994) Int Immun 6:579). A fully human antibody also can be constructed by genetic or chromosomal transfection methods, as well as phage display technology, all of which are known in the art (see, e.g., McCafferty et al. (1990) Nature 348:552-553). Human antibodies may also be generated by in vitro activated B cells (see, e.g., U.S. Pat. Nos. 5,567,610 and 5,229,275).

A "chimeric antibody" is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity.

The term "hypervariable region" when used herein refers to the amino acid residues of an antibody that are responsible for antigen binding. The hypervariable region generally comprises amino acid residues from a "complementarity-determining region" or "CDR" (e.g. residues 24-34 (L1), 50-56 (L2) and 89-97 (L3) in the light-chain variable domain and 31-35 (H1), 50-65 (H2) and 95-102 (H3) in the heavy-chain variable domain; Kabat et al. 1991) and/or those residues from a "hypervariable loop" (e.g. residues 26-32 (L1), 50-52 (L2) and 91-96 (L3) in the light-chain variable domain and 26-32 (H1), 53-55 (H2) and 96-101 (H3) in the heavy-chain variable domain; Chothia and Lesk, J. Mol. Biol 1987;196:901-917), or a similar system for determining essential amino acids responsible for antigen binding. Typically, the numbering of amino acid residues in this region is performed by the method described in Kabat et al., supra. Phrases such as "Kabat position", "variable domain residue numbering as in Kabat" and "according to Kabat" herein refer to this numbering system for heavy chain variable domains or light chain variable domains. Using the Kabat numbering system, the actual linear amino acid sequence of a peptide may contain fewer or additional amino acids corresponding to a shortening of, or insertion into, a FR or CDR of the variable domain. For example, a heavy chain variable domain may include a single amino acid insert (residue 52a according to Kabat) after residue 52 of CDR H2 and inserted residues (e.g. residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

By "framework" or "FR" residues as used herein is meant the region of an antibody variable domain exclusive of those regions defined as CDRs. Each antibody variable domain framework can be further subdivided into the contiguous regions separated by the CDRs (FR1, FR2, FR3 and FR4).

The terms "Fc domain," "Fc portion," and "Fc region" refer to a C-terminal fragment of an antibody heavy chain, e.g., from about amino acid (aa) 230 to about aa 450 of human $\gamma$ (gamma) heavy chain or its counterpart sequence in other types of antibody heavy chains (e.g., $\alpha$, $\delta$, $\epsilon$ and $\mu$ for human antibodies), or a naturally occurring allotype thereof. Unless otherwise specified, the commonly accepted Kabat amino acid numbering for immunoglobulins is used throughout this disclosure (see Kabat et al. (1991 ) Sequences of Protein of Immunological Interest, 5th ed., United States Public Health Service, National Institute of Health, Bethesda, MD).

The terms "isolated", "purified" or "biologically pure" refer to material that is substantially or essentially free from components which normally accompany it as found in its native state. Purity and homogeneity are typically determined using analytical chemistry techniques such as polyacrylamide gel electrophoresis or high performance liquid chromatography. A protein that is the predominant species present in a preparation is substantially purified.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "recombinant" when used with reference, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (nonrecombinant) form of the cell or express native genes that are otherwise abnormally expressed, under expressed or not expressed at all.

Within the context herein, the term antibody that "binds" a polypeptide or epitope designates an antibody that binds said determinant with specificity and/or affinity.

The term "identity" or "identical", when used in a relationship between the sequences of two or more polypeptides, refers to the degree of sequence relatedness between polypeptides, as determined by the number of matches between strings of two or more amino acid residues. "Identity" measures the percent of identical matches between the smaller of two or more sequences with gap alignments (if any) addressed by a particular mathematical model or computer program (i.e., "algorithms"). Identity of related polypeptides can be readily calculated by known methods. Such methods include, but are not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48, 1073 (1988).

Methods for determining identity are designed to give the largest match between the sequences tested. Methods of determining identity are described in publicly available computer programs. Computer program methods for determining identity between two sequences include the GCG program package, including GAP (Devereux et al., Nucl. Acid. Res. 12, 387 (1984); Genetics Computer Group, University of Wisconsin, Madison, Wis.), BLASTP, BLASTN, and FASTA (Altschul et al., J. Mol. Biol. 215, 403-410 (1990)). The BLASTX program is publicly available from the National Center for Biotechnology Information (NCBI) and other sources (BLAST Manual, Altschul et al. NCB/NLM/NIH Bethesda, Md. 20894; Altschul et al., supra). The well-known Smith Waterman algorithm may also be used to determine identity.

**Production of antibodies**

The anti-CD73 agent that can be used for the treatment of cancers binds an extracellular portion of human CD73 polypeptide and neutralizes the enzymatic activity of CD73 expressed on the surface of a cell, e.g. a tumor cell. In one aspect, the agent inhibits the 5'-ectonucleotidase activity of CD73. In one aspect, the antibody inhibits CD73-mediated generation of adenosine. In one aspect, the antibody inhibits CD73-mediated catabolism of AMP to adenosine. In one aspect, the antibody inhibits adenosine-mediated inhibition of lymphocyte activity (e.g. T cells). In one aspect, the antibody binds and/or inhibits the enzymatic active site on CD73. In one aspect, the agent is an antibody selected from a full-length antibody, an antibody fragment, and a synthetic or semi-synthetic antibody-derived molecule.

In one aspect, the agent is a humanized antibody.

In one aspect, the agent is a fragment of an antibody comprising a constant domain selected from IgG1, IgG2, IgG3 and IgG4.

In one aspect, the agent is an antibody fragment selected from a Fab fragment, a Fab' fragment, a Fab'-SH fragment, a F(ab)2 fragment, a F(ab')2 fragment, an Fv fragment, a Heavy chain Ig (a llama or camel Ig), a $V_{HH}$ fragment, a single domain FV, and a single-chain antibody fragment.

In one aspect, the agent is a synthetic or semisynthetic antibody-derived molecule selected from a scFV, a dsFV, a minibody, a diabody, a triabody, a kappa body, an IgNAR; and a multispecific antibody.

The present disclosure thus concerns antibodies or other antigen-binding agents binding to CD73.

In one aspect, the antibody is in at least partially purified form.

In one aspect, the antibody is in essentially isolated form.

The antibodies may be produced by a variety of techniques known in the art. Typically, they are produced by immunization of a non-human animal, preferably a mouse, with an immunogen comprising a CD73 polypeptide, preferably a human CD73 polypeptide. The CD73 polypeptide may comprise the full length sequence of a human CD73 polypeptide, or a fragment or derivative thereof, typically an immunogenic fragment, i.e., a portion of the polypeptide comprising an epitope exposed on the surface of cells expressing a CD73 polypeptide. Such fragments typically contain at least about 7 consecutive amino acids of the mature polypeptide sequence, even more preferably at least about 10 consecutive amino acids thereof. Fragments typically are essentially derived from the extracellular domain of the receptor. In one aspect, the immunogen comprises a wild-type human CD73 polypeptide in a lipid membrane, typically at the surface of a cell. In a specific aspect, the immunogen comprises intact cells, particularly intact human cells, optionally treated or lysed. In another aspect, the polypeptide is a recombinant CD73 polypeptide.

The step of immunizing a non-human mammal with an antigen may be carried out in any manner well known in the art for stimulating the production of antibodies in a mouse (see, for example, E. Harlow and D. Lane, Antibodies: A Laboratory Manual., Cold Spring Harbor Laboratory Press, Cold Spring Harbor, NY (1988)). The immunogen is suspended or dissolved in a buffer, optionally with an adjuvant, such as complete or incomplete Freund's adjuvant. Methods for determining the amount of immunogen, types of buffers and amounts of adjuvant are well known to those of skill in the art and are not limiting in any way. These parameters may be different for different immunogens, but are easily elucidated.

Similarly, the location and frequency of immunization sufficient to stimulate the production of antibodies is also well known in the art. In a typical immunization protocol, the non-human animals are injected intraperitoneally with antigen on day 1 and again about a week later. This is followed by recall injections of the antigen around day 20, optionally with an adjuvant such as incomplete Freund's adjuvant. The recall injections are performed intravenously and may be repeated for several consecutive days. This is followed by a booster injection at day 40, either intravenously or intraperitoneally, typically without adjuvant. This protocol results in the production of antigen-specific antibody-producing B cells after about 40 days. Other protocols may also be used as long as they result in the production of B cells expressing an antibody directed to the antigen used in immunization.

For polyclonal antibody preparation, serum is obtained from an immunized non-human animal and the antibodies present therein isolated by well-known techniques. The serum may be affinity purified using any of the immunogens set forth above linked to a solid support so as to obtain antibodies that react with CD73 polypeptides.

In an alternate aspect, lymphocytes from a non-immunized non-human mammal are isolated, grown in vitro, and then exposed to the immunogen in cell culture. The lymphocytes are then harvested and the fusion step described below is carried out.

For monoclonal antibodies, the next step is the isolation of splenocytes from the immunized non-human mammal and the subsequent fusion of those splenocytes with an immortalized cell in order to form an antibody-producing hybridoma. The isolation of splenocytes from a non-human mammal is well-known in the art and typically involves removing the spleen from an anesthetized non-human mammal, cutting it into small pieces and squeezing the splenocytes from the splenic capsule through a nylon mesh of a cell strainer into an appropriate buffer so as to produce a single cell suspension. The cells are washed, centrifuged and resuspended in a buffer that lyses any red blood cells. The solution is again centrifuged and remaining lymphocytes in the pellet are finally resuspended in fresh buffer.

Once isolated and present in single cell suspension, the lymphocytes can be fused to an immortal cell line. This is typically a mouse myeloma cell line, although many other immortal cell lines useful for creating hybridomas are known in the art. Murine myeloma lines include, but are not limited to, those derived from MOPC-21 and MPC-11 mouse tumors available from the Salk Institute Cell Distribution Center, San Diego, U. S. A., X63 Ag8653 and SP-2 cells available from the American Type Culture Collection, Rockville, Maryland U. S. A. The fusion is effected using polyethylene glycol or the like. The resulting hybridomas are then grown in selective media that contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Hybridomas are typically grown on a feeder layer of macrophages. The macrophages are preferably from littermates of the non-human mammal used to isolate splenocytes and are typically primed with incomplete Freund's adjuvant or the like several days before plating the hybridomas. Fusion methods are described in Goding, "Monoclonal Antibodies: Principles and Practice," pp. 59-103 (Academic Press, 1986).

The cells are allowed to grow in the selection media for sufficient time for colony formation and antibody production. This is usually between about 7 and about 14 days.

The hybridoma colonies are then assayed for the production of antibodies that specifically bind to CD73 polypeptide gene products. The assay is typically a colorimetric ELISA-type assay, although any assay may be employed that can be adapted to the wells that the hybridomas are grown in. Other assays include radioimmunoassays or fluorescence activated cell sorting. The wells positive for the desired antibody production are examined to determine if one or more distinct colonies are present. If more than one colony is present, the cells may be re-cloned and grown to ensure that only a single cell has given rise to the colony producing the desired antibody. Typically, the antibodies will also be tested for the ability to bind to CD73 polypeptides, e.g., CD73-expressing cells.

Hybridomas that are confirmed to produce a monoclonal antibody can be grown up in larger amounts in an appropriate medium, such as DMEM or RPMI-1640. Alternatively, the hybridoma cells can be grown in vivo as ascites tumors in an animal.

After sufficient growth to produce the desired monoclonal antibody, the growth media containing monoclonal antibody (or the ascites fluid) is separated away from the cells and the monoclonal antibody present therein is purified. Purification is typically achieved by gel electrophoresis, dialysis, chromatography using protein A or protein G-Sepharose, or an anti-mouse Ig linked to a solid support such as agarose or Sepharose beads (all described, for example, in the Antibody Purification Handbook, Biosciences, publication No. 18-1037-46, Edition AC). The bound antibody is typically eluted from protein A/protein G columns by using low pH buffers (glycine or acetate buffers of pH 3.0 or less) with immediate neutralization of antibody-containing fractions. These fractions are pooled, dialyzed, and concentrated as needed.

Positive wells with a single apparent colony are typically re-cloned and re-assayed to insure only one monoclonal antibody is being detected and produced.

Antibodies may also be produced by selection of combinatorial libraries of immunoglobulins, as disclosed for instance in (Ward et al. Nature, 341 (1989) p. 544).

The identification of one or more antibodies that bind(s) to CD73, particularly substantially or essentially the same epitope as monoclonal antibody 11E1, 8C7 or 6E1, can be readily determined using any one of a variety of immunological screening assays in which antibody competition can be assessed. Many such assays are routinely practiced and are well known in the art (see, e. g., U. S. Pat. No. 5,660,827, issued Aug. 26, 1997). It will be understood that actually determining the epitope to which an antibody described herein binds is not in any way required to identify an antibody that binds to the same or substantially the same epitope as the monoclonal antibody described herein.

The antibody may be an antibody other than any of antibodies 11E1, 8C7, 3C12 or 6E1, e.g. an antibody comprising heavy and/or light chain variable region amino acid sequences and/or CDRs that differ from those of any one or more of antibodies 11E1, 8C7, 3C12 or 6E1.

For example, where the test antibodies to be examined are obtained from different source animals, or are even of a different Ig isotype, a simple competition assay may be employed in which the control (11E1, 8C7 or 6E1, for example) and test antibodies are admixed (or pre-adsorbed) and applied to a sample containing CD73 polypeptides. Protocols based upon western blotting and the use of BIACORE analysis are suitable for use in such competition studies.

In certain aspects, one pre-mixes the control antibodies (11E1, 8C7, 3C12 or 6E1, for example) with varying amounts of the test antibodies (e.g., about 1:10 or about 1:100) for a period of time prior to applying to the CD73 antigen sample. In other aspects, the control and varying amounts of test antibodies can simply be admixed during exposure to the CD73 antigen sample. As long as one can distinguish bound from free antibodies (e. g., by using separation or washing techniques to eliminate unbound antibodies) and 11E1, 8C7, 3C12 or 6E1 from the test antibodies (e. g., by using species-specific or isotype-specific secondary antibodies or by specifically labeling 11E1, 8C7, 3C12 or 6E1 with a detectable label) one can determine if the test antibodies reduce the binding of 11E1, 8C7, 3C12 or 6E1 to the antigens, indicating that the test antibody recognizes substantially the same epitope as 11E1, 8C7, 3C12 or 6E1. The binding of the (labelled) control antibodies in the absence of a completely irrelevant antibody can serve as the control high value. The control low value can be obtained by incubating the labelled (11E1, 8C7, 3C12 or 6E1) antibodies with unlabelled antibodies of exactly the same type (11E1, 8C7, 3C12 or 6E1), where competition would occur and reduce binding of the labelled antibodies. In a test assay, a significant reduction in labelled antibody reactivity in the presence of a test antibody is indicative of a test antibody that recognizes substantially the same epitope, i.e., one that "cross-reacts" or competes with the labelled (11E1, 8C7, 3C12 or 6E1) antibody. Any test antibody that reduces the binding of 11E1, 8C7, 3C12 or 6E1 to CD73 antigens by at least about 50%, such as at least about 60%, or more preferably at least about 80% or 90% (e. g., about 65-100%), at any ratio of 11E1, 8C7, 3C12 or 6E1:test antibody between about 1:10 and about 1:100 is considered to be an antibody that binds to substantially the same epitope or determinant as 11E1, 8C7, 3C12 or 6E1. Preferably, such test antibody will reduce the binding of 11E1, 8C7, 3C12 or 6E1 to the CD73 antigen by at least about 90% (e.g., about 95%).

Competition can also be assessed by, for example, a flow cytometry test. In such a test, cells bearing a given CD73 polypeptide can be incubated first with 11E1, 8C7, 3C12 or 6E1, for example, and then with the test antibody labelled with a fluorochrome or biotin. The antibody is said to compete with 11E1, 8C7, 3C12 or 6E1 if the binding obtained upon preincubation with a saturating amount of 11E1, 8C7, 3C12 or 6E1 is about 80%, preferably about 50%, about 40% or less (e.g., about 30%, 20% or 10%) of the binding (as measured by mean of fluorescence) obtained by the antibody without preincubation with 11E1, 8C7, 3C12 or 6E1. Alternatively, an antibody is said to compete with 11E1, 8C7, 3C12 or 6E1 if the binding obtained with a labelled 11E1, 8C7, 3C12 or 6E1 antibody (by a fluorochrome or biotin) on cells preincubated with a saturating amount of test antibody is about 80%, preferably about 50%, about 40%, or less (e. g., about 30%, 20% or 10%) of the binding obtained without preincubation with the test antibody.

A simple competition assay in which a test antibody is pre-adsorbed and applied at saturating concentration to a surface onto which a CD73 antigen is immobilized may also be employed. The surface in the simple competition assay is preferably a BIACORE chip (or other media suitable for surface plasmon resonance analysis). The control antibody (e.g., 11E1, 8C7, 3C12 or 6E1) is then brought into contact with the surface at a CD73-saturating concentration and the CD73 and surface binding of the control antibody is measured. This binding of the control antibody is compared with the binding of the control antibody to the CD73-containing surface in the absence of test antibody. In a test assay, a significant reduction in binding of the CD73-containing surface by the control antibody in the presence of a test antibody indicates that the test antibody recognizes substantially the same epitope as the control antibody such that the test antibody "cross-reacts" with the control antibody. Any test antibody that reduces the binding of control (such as 11E1, 8C7, 3C12 or 6E1) antibody to a CD73 antigen by at least about 30% or more, preferably about 40%, can be considered to be an antibody that binds to substantially the same epitope or determinant as a control (e.g., 11E1, 8C7, 3C12 or 6E1). Preferably, such a test antibody will reduce the binding of the control antibody (e.g., 11E1, 8C7, 3C12 or 6E1) to the CD73 antigen by at least about 50% (e. g., at least about 60%, at least about 70%, or more). It will be appreciated that the order of control and test antibodies can be reversed: that is, the control antibody can be first bound to the surface and the test antibody is brought into contact with the surface thereafter in a competition assay. Preferably, the antibody having higher affinity for the CD73 antigen is bound to the surface first, as it will be expected that the decrease in binding seen for the second antibody (assuming the antibodies are cross-reacting) will be of greater magnitude. Further examples of such assays are provided in, e.g., Saunal (1995) J. Immunol. Methods 183: 33-41.

The antibodies will bind to CD73-expressing cells from an individual or individuals with a disease characterized by expression of CD73-positive cells, i.e. an individual that is a candidate for treatment with one of the herein-described methods using an anti-CD73 antibody. Accordingly, once an antibody that specifically recognizes CD73 on cells is obtained, it can optionally be tested for its ability to bind to CD73-positive cells (e.g. cancer cells). In particular, prior to treating a patient with one of the present antibodies, one may optionally test the ability of the antibody to bind malignant cells taken from the patient, e.g. in a blood sample or tumor biopsy, to maximize the likelihood that the therapy will be beneficial in the patient.

In one aspect, the antibodies are validated in an immunoassay to test their ability to bind to CD73-expressing cells, e.g. malignant cells. For example, a blood sample or tumor biopsy is performed and tumor cells are collected. The ability of a given antibody to bind to the cells is then assessed using standard methods well known to those in the art. Antibodies may bind for example to a substantial proportion (e.g., 20%, 30%, 40%, 50%, 60%, 70%, 80% or more) of cells known to express CD73, e.g. tumor cells, from a significant percentage of individuals or patients (e.g., 10%, 20%, 30%, 40%, 50% or more). Antibodies can be used for diagnostic purposes to determine the presence or level of malignant cells in a patient, for example as a biomarker to assess whether a patient is suitable for treatment with an anti-CD73 agent, or for use in the herein-described therapeutic methods. To assess the binding of the antibodies to the cells, the antibodies can either be directly or indirectly labelled. When indirectly labelled, a secondary, labelled antibody is typically added.

Determination of whether an antibody binds within an epitope region can be carried out in ways known to the person skilled in the art. As one example of such mapping/characterization methods, an epitope region for an anti-CD73 antibody may be determined by epitope "foot-printing" using chemical modification of the exposed amines/carboxyls in the CD73 protein. One specific example of such a foot-printing technique is the use of HXMS (hydrogen-deuterium exchange detected by mass spectrometry) wherein a hydrogen/deuterium exchange of receptor and ligand protein amide protons, binding, and back exchange occurs, wherein the backbone amide groups participating in protein binding are protected from back exchange and therefore will remain deuterated. Relevant regions can be identified at this point by peptic proteolysis, fast microbore high-performance liquid chromatography separation, and/or electrospray ionization mass spectrometry. See, e. g., Ehring H, Analytical Biochemistry, Vol. 267 (2) pp. 252-259 (1999) Engen, J. R. and Smith, D. L. (2001) Anal. Chem. 73, 256A-265A. Another example of a suitable epitope identification technique is nuclear magnetic resonance epitope mapping (NMR), where typically the position of the signals in two-dimensional NMR spectra of the free antigen and the antigen complexed with the antigen binding peptide, such as an antibody, are compared. The antigen typically is selectively isotopically labeled with 15N so that only signals corresponding to the antigen and no signals from the antigen binding peptide are seen in the NMR-spectrum. Antigen signals originating from amino acids involved in the interaction with the antigen binding peptide typically will shift position in the spectrum of the complex compared to the spectrum of the free antigen, and the amino acids involved in the binding can be identified that way. See, e. g., Ernst Schering Res Found Workshop. 2004; (44): 149-67; Huang et al., Journal of Molecular Biology, Vol. 281 (1) pp. 61-67 (1998); and Saito and Patterson, Methods. 1996 Jun; 9 (3): 516-24.

Epitope mapping/characterization also can be performed using mass spectrometry methods. See, e.g., Downard, J Mass Spectrom. 2000 Apr; 35 (4): 493-503 and Kiselar and Downard, Anal Chem. 1999 May 1; 71 (9): 1792-1801. Protease digestion techniques also can be useful in the context of epitope mapping and identification. Antigenic determinant-relevant regions/sequences can be determined by protease digestion, e.g. by using trypsin in a ratio of about 1:50 to CD73 or o/n digestion at and pH 7-8, followed by mass spectrometry (MS) analysis for peptide identification. The peptides protected from trypsin cleavage by the anti-CD73 binder can subsequently be identified by comparison of samples subjected to trypsin digestion and samples incubated with antibody and then subjected to digestion by e.g. trypsin (thereby revealing a footprint for the binder). Other enzymes like chymotrypsin, pepsin, etc., also or alternatively can be used in similar epitope characterization methods. Moreover, enzymatic digestion can provide a quick method for analyzing whether a potential antigenic determinant sequence is within a region of the CD73 polypeptide that is not surface exposed and, accordingly, most likely not relevant in terms of immunogenicity/antigenicity.

Site-directed mutagenesis is another technique useful for elucidation of a binding epitope. For example, in "alanine-scanning", each residue within a protein segment is replaced with an alanine residue, and the consequences for binding affinity measured. If the mutation leads to a significant reduction in binding affinity, it is most likely involved in binding. Monoclonal antibodies specific for structural epitopes (i.e., antibodies which do not bind the unfolded protein) can be used to verify that the alanine-replacement does not influence over-all fold of the protein. See, e.g., Clackson and Wells, Science 1995; 267:383-386; and Wells, Proc Natl Acad Sci USA 1996; 93:1-6.

Electron microscopy can also be used for epitope "foot-printing". For example, Wang et al., Nature 1992; 355:275-278 used coordinated application of cryoelectron micros-copy, three-dimensional image reconstruction, and X-ray crystallography to determine the physical footprint of a Fab-fragment on the capsid surface of native cowpea mosaic virus.

Other forms of "label-free" assay for epitope evaluation include surface plasmon resonance (SPR, BIACORE) and reflectometric interference spectroscopy (RifS). See, e.g., Fägerstam et al., Journal Of Molecular Recognition 1990;3:208-14; Nice et al., J. Chroma-togr. 1993; 646:159-168; Leipert et al., Angew. Chem. Int. Ed. 1998; 37:3308-3311; Kröger et al., Biosensors and Bioelectronics 2002; 17:937-944.

It should also be noted that an antibody binding the same or substantially the same epitope as an antibody can be identified in one or more of the exemplary competition assays described herein.

Upon immunization and production of antibodies in a non-human vertebrate or cell, particular selection steps may be performed to isolate antibodies as claimed. In this regard, in a specific aspect, the disclosure also relates to methods of producing such antibodies, comprising: (a) immunizing a non-human mammal with an immunogen comprising a CD73 polypeptide; and (b) preparing antibodies from said immunized animal; and (c) selecting antibodies from step (b) that are capable of binding CD73.

Typically, an anti-CD73 antibody provided herein has an affinity for a CD73 polypeptide (e.g. as a CD73 homodimer) in the range of about $10^4$ to about $10^{11}$ $M^{-1}$ (e.g., about $10^8$ to about $10^{10}$ $M^{-1}$). For example, in a particular aspect the disclosure provides Anti-CD73 antibody that have an average disassociation constant ($K_D$) of less than 1 x $10^{-9}$ M with respect to CD73, as determined by, e.g., surface plasmon resonance (SPR) screening (such as by analysis with a BIAcore™ SPR analytical device). In a more particular exemplary aspect, the disclosure provides anti-CD73 antibodies that have a KD of about 1 x $10^{-8}$ M to about 1 x $10^{-10}$ M, or about 1 x $10^{-9}$ M to about 1 x $10^{-11}$ M, for CD73.

Antibodies can be characterized for example by a mean KD of no more than about (i.e. better affinity than) 100, 60, 10, 5, or 1 nanomolar, preferably sub-nanomolar or optionally no more than about 500, 200, 100 or 10 picomolar. KD can be determined for example for example by immobilizing recombinantly produced human CD73 proteins on a chip surface, followed by application of the antibody to be tested in solution. In one aspect, the method further comprises a step (d), selecting antibodies from (b) that are capable of competing for binding to CD73 with antibody 11E1, 8C7, 3C12 or 6E1.

In one aspect, the antibodies prepared according to the present methods are monoclonal antibodies. In another aspect, the non-human animal used to produce antibodies according to the methods herein is a mammal, such as a rodent, bovine, porcine, fowl, horse, rabbit, goat, or sheep.

DNA encoding an antibody that binds an epitope present on CD73 polypeptides is isolated from a hybridoma and placed in an appropriate expression vector for transfection into an appropriate host. The host is then used for the recombinant production of the antibody, or variants thereof, such as a humanized version of that monoclonal antibody, active fragments of the antibody, chimeric antibodies comprising the antigen recognition portion of the antibody, or versions comprising a detectable moiety.

DNA encoding the monoclonal antibodies of the disclosure, e.g., antibody 11E1, 8C7, 3C12 or 6E1, can be readily isolated and sequenced using conventional procedures (e. g., by using oligonucleotide probes that are capable of binding specifically to genes encoding the heavy and light chains of murine antibodies). In one aspect, provided is a nucleic acid encoding a heavy chain or a light chain of an anti-CD73 antibody of any aspect herein. Once isolated, the DNA can be placed into expression vectors, which are then transfected into host cells such as E. coli cells, simian COS cells, Chinese hamster ovary (CHO) cells, or myeloma cells that do not otherwise produce immunoglobulin protein, to obtain the synthesis of monoclonal antibodies in the recombinant host cells. As described elsewhere in the present specification, such DNA sequences can be modified for any of a large number of purposes, e.g., for humanizing antibodies, producing fragments or derivatives, or for modifying the sequence of the antibody, e.g., in the antigen binding site in order to optimize the binding specificity of the antibody. In one aspect, provided is an isolated nucleic acid sequence encoding a light chain and/or a heavy chain of an antibody (e.g. 11E1, 8C7, 3C12 or 6E1), as well as a recombinant host cell comprising (e.g. in its genome) such nucleic acid. Recombinant expression in bacteria of DNA encoding the antibody is well known in the art (see, for example, Skerra et al., Curr. Opinion in Immunol., 5, pp. 256 (1993); and Pluckthun, Immunol. 130, p. 151 (1992).

Optionally, antibodies of the disclosure can be specified to be antibodies other than any one or more of antibodies 7G2 (Life Technologies Corp.), antibody 4G4 (Abcam, product ref. ab81720), antibody AD2 (Biolegend Corp, product ref. 344004), antibody 1E9 (Santa Cruz Biotechnology Corp., product sc-32299), 067-213 antibody described in US 2014/0235833, the anti-CD73 antibody referenced in Sachsenmeier et al. ((2012) J. Biomed. Screening 17:993-998 and/or in Rust et al. (2013) Mol. Cancer 12:11, or antibody MEDI9447 (Medimmune Corp, Gaithersburg MD) referenced in Huang et al. (2015) AACR Annual meeting; Abstract 1538, or derivatives of the foregoing, e.g. that comprise the antigen binding region or heavy and/or light chain CDRs, in whole or in part. In other aspects, the above-mentioned antibodies may, depending on the nature of the antibody, be modified so as to have the characteristics of the antibodies of the present disclosure.

Once antibodies are identified that are capable of binding CD73 and/or having other desired properties, they will also typically be assessed, using standard methods including those described herein, for their ability to bind to other polypeptides, including unrelated polypeptides. Ideally, the antibodies only bind with substantial affinity to CD73, and do not bind at a significant level to unrelated polypeptides, or other polypeptides of the 5'-nucleotidase family. However, it will be appreciated that, as long as the affinity for CD73 is substantially greater (e.g., 10x, 100x, 500x, 1000x, 10,000x, or more) than it is for other, unrelated polypeptides), then the antibodies are suitable for use in the present methods.

In one aspect, the anti-CD73 antibodies can be prepared such that they do not have substantial specific binding to human Fcγ receptors, e.g., any one or more of CD16A, CD16B, CD32A, CD32B and/or CD64). Such antibodies may comprise constant regions of various heavy chains that are known not to bind Fcγ receptors. One such example is a wild type human IgG4 constant region (IgG4 have minimal Fcγ receptor binding). A human IgG4 constant region can further comprise a stabilizing S228P (S241P) substitution) to retain bivalent binding ability in vivo by preventing Fab arm exchange. Alternatively, antibody fragments that do not comprise (or comprise portions of) constant regions, such as F(ab')2 fragments, can be used to avoid Fc receptor binding. Fc receptor binding can be assessed according to methods known in the art, including for example testing binding of an antibody to Fc receptor protein in a BIACORE assay. Also, generally any antibody IgG isotype can be used in which the Fc portion is modified (e.g., by introducing 1, 2, 3, 4, 5 or more amino acid substitutions) to minimize or eliminate binding to Fc receptors (see, e.g., WO 03/101485). Assays such as cell based assays, to assess Fc receptor binding are well known in the art, and are described in, e.g., WO 03/101485.

In one aspect, the antibody can comprise one or more specific mutations in the Fc region that result in "Fc silent" antibodies that have minimal interaction with effector cells. Silenced effector functions can be obtained by mutation in the Fc region of the antibodies and have been described in the art: N297A mutation, the LALA mutations, (Strohl, W., 2009, Curr. Opin. Biotechnol. vol. 20(6):685-691); and D265A (Baudino et al., 2008, J. Immunol. 181 : 6664-69) see also Heusser et al., WO2012/065950. **In** one aspect, an antibody comprises one, two, three or more amino acid substitutions in the hinge region. In one aspect, the antibody is an IgG1 or IgG2 and comprises one, two or three substitutions at residues 233-236, optionally 233-238 (EU numbering). In one aspect, the antibody is an IgG4 and comprises one, two or three substitutions at residues 327, 330 and/or 331 (EU numbering). Examples of silent Fc IgG1 antibodies are the LALA mutant comprising L234A and L235A mutation in the IgG1 Fc amino acid sequence. Another example of an Fc silent mutation is a mutation at residue D265, or at D265 and P329 for example as used in an IgG1 antibody as the DAPA (D265A, P329A) mutation (US 6,737,056). Another silent IgG1 antibody comprises a mutation at residue N297 (e.g. N297A, N297S mutation), which results in aglycosylated/non-glycosylated antibodies. Other silent mutations include: substitutions at residues L234 and G237 (L234A/G237A); substitutions at residues S228, L235 and R409 (S228P/L235E/R409K,T,M,L); substitutions at residues H268, V309, A330 and A331 (H268Q/V309L/A330S/A331S); substitutions at residues C220, C226, C229 and P238 (C220S/C226S/C229S/P238S); substitutions at residues C226, C229, E233, L234 and L235 (C226S/C229S/E233P/L234V/L235A; substitutions at residues K322, L235 and L235 (K322A/L234A/L235A); substitutions at residues L234, L235 and P331 (L234F/L235E/P331S); substitutions at residues 234, 235 and 297; substitutions at residues E318, K320 and K322 (L235E/E318A/K320A/K322A); substitutions at residues (V234A, G237A, P238S); substitutions at residues 243 and 264; substitutions at residues 297 and 299; substitutions such that residues 233, 234, 235, 237, and 238 defined by the EU numbering system, comprise a sequence selected from PAAAP, PAAAS and SAAAS (see WO2011/066501).

Fc silent antibodies result in no or low ADCC activity, meaning that an Fc silent antibody exhibits an ADCC activity that is below 50% specific cell lysis. Preferably an antibody substantially lacks ADCC activity, e.g., the Fc silent antibody exhibits an ADCC activity (specific cell lysis) that is below 5% or below 1 %. Fc silent antibodies can also result in lack of FcγR-mediated cross-linking of CD73 at the surface of a CD73-expression.

In one aspect, the antibody has a substitution in a heavy chain constant region at any one, two, three, four, five or more of residues selected from the group consisting of: 220, 226, 229, 233, 234, 235, 236, 237, 238, 243, 264, 268, 297, 298, 299, 309, 310, 318, 320, 322, 327, 330, 331 and 409 (numbering of residues in the heavy chain constant region is according to EU numbering according to Kabat). In one aspect, the antibody comprises a substitution at residues 234, 235 and 322. In one aspect, the antibody has a substitution at residues 234, 235 and 331.

In one aspect, the Fc silent antibody comprises an Fc domain comprising an amino acid substitution at residues 234, 235 and 331, for example the "TM" mutation having substitutions L234F, L235E and P331S. In one aspect, the antibody comprises an Fc domain comprising an amino acid substitution at residues 234, 235 and 322, or at residues 234, 235 and 331, described in US Patent publication no. US2015/0125444, wherein residue 234 is F (phenylalanine); residue 235 is Alanine (A), Asparagine (N), Phenylalanine (F), Glutamine (Q), or Valine (V); residue 322 is Alanine (A), Aspartic acid (D), Glutamic acid (E), Histidine (H), Asparagine (N), or Glutamine (Q); and residue 331 is Alanine (A) or Glycine (G). Amino acid residues are indicated according to EU numbering according to Kabat.

In one aspect, the antibody comprises an Fc domain comprising an amino acid substitution that increases binding to human FcRn polypeptides in order to increase the in vivo half-life of the antibody. Exemplary mutations are described in Strohl, W., 2009, Curr. Opin. Biotechnol. vol. 20(6):685-691. Examples of substitutions used in antibodies of human IgG1 isotype are substitutions at residues M252, S254 and T256; substitutions at residues T250 and M428; substitutions at residue N434; substitutions at residues H433 and N434; substitutions at residues T307, E380 and N434; substitutions at residues T307, E380, and N434; substitutions at residues M252, S254, T256, H433, N434 and 436; substitutions at residue I253; substitutions at residues P257, N434, D376 and N434.

In one aspect, the antibody comprises an Fc domain comprising an amino acid substitution that confers decreased sensitivity to cleavage by proteases. Matrix metalloproteinases (MMPs) represent the most prominent family of proteinases associated with tumorigenesis. While cancer cells can express MMPs, the bulk of the extracellular MMP is provided by different types of stromal cells that infiltrate the tumor and each produce a specific set of proteinases and proteinase inhibitors, which are released into the extracellular space and specifically alter the milieu around the tumor. The MMPs present in the tumor microenvironment can cleave antibodies within the hinge region and may thus lead to the inactivation of therapeutic antibodies that are designed to function within the tumor site. In one aspect, the Fc domain comprising an amino acid substitution has decreased sensitivity to cleavage by any one, two, three or more (or all of) of the proteases selected from the group consisting of: GluV8, IdeS, gelatinase A (MMP2), gelatinase B (MMP-9), matrix metalloproteinase-7 (MMP-7), stromelysin (MMP-3), and macrophage elastase (MMP-12). In one aspect, the antibody decreased sensitivity to cleavage comprises an Fc domain comprising an amino acid substitution at residues E233-L234 and/or L235. In one aspect, the antibody comprises an Fc domain comprising an amino acid substitution at residues E233, L234, L235 and G236. In one aspect, the antibody comprises an Fc domain comprising an amino acid substitution at one or more residues 233-238, e.g., such that E233-L234-L235-G236 sequence is replaced by P233-V234-A235 (G236 is deleted). See, e.g., WO99/58572 and WO2012087746.

An antigen-binding compound can at any desired stage be assessed for its ability to inhibit the enzymatic activity of CD73, notably to block the 5'-nucleotidase activity of CD73 and to reduce the production of adenosine by a CD73-expressing cell, and in turn restore the activity of and/or relieve the adenosine-mediated inhibition of lymphocytes.

The ability of an antibody to inhibit the enzymatic activity of CD73 can be tested in a cell-free assay using recombinant soluble human CD73 (as dimers) and AMP, where conversion of AMP to adenosine (and/or inhibition thereof) is detected directly (e.g. by measurement of substrates and products, i.e. AMP, adenosine and/or phosphate), or indirectly. In one example, AMP and/or adenosine are detected via HPLC before and after incubation of the test compound with recombinant CD73. Recombinant CD73 is available, e.g., from R&D Systems (Minneapolis, MN).

The inhibitory activity (i.e. cytotoxicity enhancing potential) of an antibody can also be assessed in any of a number of other ways. For example, in an indirect assay, a luciferase-based reagent is used (e.g. CellTiter-Glo® system available from Promega), to detect the disappearance of AMP. The luciferase reaction in the assay is inhibited by AMP. Adding the CD73 enzyme to the reaction degrades the AMP, and relieves the inhibition, producing a detectable signal (see Example 2 herein).

The assays using soluble CD73 will be include testing at conditions where the antibodies are provided at a substantial molar excess (e.g. 10-fold, 20-fold, 50-fold, 100-fold, etc.) to the CD73 polypeptide dimers. When provided in molar excess to the enzyme, the anti-CD73 antibodies will no longer be capable of forming multimeric complexes of antibodies and CD73 dimers; antibodies that retain inhibition of the enzymatic activity of CD73 can then be selected.

The ability of an antibody to inhibit the 5'-ectonucletidase enzymatic activity of CD73 can alternatively or in addition also be tested in a cellular assay (using cells that express CD73). Advantageously, antibodies can be tested or screened first in the cell-free assay to identify antibodies that block the activity of the enzyme to reduce likelihood of selecting antibodies that inhibit CD73 by causing internalization of CD73, and then tested as purified antibody in cellular assays. Cellular assays can be carried out as shown in the Examples herein. For example, a CD73-expressing cell line (e.g. MDA-MB-231 cell line) are plated in flat-bottom 96 well plates in presence of anti-CD73 antibodies and incubated. AMP is added to the cells and incubated at 4°C (to avoid CD73 down-modulation). Plates are then centrifuged and supernatant is transferred to flat bottom 96 well culture plate. Free phosphate produced by the hydrolysis of AMP into adenosine is then quantified. A decrease in hydrolysis of AMP into adenosine in the presence of antibody indicate the antibody inhibits cellular CD73.

In one aspect, an antibody preparation causes at least a 50% decrease in the enzymatic activity of a CD73 polypeptide, preferably at least a 60%, 70% or 80% decrease in the enzymatic activity of a CD73 polypeptide (e.g. a soluble homodimeric CD73 polypeptide; CD73 expressed by cells).

The activity of an antibody can also be measured in an indirect assay for its ability to modulate the activity of lymphocytes, for example to relieve the adenosine-mediated inhibition of lymphocyte activity, or to cause the activation of lymphocyte activity. This can be addressed, for example, using a cytokine-release assay. In another example, an antibody can be evaluated in an indirect assay for its ability to modulate the proliferation of lymphocytes.

The antibody can be tested for its ability to internalize or to induce down-modulation of CD73, e.g. whether by internalization or induction of CD73 shedding from the cell surface. Whether an anti-CD73 antibody internalizes upon binding CD73 on a mammalian cell, or whether a CD73 polypeptide undergoes intracellular internalization (e.g. upon being bound by an antibody) can be determined by various assays including those described in the experimental examples herein (e.g., Example 7). In other examples, to test internalization in vivo, the test antibody is labeled and introduced into a non-human animal known to have CD73 expressed on the surface of certain cells. The antibody can be radiolabeled or labeled with fluorescent or gold particles, for instance. Animals suitable for this assay include a non-human mammal such as a nude mouse that contains a human CD73-expressing tumor transplant or xenograft, or a mouse into which cells transfected with human CD73 have been introduced, or a transgenic mouse expressing the human CD73 transgene. Appropriate controls include animals that did not receive the test antibody or that received an unrelated antibody, and animals that received an antibody to another antigen on the cells of interest, which antibody is known to be internalized upon binding to the antigen. The antibody can be administered to the animal, e.g., by intravenous injection. At suitable time intervals, tissue sections of the animal can be prepared using known methods or as described in the experimental examples below, and analyzed by light microscopy or electron microscopy, for internalization as well as the location of the internalized antibody in the cell. For internalization in vitro, the cells can be incubated in tissue culture dishes in the presence or absence of the relevant antibodies added to the culture media and processed for microscopic analysis at desired time points. The presence of an internalized, labeled antibody in the cells can be directly visualized by microscopy or by autoradiography if radiolabeled antibody is used. Optionally, in microscopy, co-localization with a known polypeptide or other cellular component can be assessed; for example co-localization with endosomal/lysosomal marker LAMP-1 (CD107a) can provide information about the subcellular localization of the internalized antibody. Alternatively, in a quantitative biochemical assay, a population of cells comprising CD73-expressing cells are contacted in vitro or in vivo with a radiolabeled test antibody and the cells (if contacted in vivo, cells are then isolated after a suitable amount of time) are treated with a protease or subjected to an acid wash to remove un-internalized antibody on the cell surface. The cells are ground up and the amount of protease resistant, radioactive counts per minute (cpm) associated with each batch of cells is measured by passing the homogenate through a scintillation counter. Based on the known specific activity of the radiolabeled antibody, the number of antibody molecules internalized per cell can be deduced from the scintillation counts of the ground- up cells. Cells are "contacted" with antibody in vitro preferably in solution form such as by adding the cells to the cell culture media in the culture dish or flask and mixing the antibody well with the media to ensure uniform exposure of the cells to the antibody.

Antibody epitopes

In one aspect, the antibodies bind a common antigenic determinant present on both soluble CD73 and CD73 expressed at the cell surface.

In one aspect, the antibodies bind substantially the same epitope as antibody 11E1, 8C7, 3C12 or 6E1. In one aspect, the antibodies bind to an epitope of CD73 that at least partially overlaps with, or includes at least one residue in, the epitope bound by antibody 11E1, 8C7, 3C12 or 6E1. The residues bound by the antibody can be specified as being present on the surface of the of the CD73 polypeptide, e.g. in a CD73 polypeptide expressed on the surface of a cell. The amino acid residues on CD73 bound by the antibody can for example be selected from the group consisting of the residues listed in Table 1.

Binding of anti-CD73 antibody to cells transfected with CD73 mutants can be measured and compared to the ability of anti-CD73 antibody to bind wild-type CD73 polypeptide (e.g., SEQ ID NOS: 1 or 2). A reduction in binding between an anti-CD73 antibody and a mutant CD73 polypeptide (e.g., a mutant CD73 of Table 1) means that there is a reduction in binding affinity (e.g., as measured by known methods such FACS testing of cells expressing a particular mutant, or by Biacore testing of binding to mutant polypeptides) and/or a reduction in the total binding capacity of the anti- CD73 antibody (e.g., as evidenced by a decrease in Bmax in a plot of anti-CD73 antibody concentration versus polypeptide concentration). A significant reduction in binding indicates that the mutated residue is directly involved in binding to the anti-CD73 antibody or is in close proximity to the binding protein when the anti-CD73 antibody is bound to CD73.

In some aspects, a significant reduction in binding means that the binding affinity and/or capacity between an anti-CD73 antibody and a mutant CD73 polypeptide is reduced by greater than 40 %, greater than 50 %, greater than 55 %, greater than 60 %, greater than 65 %, greater than 70 %, greater than 75 %, greater than 80 %, greater than 85 %, greater than 90% or greater than 95% relative to binding between the antibody and a wild type CD73 polypeptide. In certain aspects, binding is reduced below detectable limits. In some aspects, a significant reduction in binding is evidenced when binding of an anti-CD73 antibody to a mutant CD73 polypeptide is less than 50% (e.g., less than 45%, 40%, 35%, 30%, 25%, 20%, 15% or 10%) of the binding observed between the anti-CD73 antibody and a wild-type CD73 polypeptide.

In some aspects, anti-CD73 antibodies are provided that exhibit significantly lower binding for a mutant CD73 polypeptide in which a residue in a segment comprising an amino acid residue bound by antibody 11E1, 8C7, 3C12 or 6E1 is substituted with a different amino acid. In one aspect, the mutant is a mutant selected from mutants 3 and 16-22 of Table 1, compared to binding to a wild-type CD73 polypeptide (e.g. the polypeptide of SEQ ID NO: 1).

Optionally an antibody that loses binding to one or more mutants of mutants 3 and/or 16-22 does not exhibit significantly lower binding for one or more other mutants CD73 polypeptides of Table 1, e.g., one or more (or all of) mutants 2, 5, 6 or 7.

In one aspect, the anti-CD73 antibodies have reduced binding to a CD73 polypeptide having a mutation at residue K136 (with reference to SEQ ID NO: 1); optionally, the mutant CD73 polypeptide has the mutation: K136A.

In one aspect, the anti-CD73 antibodies have reduced binding to a CD73 polypeptide having a mutation at a residue selected from the group consisting of: K97, E125, Q153 and K330 (with reference to SEQ ID NO: 1); optionally, the mutant CD73 polypeptide has the mutations: K97A, E125A, Q153A and/or K330A (e.g., K97A, E125A and K330A; K97A, E125A and/or Q153A).

In one aspect, the anti-CD73 antibodies have reduced binding to a CD73 polypeptide having a mutation at a residue selected from the group consisting of: A99, E129, K133, E134, and A135 (with reference to SEQ ID NO: 1); optionally, the mutant CD73 polypeptide has the mutations: A99S, E129A, K133A, E134N, and/or A135S.

In one aspect, the anti-CD73 antibodies bind an epitope on CD73 comprising residue K136 (with reference to SEQ ID NO: 1).

In one aspect, the anti-CD73 antibodies bind an epitope on CD73 comprising one, two, three or four of the residues selected from the group consisting of K97, E125, Q153 and K330 (with reference to SEQ ID NO: 1).

In one aspect, the anti-CD73 antibodies bind an epitope on CD73 comprising one, two, three, four or five of the residues selected from the group consisting of A99, E129, K133, E134, and A135 (with reference to SEQ ID NO: 1).

In one aspect, the anti-CD73 antibodies bind an epitope on CD73 comprising one, two, three, four, five or more of the residues selected from the group consisting of K97, A99, E125, E129, K133, E134, A135 and K136 (with reference to SEQ ID NO: 1). In one aspect, the anti-CD73 antibodies bind an epitope on CD73 comprising one, two, three, four, five or more of the residues selected from the group consisting of K97, A99, E125, E129, K133, E134, A135, K136, Q153 and K330 (with reference to SEQ ID NO: 1.

Antibody CDR Sequences

Antibody 11E1

The amino acid sequence of the heavy chain variable region of antibody 11E1 is listed as SEQ ID NO: 3, the amino acid sequence of the light chain variable region is listed as SEQ ID NO: 4. In a specific aspect, the disclosure provides an antibody that binds essentially the same epitope or determinant as monoclonal antibodies 11E1; optionally the antibody comprises the hypervariable region of antibody 11E1. In any of the aspects herein, antibody 11E1 can be characterized by

EP 3 259 288 B1

the amino acid sequences and/or nucleic acid sequences encoding it. In one aspect, the monoclonal antibody comprises the Fab or F(ab')$_2$ portion of 11E1. Also provided is a monoclonal antibody that comprises the heavy chain variable region of 11E1. According to one aspect, the monoclonal antibody comprises the three CDRs of the heavy chain variable region of 11E1. Also provided is a monoclonal antibody that further comprises the variable light chain variable region of 11E1 or one, two or three of the CDRs of the light chain variable region of 11E1. Optionally any one or more of said light or heavy chain CDRs may contain one, two, three, four or five or more amino acid modifications (e.g. substitutions, insertions or deletions). Optionally, provided is an antibody where any of the light and/or heavy chain variable regions comprising part or all of an antigen binding region of antibody 11E1 are fused to an immunoglobulin constant region of the human IgG type, optionally a human constant region, optionally a human IgG1, IgG2, IgG3 or IgG4 isotype, optionally further comprising an amino acid substitution to reduce effector function (binding to human Fcγ receptors).

In another aspect, the disclosure provides an antibody, wherein the antibody comprises: a HCDR1 region of 11E1 comprising an amino acid sequence as set forth in Table A-1, or a sequence of at least 4, 5, 6, 7, 8, 9 or 10 contiguous amino acids thereof, optionally wherein one or more of these amino acids may be substituted by a different amino acid; a HCDR2 region of 11E1 comprising an amino acid sequence as set forth in Table A-1, or a sequence of at least 4, 5, 6, 7, 8, 9 or 10 contiguous amino acids thereof, optionally wherein one or more of these amino acids may be substituted by a different amino acid; a HCDR3 region of 11E1 comprising an amino acid sequence as set forth in Table A-1, or a sequence of at least 4, 5, 6, 7, 8, 9 or 10 contiguous amino acids thereof, optionally wherein one or more of these amino acids may be substituted by a different amino acid; a LCDR1 region of 11E1 comprising an amino acid sequence as set forth in Table A-1, or a sequence of at least 4, 5, 6, 7, 8, 9 or 10 contiguous amino acids thereof, optionally wherein one or more of these amino acids may be substituted by a different amino acid; a LCDR2 region of 11E1 comprising an amino acid sequence as set forth in Table A-1, or a sequence of at least 4, 5, 6, 7, 8, 9 or 10 contiguous amino acids thereof, optionally wherein one or more of these amino acids may be substituted by a different amino acid; a LCDR3 region of 11E1 comprising an amino acid sequence as set forth in Table A-1, or a sequence of at least 4, 5, 6, 7, 8, 9 or 10 contiguous amino acids thereof, optionally wherein one or more of these amino acids may be deleted or substituted by a different amino acid. The HCDR1, 2, 3 and LCDR1, 2, 3 sequences can optionally be specified as all (or each, independently) being those of the Kabat numbering system (as indicated in Table A-1 for each CDR), those of the Chotia numbering system as indicated in Table A-1 for each CDR), those of the IMGT numbering system as indicated in Table A-1 for each CDR), or any other suitable numbering system.

**Table A-1**

| mAb | CDR definition | HCDR1 | | HCDR2 | | HCDR3 | |
|-----|----------------|-------|----------|-------|----------|-------|----------|
| | | SEQ ID | Sequence | SEQ ID | Sequence | SEQ ID | Sequence |
| 1E11 | Kabat | 5 | SYNMY | 8 | YIDPYNGGTSYNQKFKG | 11 | GYGNYKAWFAY |
| | Chotia | 6 | GYAFTSY | 9 | PYNG | 12 | YGNYKAWFA |
| | IMGT | 7 | GYAFTSYN | 10 | IDPYNGGT | 13 | ARGYGNYKAWFAY |

| mAb | CDR definition | LCDR1 | | LCDR2 | | LCDR3 | |
|-----|----------------|-------|----------|-------|----------|-------|----------|
| | | SEQ | Sequence | SEQ ID | Sequence | SEQ ID | Sequence |
| 1E11 | Kabat | 14 | KASQSVTNDVA | 17 | YASNRYT | 19 | QQDYSSLT |
| | Chotia | 15 | SQSVTND | 18 | YAS | 20 | DYSSL |
| | IMGT | 16 | QSVTND | 18 | YAS | 19 | QQDYSSLT |

Antibody 6E1

The amino acid sequence of the heavy chain variable region of antibody 6E1 is listed as SEQ ID NO: 21, the amino acid sequence of the light chain variable region is listed as SEQ ID NO: 22. In a specific aspect, the disclosure provides an antibody that binds essentially the same epitope or determinant as monoclonal antibodies 6E1; optionally the antibody comprises the hypervariable region of antibody 6E1. In any of the aspects herein, antibody 6E1 can be characterized by the amino acid sequences and/or nucleic acid sequences encoding it. In one aspect, the monoclonal antibody comprises the Fab or F(ab')$_2$ portion of 6E1. Also provided is a monoclonal antibody that comprises the heavy chain variable region of 6E1. According to one aspect, the monoclonal antibody comprises the three CDRs of the heavy

**EP 3 259 288 B1**

chain variable region of 6E1. Also provided is a monoclonal antibody that further comprises the variable light chain variable region of 6E1 or one, two or three of the CDRs of the light chain variable region of 6E1. Optionally any one or more of said light or heavy chain CDRs may contain one, two, three, four or five or more amino acid modifications (e.g. substitutions, insertions or deletions). Optionally, provided is an antibody where any of the light and/or heavy chain variable regions comprising part or all of an antigen binding region of antibody 6E1 are fused to an immunoglobulin constant region of the human IgG type, optionally a human constant region, optionally a human IgG1, IgG2, IgG3 or IgG4 isotype, optionally further comprising an amino acid substitution to reduce effector function (binding to human Fcγ receptors).

In another aspect, the disclosure provides an antibody, wherein the antibody comprises: a HCDR1 region of 6E1 comprising an amino acid sequence as set forth in Table A-2, or a sequence of at least 4, 5, 6, 7, 8, 9 or 10 contiguous amino acids thereof, optionally wherein one or more of these amino acids may be substituted by a different amino acid; a HCDR2 region of 6E1 comprising an amino acid sequence as set forth in Table A-2, or a sequence of at least 4, 5, 6, 7, 8, 9 or 10 contiguous amino acids thereof, optionally wherein one or more of these amino acids may be substituted by a different amino acid; a HCDR3 region of 6E1 comprising an amino acid sequence as set forth in Table A-2, or a sequence of at least 4, 5, 6, 7, 8, 9 or 10 contiguous amino acids thereof, optionally wherein one or more of these amino acids may be substituted by a different amino acid; a LCDR1 region of 6E1 comprising an amino acid sequence as set forth in Table A-2, or a sequence of at least 4, 5, 6, 7, 8, 9 or 10 contiguous amino acids thereof, optionally wherein one or more of these amino acids may be substituted by a different amino acid; a LCDR2 region of 6E1 comprising an amino acid sequence as set forth in Table A-2, or a sequence of at least 4, 5, 6, 7, 8, 9 or 10 contiguous amino acids thereof, optionally wherein one or more of these amino acids may be substituted by a different amino acid; a LCDR3 region of 6E1 comprising an amino acid sequence as set forth in Table A-2, or a sequence of at least 4, 5, 6, 7, 8, 9 or 10 contiguous amino acids thereof, optionally wherein one or more of these amino acids may be deleted or substituted by a different amino acid. The HCDR1, 2, 3 and LCDR1, 2, 3 sequences can optionally be specified as all (or each, independently) being those of the Kabat numbering system (as indicated in Table A-2 for each CDR), those of the Chotia numbering system as indicated in Table A-2 for each CDR), those of the IMGT numbering system as indicated in Table A-2 for each CDR), or any other suitable numbering system.

**Table A-2**

| mAb | CDR definition | HCDR1 | | HCDR2 | | HCDR3 | |
|---|---|---|---|---|---|---|---|
| | | SEQ ID | Sequence | SEQ ID | Sequence | SEQ ID | Sequence |
| 6E1 | Kabat | 5 | SYNMY | 23 | YIDPYNGGSSYNQKFKG | 25 | GYNNYKAWFAY |
| | Chotia | 6 | GYAFTSY | 9 | PYNG | 26 | YNNYKAWFA |
| | IMGT | 7 | GYAFTSYN | 24 | IDPYNGGS | 27 | ARGYNNYKAW FAY |

| mAb | CDR definition | LCDR1 | | LCDR2 | | LCDR3 | |
|---|---|---|---|---|---|---|---|
| | | SEQ | Sequence | SEQ ID | Sequence | SEQ ID | Sequence |
| 6E1 | Kabat | 14 | KASQSVTNDVA | 17 | YASNRYT | 19 | QQDYSSLT |
| | Chotia | 15 | SQSVTND | 18 | YAS | 20 | DYSSL |
| | IMGT | 16 | QSVTND | 18 | YAS | 19 | QQDYSSLT |

Antibody 3C12

The amino acid sequence of the heavy chain variable region of antibody 3C12 is listed as SEQ ID NO: 36, the amino acid sequence of the light chain variable region is listed as SEQ ID NO: 37. In a specific aspect, the disclosure provides an antibody that binds essentially the same epitope or determinant as monoclonal antibodies 3C12; optionally the antibody comprises the hypervariable region of antibody 3C12. In any of the aspects herein, antibody 3C12 can be characterized by the amino acid sequences and/or nucleic acid sequences encoding it. In one aspect, the monoclonal antibody comprises the Fab or F(ab')$_2$ portion of 3C12. Also provided is a monoclonal antibody that comprises the heavy chain variable region of 3C12. According to one aspect, the monoclonal antibody comprises the three CDRs of the heavy chain variable region of 3C12. Also provided is a monoclonal antibody that further comprises the variable light chain variable region of 3C12 or one, two or three of the CDRs of the light chain variable region of 3C12. Optionally any one or

23

more of said light or heavy chain CDRs may contain one, two, three, four or five or more amino acid modifications (e.g. substitutions, insertions or deletions). Optionally, provided is an antibody where any of the light and/or heavy chain variable regions comprising part or all of an antigen binding region of antibody 3C12 are fused to an immunoglobulin constant region of the human IgG type, optionally a human constant region, optionally a human IgG1, IgG2, IgG3 or IgG4 isotype, optionally further comprising an amino acid substitution to reduce effector function (binding to human Fcγ receptors).

In another aspect, the disclosure provides an antibody, wherein the antibody comprises: a HCDR1 region of 3C12 comprising an amino acid sequence as set forth in Table A-3, or a sequence of at least 4, 5, 6, 7, 8, 9 or 10 contiguous amino acids thereof, optionally wherein one or more of these amino acids may be substituted by a different amino acid; a HCDR2 region of 3C12 comprising an amino acid sequence as set forth in Table A-3, or a sequence of at least 4, 5, 6, 7, 8, 9 or 10 contiguous amino acids thereof, optionally wherein one or more of these amino acids may be substituted by a different amino acid; a HCDR3 region of 3C12 comprising an amino acid sequence as set forth in Table A-3, or a sequence of at least 4, 5, 6, 7, 8, 9 or 10 contiguous amino acids thereof, optionally wherein one or more of these amino acids may be substituted by a different amino acid; a LCDR1 region of 3C12 comprising an amino acid sequence as set forth in Table A-3, or a sequence of at least 4, 5, 6, 7, 8, 9 or 10 contiguous amino acids thereof, optionally wherein one or more of these amino acids may be substituted by a different amino acid; a LCDR2 region of 3C12 comprising an amino acid sequence as set forth in Table A-3, or a sequence of at least 4, 5, 6, 7, 8, 9 or 10 contiguous amino acids thereof, optionally wherein one or more of these amino acids may be substituted by a different amino acid; a LCDR3 region of 3C12 comprising an amino acid sequence as set forth in Table A-3, or a sequence of at least 4, 5, 6, 7, 8, 9 or 10 contiguous amino acids thereof, optionally wherein one or more of these amino acids may be deleted or substituted by a different amino acid. The HCDR1, 2, 3 and LCDR1, 2, 3 sequences can optionally be specified as all (or each, independently) being those of the Kabat numbering system (as indicated in Table A-3 for each CDR), those of the Chotia numbering system as indicated in Table A-3 for each CDR), those of the IMGT numbering system as indicated in Table A-3 for each CDR), or any other suitable numbering system.

**Table A-3**

| mAb | CDR definition | HCDR1 | | HCDR2 | | HCDR3 | |
|---|---|---|---|---|---|---|---|
| | | SEQ ID | Sequence | SEQ ID | Sequence | SEQ ID | Sequence |
| 3C12 | Kabat | 30 | SYNMN | 33 | YIDPYNGGSSYNLTFKG | 11 | GYGNYKAWFAY |
| | Chotia | 31 | GYAFASY | 9 | PYNG | 12 | YGNYKAWFA |
| | IMGT | 32 | GYAFASYN | 24 | IDPYNGGS | 13 | ARGYGNYKAWFAY |

| mAb | CDR definition | LCDR1 | | LCDR2 | | LCDR3 | |
|---|---|---|---|---|---|---|---|
| | | SEQ | Sequence | SEQ ID | Sequence | SEQ ID | Sequence |
| 3C12 | Kabat | 38 | KASQSVSNDVA | 17 | YASTRYT | 19 | QQDYSSLT |
| | Chotia | 34 | SQSVSND | 18 | YAS | 20 | DYSSL |
| | IMGT | 35 | QSVSND | 18 | YAS | 19 | QQDYSSLT |

Antibody 8C7

The amino acid sequence of the heavy chain variable region of antibody 8C7 is listed as SEQ ID NO: 28, the amino acid sequence of the light chain variable region is listed as SEQ ID NO: 29. In a specific aspect, the disclosure provides an antibody that binds essentially the same epitope or determinant as monoclonal antibodies 8C7; optionally the antibody comprises the hypervariable region of antibody 8C7. In any of the aspects herein, antibody 8C7 can be characterized by the amino acid sequences and/or nucleic acid sequences encoding it. In one aspect, the monoclonal antibody comprises the Fab or F(ab')$_2$ portion of 8C7. Also provided is a monoclonal antibody that comprises the heavy chain variable region of 8C7. According to one aspect, the monoclonal antibody comprises the three CDRs of the heavy chain variable region of 8C7. Also provided is a monoclonal antibody that further comprises the variable light chain variable region of 8C7 or one, two or three of the CDRs of the light chain variable region of 8C7. Optionally any one or more of said light or heavy chain CDRs may contain one, two, three, four or five or more amino acid modifications (e.g. substitutions, insertions or deletions). Optionally, provided is an antibody where any of the light and/or heavy chain variable regions comprising part or all of an antigen binding region of antibody 8C7 are fused to an immunoglobulin constant region of the human IgG type, optionally a human constant region, optionally a human IgG1, IgG2, IgG3 or IgG4 isotype, optionally further comprising an amino acid substitution to reduce effector function (binding to human Fcγ receptors).

In another aspect, the disclosure provides an antibody, wherein the antibody comprises: a HCDR1 region of 8C7 comprising an amino acid sequence as set forth in Table A-4, or a sequence of at least 4, 5, 6, 7, 8, 9 or 10 contiguous amino acids thereof, optionally wherein one or more of these amino acids may be substituted by a different amino acid; a HCDR2 region of 8C7 comprising an amino acid sequence as set forth in Table A-4, or a sequence of at least 4, 5, 6, 7, 8, 9 or 10 contiguous amino acids thereof, optionally wherein one or more of these amino acids may be substituted by a different amino acid; a HCDR3 region of 8C7 comprising an amino acid sequence as set forth in Table A-4, or a sequence of at least 4, 5, 6, 7, 8, 9 or 10 contiguous amino acids thereof, optionally wherein one or more of these amino acids may be substituted by a different amino acid; a LCDR1 region of 8C7 comprising an amino acid sequence as set forth in Table A-4, or a sequence of at least 4, 5, 6, 7, 8, 9 or 10 contiguous amino acids thereof, optionally wherein one or more of these amino acids may be substituted by a different amino acid; a LCDR2 region of 8C7 comprising an amino acid sequence as set forth in Table A-4, or a sequence of at least 4, 5, 6, 7, 8, 9 or 10 contiguous amino acids thereof, optionally wherein one or more of these amino acids may be substituted by a different amino acid; a LCDR3 region of 8C7 comprising an amino acid sequence as set forth in Table A-4, or a sequence of at least 4, 5, 6, 7, 8, 9 or 10 contiguous amino acids thereof, optionally wherein one or more of these amino acids may be deleted or substituted by a different amino acid. The HCDR1, 2, 3 and LCDR1, 2, 3 sequences can optionally be specified as all (or each, independently) being those of the Kabat numbering system (as indicated in Table A-4 for each CDR), those of the Chotia numbering system as indicated in Table A-4 for each CDR), those of the IMGT numbering system as indicated in Table A-4 for each CDR), or any other suitable numbering system.

**Table A-4**

| mAb | CDR definition | HCDR1 | | HCDR2 | | HCDR3 | |
|---|---|---|---|---|---|---|---|
| | | SEQ ID | Sequence | SEQ ID | Sequence | SEQ ID | Sequence |
| 8C7 | Kabat | 30 | SYNMN | 33 | YIDPYNGGSSYNLTFKG | 11 | GYGNYKAWFAY |
| | Chotia | 31 | GYAFASY | 9 | PYNG | 12 | YGNYKAWFA |
| | IMGT | 32 | GYAFASYN | 24 | IDPYNGGS | 13 | ARGYGNYKAWF AY |

| mAb | CDR definition | LCDR1 | | LCDR2 | | LCDR3 | |
|---|---|---|---|---|---|---|---|
| | | SEQ | Sequence | SEQ ID | Sequence | SEQ ID | Sequence |
| 8C7 | Kabat | 44 | KASQSVSNDVA | 17 | YASTRYT | 19 | QQDYSSLT |
| | Chotia | 34 | SQSVSND | 18 | YAS | 20 | DYSSL |
| | IMGT | 35 | QSVSND | 18 | YAS | 19 | QQDYSSLT |

In another aspect of any of the aspects herein, any of the CDRs 1, 2 and 3 of the heavy and light chains of 11E1, 8C7, 3C12 or 6E1 may be characterized by a sequence of at least 4, 5, 6, 7, 8, 9 or 10 contiguous amino acids thereof, and/or as having an amino acid sequence that shares at least 50%, 60%, 70%, 80%, 85%, 90% or 95% sequence identity with the particular CDR or set of CDRs listed in the corresponding SEQ ID NO.

**[0177]** In one aspect, an antibody may comprise a heavy chain comprising:

a a heavy chain CDR1 (HCDR1) comprising an amino acid sequence of SEQ ID NO: 38; and/or
b a heavy chain CDR2 (HCDR2) comprising an amino acid sequence of SEQ ID NO: 39 (or 40); and/or
c a heavy chain CDR3 (HCDR3) comprising an amino acid sequence of SEQ ID NO: 41.

In one aspect, an antibody may comprise a light chain comprising:

a a light chain CDR1 (LCDR1) comprising an amino acid sequence selected from SEQ ID NO: 42; and/or
b a light chain CDR2 (LCDR2) comprising an amino acid sequence of SEQ ID NO: 43; and/or
c a light chain CDR3 (LCDR3) comprising an amino acid sequence of SEQ ID NO: 19 or 20.

In any of the antibodies, e.g., 11E1, 8C7, 3C12 or 6E1, the specified variable region and CDR sequences may comprise sequence modifications, e.g. a substitution (1, 2, 3, 4, 5, 6, 7, 8 or more sequence modifications). In one aspect, a CDRs 1, 2 and/or 3 of the heavy and light chains comprises one, two, three or more amino acid substitutions, where the residue substituted is a residue present in a sequence of human origin. In one aspect, the substitution is a conservative modification. A conservative sequence modification refers to an amino acid modification that does not significantly affect or alter the binding characteristics of the antibody containing the amino acid sequence. Such conservative modifications include amino acid substitutions, additions and deletions. Modifications can be introduced into an antibody by standard techniques known in the art, such as site-directed mutagenesis and PCR-mediated mutagenesis. Conservative amino acid substitutions are typically those in which an amino acid residue is replaced with an amino acid residue having a side chain with similar physicochemical properties. Specified variable region and CDR sequences may comprise one, two, three, four or more amino acid insertions, deletions or substitutions. Where substitutions are made, preferred substitutions will be conservative modifications. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g. glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g. threonine, valine, isoleucine) and aromatic side chains (e.g., tyrosine, phenylalanine, tryptophan, histidine). Thus, one or more amino acid residues within the CDR regions of an antibody can be replaced with other amino acid residues from the same side chain family and the altered antibody can be tested for retained function (i.e., the properties set forth herein) using the assays described herein.

The sequences of the variable regions of the antibodies are listed in Table B below (if leader sequences are present any antibody chain can be specified to start at the amino acid position immediately following the end of the leader sequence). In any aspect herein, a VL or VH sequence can be specified or numbered so as to contain or lack a signal peptide or any part thereof.

**Table B**

| | SEQ ID NO: | Amino Acid Sequence |
|---|---|---|
| 11E1 VH | 3 | EIQLQQSGPELVKPGASVKVSCKASGYAFTSYNMYWVKQSHGKSLEWIG YIDPYNGGTSYNQKFKGKATLTVDKSSSTAYMHLNSLTSEDSAVYYCAR GYGNYKAWFAYWGQGTLVTVSA |
| 11E1 VL | 4 | DAVMTQTPKFLLVSAGDRVTITCKASQSVTNDVAWYQQKPGQSPKLLIY YASNRYTGVPDRFTGSGYGTDFTFTISTVQAEDLAVYFCQQDYSSLTFG AGTKLELK |
| 6E1 VH | 21 | EFQLQQSGPELVKPGASVKVSCKASGYAFTSYNMYWVKQSHGKRLEWIG YIDPYNGGSSYNQKFKGKATLTVDKSSSTAYMHLNNLTSEDSAVYYCAR GYNNYKAWFAYWGQGTLVTVSA |
| 6E1 VL | 22 | SIVMTQTPKFLLVSAGDRVTITCKASQSVTNDVAWYQQKPGQSPKLLIY YASNRYTGVPDRFTGSGYGTDFTFTISTMQAEDLAVYFCQQDYSSLTFG AGTKLELK |
| 8C7 VH | 28 | EVQLQQSGPELVKPGASVKVSCKASGYAFASYNMNWVKQSHGKSLDWIGYIDPY NGGSSYNLTFKGKATLTVDKSSTTAYMHLNSLTSEDSAVYYCARGYGNYKAWFA YWGQGTLVTVSAASTKGP |
| 8C7 VL | 29 | SIVMTPTPKFLLVSAGDRVTITCKASQSVSNDVAWYQQKPGQSPKLLIYYASTR YTGVPDRFTGSGYGTDFTFTISTVQAEDLAVYFCQQDYSSLTFGAGTKLELKRT VAAP |

(continued)

| | SEQ ID NO: | Amino Acid Sequence |
|---|---|---|
| 3C12 VH | 36 | QIQLQQSGPELVKPGASVKVSCKASGYAFASYNMNWVKQSHGKSLDWIGYIDPY NGGSSYNLTFKGKATLTVDKSSTTAYMHLNSLTSEDSAVYYCARGYGNYKAWFA YWGQGTLVTVSAASTKGP |
| 3C12 VL | 37 | DVVMTQTPKFLLVSAGDRVTITCKASQSVSNDVAWYQQKPGQSPKLLIYYASTR YTGVPDRFTGSGYGTDFTFTISTVQAEDLAVYFCQQDYSSLTFGAGTKLELKRT VAAP |

Fragments and derivatives of antibodies (which are encompassed by the term "antibody" or "antibodies" as used in this specification, unless otherwise stated or clearly contradicted by context) can be produced by techniques that are known in the art. "Fragments" comprise a portion of the intact antibody, generally the antigen binding site or variable region. Examples of antibody fragments include Fab, Fab', Fab'-SH, F (ab') 2, and Fv fragments; diabodies; any antibody fragment that is a polypeptide having a primary structure consisting of one uninterrupted sequence of contiguous amino acid residues (referred to herein as a "single-chain antibody fragment" or "single chain polypeptide"), including without limitation (1) single-chain Fv molecules (2) single chain polypeptides containing only one light chain variable domain, or a fragment thereof that contains the three CDRs of the light chain variable domain, without an associated heavy chain moiety and (3) single chain polypeptides containing only one heavy chain variable region, or a fragment thereof containing the three CDRs of the heavy chain variable region, without an associated light chain moiety; and multispecific (e.g. bispecific) antibodies formed from antibody fragments. Included, *inter alia,* are a nanobody, domain antibody, single domain antibody or a "dAb".

In certain aspects, the DNA of a hybridoma producing an antibody, can be modified prior to insertion into an expression vector, for example, by substituting the coding sequence for human heavy- and light-chain constant domains in place of the homologous non-human sequences (e.g., Morrison et al., PNAS pp. 6851 (1984)), or by covalently joining to the immunoglobulin coding sequence all or part of the coding sequence for a non-immunoglobulin polypeptide. In that manner, "chimeric" or "hybrid" antibodies are prepared that have the binding specificity of the original antibody. Typically, such non-immunoglobulin polypeptides are substituted for the constant domains of an antibody.

The antibody is humanized. "Humanized" forms of antibodies are specific chimeric immunoglobulins, immunoglobulin chains or fragments thereof (such as Fv, Fab, Fab', F (ab') 2, or other antigen-binding subsequences of antibodies) which contain minimal sequence derived from the murine immunoglobulin. For the most part, humanized antibodies are human immunoglobulins (recipient antibody) in which residues from a complementary-determining region (CDR) of the recipient are replaced by residues from a CDR of the original antibody (donor antibody) while maintaining the desired specificity, affinity, and capacity of the original antibody.

In some instances, Fv framework residues of the human immunoglobulin may be replaced by corresponding non-human residues. Furthermore, humanized antibodies can comprise residues that are not found in either the recipient antibody or in the imported CDR or framework sequences. These modifications are made to further refine and optimize antibody performance. In general, the humanized antibody will comprise substantially all of at least one, and typically two, variable domains, in which all or substantially all of the CDR regions correspond to those of the original antibody and all or substantially all of the FR regions are those of a human immunoglobulin consensus sequence. The humanized antibody optimally also will comprise at least a portion of an immunoglobulin constant region (Fc), typically that of a human immunoglobulin. For further details see Jones et al., Nature, 321, pp. 522 (1986); Reichmann et al, Nature, 332, pp. 323 (1988); Presta, Curr. Op. Struct. Biol., 2, pp. 593 (1992); Verhoeyen et Science, 239, pp. 1534; and U.S. Patent No. 4,816,567). Methods for humanizing the antibodies are well known in the art.

The choice of human variable domains, both light and heavy, to be used in making the humanized antibodies is very important to reduce antigenicity. According to the so-called "best-fit" method, the sequence of the variable domain of an antibody is screened against the entire library of known human variable-domain sequences. The human sequence which is closest to that of the mouse is then accepted as the human framework (FR) for the humanized antibody (Sims et al., J. Immunol. 151, pp. 2296 (1993); Chothia and Lesk, J. Mol. 196, 1987, pp. 901). Another method uses a particular framework from the consensus sequence of all human antibodies of a particular subgroup of light or heavy chains. The same framework can be used for several different humanized antibodies (Carter et al., PNAS 89, pp. 4285 (1992); Presta et al., J. Immunol., 151, p. 2623 (1993)).

It is further important that antibodies be humanized with retention of high affinity for CD73 and other favorable biological properties. To achieve this goal, according to one method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the consensus and import sequences so that the desired antibody characteristic, such as increased affinity for the target antigen (s), is achieved. In general, the CDR residues are directly and most substantially involved in influencing antigen binding. The FRs of a humanized antibody chain are derived from a human variable region having at least about 60% overall sequence identity, and preferably at least about 80% overall sequence identity, with the variable region of the nonhuman donor (e.g., a 11E1, 8C7, 3C12 or 6E1antibody). The humanized heavy and/or light chain variable region shares at least about 60%, 70% or 80% overall sequence identity with the respective heavy and/or light chain variable region of the nonhuman donor (e.g., a 11E1, 8C7, 3C12 or 6E1 antibody).

Another method of making "humanized" monoclonal antibodies is to use a XenoMouse (Abgenix, Fremont, CA) as the mouse used for immunization. A XenoMouse is a murine host according that has had its immunoglobulin genes replaced by functional human immunoglobulin genes. Thus, antibodies produced by this mouse or in hybridomas made from the B cells of this mouse, are already humanized. The XenoMouse is described in United States Patent No. 6,162,963.

Human antibodies may also be produced according to various other techniques, such as by using, for immunization, other transgenic non-human animals that have been engineered to express a human antibody repertoire (Jakobovitz et al., Nature 362 (1993) 255), or by selection of antibody repertoires using phage display methods. Such techniques are known to the skilled person and can be implemented starting from monoclonal antibodies as disclosed in the present application.

## Antibody Formulations

An anti-CD73 antibody can be incorporated in a pharmaceutical formulation comprising in a concentration from 1 mg/ml to 500 mg/ml, wherein said formulation has a pH from 2.0 to 10.0. The formulation may further comprise a buffer system, preservative(s), tonicity agent(s), chelating agent(s), stabilizers and surfactants. In one aspect, the pharmaceutical formulation is an aqueous formulation, i.e., formulation comprising water. Such formulation is typically a solution or a suspension. In a further aspect, the pharmaceutical formulation is an aqueous solution. The term "aqueous formulation" is defined as a formulation comprising at least 50 %w/w water. Likewise, the term "aqueous solution" is defined as a solution comprising at least 50 %w/w water, and the term "aqueous suspension" is defined as a suspension comprising at least 50 %w/w water.

In another aspect, the pharmaceutical formulation is a freeze-dried formulation, whereto the physician or the patient adds solvents and/or diluents prior to use.

In another aspect, the pharmaceutical formulation is a dried formulation (e.g. freeze-dried or spray-dried) ready for use without any prior dissolution.

In a further aspect, the pharmaceutical formulation comprises an aqueous solution of such an antibody, and a buffer, wherein the antibody is present in a concentration from 1 mg/ml or above, and wherein said formulation has a pH from about 2.0 to about 10.0.

In a another aspect, the pH of the formulation is in the range selected from the list consisting of from about 2.0 to about 10.0, about 3.0 to about 9.0, about 4.0 to about 8.5, about 5.0 to about 8.0, and about 5.5 to about 7.5.

In a further aspect, the buffer is selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethan, bicine, tricine, malic acid, succinate, maleic acid, fumaric acid, tartaric acid, aspartic acid or mixtures thereof. Each one of these specific buffers constitutes an alternative aspect.

In a further aspect, the formulation further comprises a pharmaceutically acceptable preservative. In a further aspect, the formulation further comprises an isotonic agent. In a further aspect, the formulation also comprises a chelating agent. In a further aspect, the formulation further comprises a stabilizer. In a further aspect, the formulation further comprises a surfactant. For convenience reference is made to Remington: The Science and Practice of Pharmacy, 19th edition, 1995.

It is possible that other ingredients may be present in the peptide pharmaceutical formulation. Such additional ingredients may include wetting agents, emulsifiers, antioxidants, bulking agents, tonicity modifiers, chelating agents, metal ions, oleaginous vehicles, proteins (e.g., human serum albumin, gelatine or proteins) and a zwitterion (e.g., an amino acid such as betaine, taurine, arginine, glycine, lysine and histidine). Such additional ingredients, of course, should not adversely affect the overall stability of the pharmaceutical formulation.

Pharmaceutical compositions containing an antibody may be administered to a patient in need of such treatment at several sites, for example, at topical sites, for example, skin and mucosal sites, at sites which bypass absorption, for example, administration in an artery, in a vein, in the heart, and at sites which involve absorption, for example, administration in the skin, under the skin, in a muscle or in the abdomen. Administration of pharmaceutical compositions may be through several routes of administration, for example, subcutaneous, intramuscular, intraperitoneal, intravenous, lingual, sublingual, buccal, in the mouth, oral, in the stomach and intestine, nasal, pulmonary, for example, through the bronchioles and alveoli or a combination thereof, epidermal, dermal, transdermal, vaginal, rectal, ocular, for examples through the conjunctiva, uretal, and parenteral to patients in need of such a treatment.

Suitable antibody formulations can also be determined by examining experiences with other already developed therapeutic monoclonal antibodies. Several monoclonal antibodies have been shown to be efficient in clinical situations, such as Rituxan (Rituximab), Herceptin (Trastuzumab) Xolair (Omalizumab), Bexxar (Tositumomab), Campath (Alemtuzumab), Zevalin, Oncolym and similar formulations may be used with the antibodies. For example, a monoclonal antibody can be supplied at a concentration of 10 mg/mL in either 100 mg (10 mL) or 500 mg (50 mL) single-use vials, formulated for IV administration in 9.0 mg/mL sodium chloride, 7.35 mg/mL sodium citrate dihydrate, 0.7 mg/mL polysorbate 80, and Sterile Water for Injection. The pH is adjusted to 6.5. In another aspect, the antibody is supplied in a formulation comprising about 20 mM Na-Citrate, about 150 mM NaCl, at pH of about 6.0.

## Diagnosis and treatment of malignancies

Methods of treating an individual, notably a human patient, using an anti-CD73 antibody as described herein are also provided for. In one aspect, the disclosure provides for the use of an antibody as described herein in the preparation of a pharmaceutical composition for administration to a human patient. Typically, the patient suffers from, or is at risk for, cancer.

For example, in one aspect, provided is a neutralizing anti-CD73 antibody for use for restoring or potentiating the activity of lymphocytes in a patient in need thereof. The antibody can be for example a human or humanized anti-CD73 antibody, which antibody reduces or abrogates the 5'-nucleotidase activity of human CD73. In one aspect, the use directed at increasing the activity of lymphocytes (e.g. T cells) in patients having a disease in which increased lymphocyte activity is beneficial or which is caused or characterized by immunosuppression, immunosuppressive cells, or, e.g., adenosine generated by CD4 T cells, CD8 T cells, B cells). The treatments will be particularly useful for example patients having a solid tumor in which it is suspected the tumor microenvironment (and CD73-mediated adenosine production therein) may contribute to lack of recognition by the immune system (immune escape). The tumor may, for example, be characterized by CD73-expressing immune cells, e.g., CD4 T cells, CD8 T cells, B cells.

More specifically, the antibodies and compositions are utilized for use for the treatment of a variety of cancers and other proliferative diseases. Because these methods operate by reducing adenosine that inhibits the anti-tumor activity of lymphocytes and possibly additionally by increasing ATP that can increase the anti-tumor activity of lymphocytes, they are applicable to a very broad range of cancers, including in particular solid tumors in which adenosine in the tumor microenvironment may play a strong role in suppressing the anti-tumor immune response. In one aspect, a human patient treated with an anti-CD73 antibody has liver cancer, bone cancer, pancreatic cancer, skin cancer, cancer of the head or neck, breast cancer, lung cancer, non- small cell lung cancer (NSCLC), castrate resistant prostate cancer (CRPC), melanoma, uterine cancer, colon cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancers including those induced by asbestos, hematologic malignancies including, for example, multiple myeloma, B-cell lymphoma, Hodgkin lymphoma/primary mediastinal B-cell lymphoma, non-Hodgkin's lymphomas, acute myeloid lymphoma, chronic myelogenous leukemia, chronic lymphoid leukemia, follicular lymphoma, diffuse large B-cell lymphoma, Burkitt's lymphoma, immunoblastic large cell lymphoma, precursor B -lymphoblastic lymphoma, mantle cell lymphoma, acute lymphoblastic leukemia, mycosis fungoides, anaplastic large cell lymphoma, T-cell lymphoma, and precursor T-lymphoblastic lymphoma, and any combinations of said cancers. The present disclosure is also applicable to treatment of metastatic cancers. Patients can be tested or selected for one or more of the above described clinical attributes prior to, during or after treatment.

In one aspect, the anti-CD73 antibody is to be administered an amount effective to achieve and/or maintain in an individual (e.g. for 1, 2, 3, 4 weeks, and/or until the subsequent administration of antigen binding compound) a blood concentration of at least the $EC_{50}$, optionally the $EC_{70}$, optionally substantially the $EC_{100}$, for neutralization of the enzymatic activity of CD73. In one aspect, the active amount of anti-CD73 antibody is an amount effective to achieve the $EC_{50}$, optionally the $EC_{70}$, optionally substantially the $EC_{100}$, for neutralization of the enzymatic activity of CD73 in an extravascular tissue of an individual. In one aspect, the active amount of anti-CD73 antibody is an amount effective to achieve (or maintain) in an individual the $EC_{50}$, optionally the $EC_{70}$, optionally substantially the $EC_{100}$, for inhibition of neutralize the enzymatic activity of CD73.

Optionally, in one aspect, in contrast to some antibodies that are directed to the depletion of CD73-expressing tumor cells by ADCC (which, e.g., can provide full efficacy at concentrations equal or substantially lower than that which provides receptor saturation), the anti-CD73 antibody is a pure blocker (no substantial Fcγ receptor-mediated activity) and is to be administered in an amount effective to neutralize the enzymatic activity of CD73 for a desired period of time, e.g. 1 week, 2 weeks, a month, until the next successive administration of anti-CD73 antibody.

In one aspect, the anti-CD73 antibody is to be administered in an amount effective to achieve and/or maintain (e.g. for 1, 2, 3, 4 weeks, and/or until the subsequent administration of anti-CD73 antibody) in an individual a blood concentration of at least the $EC_{50}$, optionally the $EC_{70}$, optionally substantially the $EC_{100}$, for inhibition of CD73-mediated catabolism of AMP to adenosine (e.g., by assessing neutralization of 5' ectonucleotidase activity in MDA-MB-231 cells by quantifying hydrolysis of AMP to adenosine, see Example 5). In one aspect, the amount of anti-CD73 antibody is an amount effective to achieve (or maintain) the $EC_{50}$, optionally the $EC_{70}$, optionally substantially the $EC_{100}$, for inhibition of CD73-mediated catabolism of AMP to adenosine in an extravascular tissue of an individual.

In one aspect, provided is an anti-CD73 antibody for use for treating or preventing cancer in an individual, wherein the antibody is to be administered to an individual having disease in an amount that achieves or maintains for a specified period of time a concentration in circulation, optionally in an extravascular tissue of interest (e.g. the tumor or tumor environment), that is higher than the concentration required for 50%, 70%, or full (e.g. 90%) receptor saturation CD73-expressing cells in circulation (for example as assessed in PBMC). Optionally the concentration achieved is at least 20%, 50% or 100% higher than the concentration required for the specified receptor saturation.

In one aspect, provided is an anti-CD73 antibody for use for treating or preventing cancer in an individual, wherein the antibody is to be administered to the individual in an amount that achieves or maintains for a specified period of time a concentration in circulation, optionally in an extravascular tissue of interest (e.g. the tumor or tumor environment), that is higher than the $EC_{50}$, optionally $EC_{70}$ or optionally $EC_{100}$, for binding to CD73-expressing cells (e.g., as assessed by titrating anti-CD73 antibody on CD73-expressing cells, for example MDA-MB-231 cells as in Example 4). Optionally the concentration achieved is at least 20%, 50% or 100% higher than the $EC_{50}$, optionally $EC_{70}$ or optionally $EC_{100}$, for binding to CD73-expressing cells.

In any aspect, the antibody can for example have an $EC_{50}$, optionally $EC_{70}$ or optionally $EC_{100}$, for binding to CD73-expressing cells in human PBMC of between 0.5-100 ng/ml, optionally 1-100 ng/ml, optionally 30-100 ng/ml, e.g. about 30-90 ng/ml, (e.g., as assessed by titrating anti-CD73 antibody on CD73-expressing cells, for example MDA-MB-231 cells as in Example 4). For example the $EC_{50}$ may be about 30, 37, 39, 43, 57, 58, 61, 62, 90, 95, 143 ng/ml.

The $EC_{50}$ for neutralization of the enzymatic activity of CD73 with the anti-CD73 antibody can be for example between about 0.01 μg/ml and 1 μg/ml, optionally between 0.1 μg/ml and 10 μg/ml, optionally between 0.1 μg/ml and 1 μg/ml. For example the $EC_{50}$ may be about 0.1 μg/ml, about 0.2 μg/ml or about 0.3 μg/ml. Thus an amount of this anti-CD73 antibody is for example administered so at to achieve and/or maintain a blood concentration of at least 0.1 μg/ml, optionally at least 0.2 μg/ml, optionally at least 1 μg/ml, or optionally at least 2 μg/ml.

When tissues outside of the vasculature are targeted (the tumor environment, e.g., in the treatment of solid tumors), an approximately 10-fold higher dose is typically believed to be needed, compared to the dose that provides the corresponding concentration in circulation. An amount of anti-CD73 antibody administered so at to achieve (and/or maintain) a concentration in circulation (blood) of about 1 μg/ml, 2 μg/ml, 10 μg/ml, or 20 μg/ml is expected to achieve (and/or maintain) an extravascular tissue (e.g. tumor tissue) concentration of about 0.1 μg/ml, 0.2 μg/ml, 1 μg/ml, 2 μg/ml, respectively.

In one aspect, an anti-CD73 antibody is for example to be administered in an amount so at to achieve and/or maintain a tissue (e.g. tumor environment) concentration of at least 0.1 μg/ml, optionally at least 0.2 μg/ml, optionally at least 1 μg/ml, or optionally at least 2 μg/ml. The antibody can for example be administered in an amount to achieve and/or maintained a blood concentration of at least about 1 μg/ml, 2 μg/ml, 10 μg/ml, or 20 μg/ml, e.g. between 1-100 μg/ml, 10-100 μg/ml, 1-50 μg/ml, 1-20 μg/ml, or 1-10 μg/ml. The amount administered can be adjusted to as to provide for maintenance of the desired concentration for the duration of a specified period of time following administration (e.g. 1, 2, 3, 4 weeks, etc).

In some aspects, an amount of anti-CD73 antibody is to be administered so as to obtain a concentration in blood (serum) or an extravascular tissue (e.g. tumor environment) that corresponds to at least the $EC_{70}$ or the $EC_{100}$ for neutralization of the enzymatic activity of CD73. The antibody can for example be administered in an amount to achieve and/or maintained a blood concentration or an extravascular tissue (e.g. tumor environment) of at least about 1 μg/ml, 2 μg/ml, 10 μg/ml, or 20 μg/ml.

The $EC_{50}$, $EC_{70}$ or the $EC_{100}$ can be assessed for example in a cellular assay for neutralization of the enzymatic activity of CD73 as shown in the Examples herein (e.g. neutralization of 5' ectonucleotidase activity in MDA-MB-231 cells by quantifying hydrolysis of AMP to adenosine, see Example 5). "$EC_{50}$" with respect to neutralization of the enzymatic activity of CD73, refers to the efficient concentration of anti-CD73 antibody which produces 50% of its maximum response or effect with respect to neutralization of the enzymatic activity. ). "$EC_{70}$" with respect to neutralization of the enzymatic activity of CD73, refers to the efficient concentration of anti-CD73 antibody which produces 70% of its maximum response or effect. "$EC_{100}$" with respect to neutralization of the enzymatic activity of CD73, refers to the efficient concentration of anti-CD73 antibody which produces its substantially maximum response or effect with respect to such neutralization of the enzymatic activity.

In some aspects, particularly for the treatment of solid tumors, the concentration achieved is designed to lead to a concentration in tissues (outside of the vasculature, e.g. in the tumor or tumor environment) that corresponds to at least the $EC_{50}$ for neutralization of the enzymatic activity, optionally at about, or at least about, the $EC_{100}$.

In one aspect, the amount of anti-CD73 antibody is between 1 and 20 mg/kg body weight. In one aspect, the amount is to be administered to an individual weekly, every two weeks, monthly or every two months.

In one aspect, provided is an anti-CD73 antibody of the disclosure for use for treating a human individual having a cancer, wherein the antibody is to be administered to the individual in an effective amount of for at least one administration cycle (optionally at least 2, 3, 4 or more administration cycles), wherein the cycle is a period of eight weeks or less, wherein for each of the at least one cycles, one, two, three or four doses of the anti-CD73 antibody are administered at a dose of 1-20 mg/kg body weight. In one aspect, the anti-CD73 antibody is to be administered by intravenous infusion.

Suitable treatment protocols for treating a human include, for example, administering to the patient an amount as disclosed herein of an anti-CD73 antibody, wherein the method comprises at least one administration cycle in which at least one dose of the anti-CD73 antibody is to be administered. Optionally, at least 2, 3, 4, 5, 6, 7 or 8 doses of the anti-CD73 antibody are administered. In one aspect, the administration cycle is between 2 weeks and 8 weeks.

In one aspect, provided is an anti-CD73 antibody for use for treating or preventing a disease (e.g. a cancer, a solid tumor, a hematological tumor) in an individual, wherein the antibody is to be administered to an individual having disease (e.g. a cancer, a solid tumor) so that the anti-CD73 antibody neutralizes the enzymatic activity of CD73 for at least one administration cycle, the administration cycle comprising at least a first and second (and optionally a 3rd, 4th, 5th, 6th, 7th and/or 8th or further) administration of the anti-CD73 antibody, wherein the anti-CD73 antibody is to be administered in an amount effective to achieve, or to maintain between two successive administrations, a blood (serum) concentration of anti-CD73 antibody of at least 0.1 $\mu$g/ml, optionally at least 0.2 $\mu$g/ml, optionally at least 1 $\mu$g/ml, or optionally at least 2 $\mu$g/ml (e.g. for treatment of a hematological tumor), or optionally at least about 1 $\mu$g/ml, 2 $\mu$g/ml, 10 $\mu$g/ml, or 20 $\mu$g/ml, e.g. between 1-100 $\mu$g/ml, 1-50 $\mu$g/ml, 1-20 $\mu$g/ml, or 1-10 $\mu$g/ml (e.g. for treatment of a solid tumor, for treatment of a hematological tumor). In one aspect, a specified continuous blood concentration is maintained, wherein the blood concentration does not drop substantially below the specified blood concentration for the duration of the specified time period (e.g. between two administrations of antibody, number of weeks, 1 week, 2 weeks, 3 weeks, 4 weeks), i.e. although the blood concentration can vary during the specified time period, the specified blood concentration maintained represents a minimum or "trough" concentration. In one aspect, a therapeutically active amount of an anti-CD73 antibody is an amount of such antibody capable of providing (at least) the $EC_{50}$ concentration, optionally the $EC_{70}$ concentration optionally the $EC_{100}$ concentration, in blood and/or in a tissue for neutralization of the enzymatic activity of CD73 for a period of at least about 1 week, about 2 weeks, or about one month, following administration of the antibody.

Prior to or during a course of treatment with an anti-CD73 antibody of the disclosure, presence or levels or CD73-expressing cells, adenosine, ADP and/or AMP levels can be assessed within and/or adjacent to a patient's tumor to assess whether the patient is suitable for treatment (e.g. to predict whether the patient is likely to respond to treatment). Increased presence or levels or CD73-expressing cells, levels of adenosine, ADP and/or AMP may indicate an individual is suitable for treatment with (e.g. likely to benefit from) an anti-CD73 antibody of the disclosure (including but not limited to an antibody that inhibits substrate-bound CD73).

Prior to or during a course of treatment with an anti-CD73 antibody of the disclosure, adenosine, ADP and/or AMP levels can also be assessed within and/or adjacent to a patient's tumor to assess whether the patient is benefitting from treatment with an anti-CD73 antibody. Decreased levels of adenosine, ADP and/or AMP compared following an administration (or dosing of antibody) compared to levels prior to treatment (or dosing of antibody) may indicate an individual is benefitting from treatment with an anti-CD73 antibody of the disclosure (including but not limited to an antibody that inhibits substrate-bound CD73). Optionally, if a patient is benefitting from treatment with the anti-CD73 antibody, methods can further comprise administering a further dose of the anti-CD73 antibody to the patient (e.g., continuing treatment).

In one aspect, assessing adenosine, ADP and/or AMP levels within and/or adjacent to a patient's tumor the tissue sample comprises obtaining from the patient a biological sample of a human tissue selected from the group consisting of tissue from a cancer patient, e.g., cancer tissue, tissue proximal to or at the periphery of a cancer, cancer adjacent tissue, adjacent non-tumorous tissue or normal adjacent tissue, and detecting adenosine, ADP and/or AMP levels within the tissue. The levels from the patient can be comparing the level to a reference level, e.g. corresponding to a healthy individual.

In one aspect, the disclosure provides use for the treatment or prevention of a cancer in an individual in need thereof, the use comprising:

a) detecting CD73-expressing cells (or adenosine, ADP and/or AMP) the tumor environment, optionally within the tumor and/or within adjacent tissue, and

b) upon a determination that tumor environment comprises CD73-expressing cells (or adenosine, ADP and/or AMP), optionally at a level that is increased compared to a reference level, administering to the individual an anti-CD73 antibody. Optionally, detecting CD73-expressing cells (or adenosine, ADP and/or AMP) within the tumor environment comprises obtaining from the individual a biological sample that comprises cancer tissue and/or tissue proximal to or at the periphery of a cancer (e.g., cancer adjacent tissue, adjacent non-tumorous tissue or normal adjacent tissue), and detecting levels of CD73-expressing cells (or adenosine, ADP and/or AMP). CD73-expressing cells may comprise, for example, tumor cells, CD4 T cells, CD8 T cells, B cells.

A patient having a cancer can be treated with the anti-CD73 antibody with our without a prior detection step to assess expression of CD73 on cells in the tumor microenvironment (e.g. on tumor cells, CD4 T cells, CD8 T cells, B cells). Optionally, the use for treatment can comprises a step of detecting a CD73 nucleic acid or polypeptide in a biological sample of a tumor from an individual (e.g., in cancer tissue, tissue proximal to or at the periphery of a cancer, cancer adjacent tissue, adjacent non-tumorous tissue or normal adjacent tissue). A determination that a biological sample comprises cells expressing CD73 (e.g. prominently expressing; expressing CD73 at a high level, high intensity of staining with an anti-CD73 antibody, compared to a reference) indicates that the patient has a cancer that may have a strong benefit from treatment with an agent that inhibits CD73. In one aspect, the use comprises determining the level of expression of a CD73 nucleic acid or polypeptide in a biological sample and comparing the level to a reference level corresponding to a healthy individual. A determination that a biological sample comprises cells expressing CD73 nucleic acid or polypeptide at a level that is increased compared to the reference level indicates that the patient has a cancer that can be treated with an anti-CD73 antibody of the disclosure. Optionally, detecting a CD73 polypeptide in a biological sample comprises detecting CD73 polypeptide expressed on the surface of a malignant cell, a CD4 T cell, CD8 T cell, B cell. In one aspect, a determination that a biological sample comprises cells that prominently expresses CD73 nucleic acid or polypeptide indicates that the patients has a cancer that can be treated with an anti-CD73 antibody of the disclosure. "Prominently expressed", when referring to a CD73 polypeptide, means that the CD73 polypeptide is expressed in a substantial number of cells taken from a given patient. While the definition of the term "prominently expressed" is not bound by a precise percentage value, in some examples a receptor said to be "prominently expressed" will be present on at least 10%, 20% 30%, 40%, 50°%, 60%, 70%, 80%, or more of the tumor cells taken from a patient.

Determining whether an individual has a cancer characterized by cells that express a CD73 polypeptide can for example comprise obtaining a biological sample (e.g. by performing a biopsy) from the individual that comprises cells from the cancer environment (e.g. tumor or tumor adjacent tissue), bringing said cells into contact with an antibody that binds an CD73 polypeptide, and detecting whether the cells express CD73 on their surface. Optionally, determining whether an individual has cells that express CD73 comprises conducting an immunohistochemistry assay.

In one aspect, the disclosure provides a use for the treatment or prevention of a cancer in an individual in need thereof, the use comprising:

a) determining the CD73 polypeptide status of cells within the tumor environment, optionally within the tumor and/or within adjacent tissue, and

b) upon a determination that tumor environment comprises cells that express CD73 polypeptide, optionally at a level that is increased compared to a reference level, administering to the individual an anti-CD73 antibody. In one aspect, the cells are tumor cells. In another aspect, the cells within the tumor environment, tumor and/or adjacent tissue are non-malignant immune cells, e.g., T cells. Optionally, determining the CD73 polypeptide status within the tumor environment comprises obtaining from the individual a biological sample that comprises cancer tissue and/or tissue proximal to or at the periphery of a cancer (e.g., cancer adjacent tissue, adjacent non-tumorous tissue or normal adjacent tissue), bringing said cells into contact with an antibody that binds a CD73 polypeptide, and detecting cells that express CD73.

The antibody compositions may be used in as monotherapy or combined treatments with one or more other therapeutic agents, including agents normally utilized for the particular therapeutic purpose for which the antibody is being administered. The additional therapeutic agent will normally be administered in amounts and treatment regimens typically used for that agent in a monotherapy for the particular disease or condition being treated. Such therapeutic agents include, but are not limited to anti-cancer agents and chemotherapeutic agents.

In one aspect, the second or additional second therapeutic agent is an antibody or other Fc domain-containing protein capable of inducing ADCC toward a cell to which it is bound, e.g. via CD16 expressed by an NK cell. Typically, such antibody or other protein will comprise a domain that binds to an antigen of interest, e.g. an antigen present on a tumor cell (tumor antigen), and an Fc domain or portion thereof, and will exhibit binding to the antigen via the antigen binding domain and to Fcγ receptors (e.g. CD16) via the Fc domain. In one aspect, its ADCC activity will be mediated at least in part by CD16. In one aspect, the additional therapeutic agent is an antibody having a native or modified human Fc domain, for example a Fc domain from a human IgG1 or IgG3 antibody. The term "antibody-dependent cell-mediated cytotoxicity" or "ADCC" is a term well understood in the art, and refers to a cell-mediated reaction in which non-specific cytotoxic cells that express Fc receptors (FcRs) recognize bound antibody on a target cell and subsequently cause lysis of the target cell. Non-specific cytotoxic cells that mediate ADCC include natural killer (NK) cells, macrophages, monocytes, neutrophils, and eosinophils. The term "ADCC-inducing antibody" refers to an antibody that demonstrates ADCC as measured by assay(s) known to those of skill in the art. Such activity is typically characterized by the binding of the Fc region with various FcRs. Without being limited by any particular mechanism, those of skill in the art will recognize that the ability of an antibody to demonstrate ADCC can be, for example, by virtue of it subclass (such as IgG1 or IgG3), by mutations introduced into the Fc region, or by virtue of modifications to the carbohydrate patterns in the Fc region of the antibody.

In one aspect, the second or additional second therapeutic agent is an agent (e.g., an antibody) that inhibits CTLA-4 or the PD-1 axis (i.e. inhibits PD-1 or PD-L1). Antibodies that bind CTLA-4, PD1 or PD-L1 can be used, for example, at the exemplary the doses and/or frequencies that such agents are used as monotherapy, e.g., as described below.

PD-1 is an inhibitory member of the CD28 family of receptors that also includes CD28, CTLA-4, ICOS and BTLA. PD-1 is expressed on activated B cells, T cells, and myeloid cells Okazaki et al. (2002) Curr. Opin. Immunol. 14: 391779-82; Bennett et al. (2003) J Immunol 170:711-8). Two ligands for PD-1 have been identified, PD-L1 and PD-L2, that have been shown to downregulate T cell activation upon binding to PD-1 (Freeman et al. (2000) J Exp Med 192:1027-34; Latchman et al. (2001) Nat Immunol 2:261-8; Carter et al. (2002) Eur J Immunol 32:634-43). PD-L1 is abundant in a variety of human cancers (Dong et al. (2002) Nat. Med. 8:787-9). The interaction between PD-1 and PD-L1 results in a decrease in tumor infiltrating lymphocytes, a decrease in T-cell receptor mediated proliferation, and immune evasion by the cancerous cells. Immune suppression can be reversed by inhibiting the local interaction of PD-1 with PD-L1, and the effect is additive when the interaction of PD-1 with PD-L2 is blocked as well. Blockade of PD-1 can advantageously involve use of an antibody that prevents PD-L1-induced PD-1 signalling, e.g. by blocking the interaction with its natural ligand PD-L1. In one aspect the antibody binds PD-1 (an anti-PD-1 antibody); such antibody may block the interaction between PD-1 and PD-L1 and/or between PD-1 and PD-L2. In another aspect the antibody binds PD-L1 (an anti-PD-L1 antibody) and blocks the interaction between PD-1 and PD-L1.

There are currently at least six agents blocking the PD-1/PD-L1 pathway that are marketed or in clinical evaluation, any of these may be useful in combination with the anti-CD73 antibodies of the disclosure. One agent is BMS-936558 (Nivolumab/ONO-4538, Bristol-Myers Squibb; formerly MDX-1106). Nivolumab, (Trade name Opdivo®) is an FDA-approved fully human IgG4 anti-PD-L1 mAb that inhibits the binding of the PD-L1 ligand to both PD-1 and CD80 and is described as antibody 5C4 in WO 2006/121168. For melanoma patients, the most significant OR was observed at a dose of 3 mg/kg, while for other cancer types it was at 10 mg/kg. Nivolumab is generally dosed at 10 mg/kg every 3 weeks until cancer progression.

MK-3475 (human IgG4 anti-PD1 mAb from Merck), also referred to as lambrolizumab or pembrolizumab (Trade name Keytruda®) has been approved by the FDA for the treatment of melanoma and is being tested in other cancers. Pembrolizumab was tested at 2 mg/kg or 10 mg/kg every 2 or 3 weeks until disease progression. DNA constructs encoding the variable regions of the heavy and light chains of the humanized antibodies h409All have been deposited with the American Type Culture Collection Patent Depository (10801 University Blvd., Manassas, VA). The plasmid containing the DNA encoding the heavy chain of h409A-I 1 was deposited on June 9, 2008 and identified as 081469_SPD-H and the plasmid containing the DNA encoding the light chain of h409Al 1 was deposited on June 9, 2008 and identified as 0801470_SPD-L-I 1.

MPDL3280A/RG7446 (anti-PD-L1 from Roche/Genentech) is a human anti-PD-L1 mAb that contains an engineered Fc domain designed to optimize efficacy and safety by minimizing FcyR binding and consequential antibody-dependent cellular cytotoxicity (ADCC). Doses of ≤1, 10, 15, and 25 mg/kg MPDL3280A were administered every 3 weeks for up to 1 year. In phase 3 trial, MPDL3280A is administered at 1200 mg by intravenous infusion every three weeks in NSCLC.

AMP-224 (Amplimmune and GSK) is an immunoadhesin comprising a PD-L2 extracellular domain fused to an Fc domain.

Pidlizumab (CT-011; CureTech) (humanized IgG1 anti-PD1 mAb from CureTech/Teva), Pidlizumab (CT-011; CureTech) (see e.g., WO2009/101611) Thirty patients with rituximab-sensitive relapsed FL were treated with 3 mg/kg intravenous CT-011 every 4 weeks for 4 infusions in combination with rituximab dosed at 375 mg/m2 weekly for 4 weeks, starting 2 weeks after the first infusion of CT-011.

Further known PD-1 antibodies and other PD-1 inhibitors include AMP-224 (a B7-DC/IgG1 fusion protein licensed to GSK), AMP-514 described in WO 2012/145493, antibody MEDI-4736 (an anti-PD-L1 developed by AstraZeneca/Medimmune) described in WO2011/066389 and US2013/034559, antibody YW243.55.S70 (an anti-PD-L1) described in WO2010/077634, MDX-1105, also known as BMS-936559, is an anti-PD-L1 antibody developed by Bristol-Myers Squibb described in WO2007/005874, and antibodies and inhibitors described in WO2006/121168, WO2009/014708, WO2009/114335 and WO2013/019906. Further examples of anti-PD1 antibodies are disclosed in WO2015/085847 (Shanghai Hengrui Pharmaceutical Co. Ltd.), for example antibodies having light chain variable domain CDR1, 2 and 3 of SEQ ID NO: 6, SEQ ID NO: 7 and/or SEQ ID NO: 8, respectively, and antibody heavy chain variable domain CDR1, 2 and 3 of SEQ ID NO: 3, SEQ ID NO: 4 or SEQ ID NO: 5, respectively, wherein the SEQ ID NO references are the numbering according to WO2015/085847. Antibodies that compete with any of these antibodies for binding to PD-1 or PD-L1 also can be used.

CTLA-4 (cytotoxic T-lymphocyte-associated protein 4), also known as CD152 is another inhibitory member of the CD28 family of receptors, and is expressed on T cells. Antibodies that bind and inhibit CTLA-4 are known in the art. In one example, the antibody is ipilimumab (trade name Yervoy®, Bristol-Myers Squibb), a human IgG antibody. An exemplary administration regimen for Yervoy is 3 mg/kg invtravenously over 90 minutes every three weeks. In one example, the antibody used in combination with the anti-CD73 antibodies of the disclosure is an antibody that competes with ipilimumab for binding to CTLA-4.

In the treatment methods, the CD73-binding compound and the second therapeutic agent can be administered separately, together or sequentially, or in a cocktail. In some aspects, the antigen-binding compound is to be administered prior to the administration of the second therapeutic agent. For example, the CD73-binding compound can be administered approximately 0 to 30 days prior to the administration of the second therapeutic agent. In some aspects, an CD73-binding compound is to be administered from about 30 minutes to about 2 weeks, from about 30 minutes to about 1 week, from about 1 hour to about 2 hours, from about 2 hours to about 4 hours, from about 4 hours to about 6 hours, from about 6 hours to about 8 hours, from about 8 hours to 1 day, or from about 1 to 5 days prior to the administration of the second therapeutic agent. In some aspects, a CD73-binding compound is to be administered concurrently with the administration of the therapeutic agents. In some aspects, a CD73-binding compound is to be administered after the administration of the second therapeutic agent. For example, a CD73-binding compound can be administered approximately 0 to 30 days after the administration of the second therapeutic agent. In some aspects, a CD73-binding compound is to be administered from about 30 minutes to about 2 weeks, from about 30 minutes to about 1 week, from about 1 hour to about 2 hours, from about 2 hours to about 4 hours, from about 4 hours to about 6 hours, from about 6 hours to about 8 hours, from about 8 hours to 1 day, or from about 1 to 5 days after the administration of the second therapeutic agent.

## Examples

### Example 1: Generation of new anti-huCD73 antibodies

To obtain anti-human CD73 antibodies, Balb/c mice were immunized with a recombinant human CD73-His extracellular domain recombinant protein (cloned and produced at Innate Pharma as described below). Mice received one primo-immunization with an emulsion of 50 μg CD73 protein and Complete Freund Adjuvant, intraperitoneally, a 2nd immunization with an emulsion of 50 μg CD73 protein and Incomplete Freund Adjuvant, intraperitoneally, and finally a boost with 10 μg CD73 protein, intravenously. Immune spleen cells were fused 3 days after the boost with X63.Ag8.653 immortalized B cells, and cultured in the presence of irradiated spleen cells. Hydridomas were plated in semi-solid methylcellulose-containing medium and growing clones were picked using a clonepix 2 apparatus (Molecular Devices).

Primary screen: Supernatant (SN) of growing clones were tested in a primary screen by flow cytometry using parental and huCD73-, cynoCD73- or moCD73-expressing recombinant host cell lines. HuCD73- and cynoCD73-expressing recombinant host cells were stained with 0.35μM and 0.035μM CFSE, respectively. For the flow cytometry screening, all cells were equally mixed and the presence of reacting antibodies in supernanants was revealed by Goat anti-mouse polyclonal antibody (pAb) labeled with PE. 47 antibodies were found to bind both human and cynomolgus CD73. All antibodies that bound huCD73 and cynoCD73 were produced as chimeric human IgG1 antibodies with a heavy chain N297Q (Kabat EU numbering) mutation which results in lack of N-linked glycosylation and substantial lack of binding to Fcγ receptors.

Secondary screen: this informative screen (see Example 2) was done on recombinant CD73 protein to evaluate the CD73 enzymatic activity blockade properties of the 47-selected antibodies. 35/47 antibodies appear to completely or partially block CD73 activity.

Cloning, production and purification of recombinant huCD73

*Molecular Biology*

The huCD73 protein was cloned from MIAPACA-2 cDNA using the following primers TACGACTCACAAGCT TGCCGCCACCATGTGTCCCCGAGCCGCGCG (SEQ ID NO: 45) (Forward) and CCGCCCCGACTCTAGAtcaGTGA TGGTGATGATGGTGcttgatccgaccttcaactg SEQ ID NO: 46) (Reverse). The purified PCR product was then cloned into an expression vector using the InFusion cloning system. A 6xHis tag was added in the C-terminal part of the protein for the purification step.

*Amino acid sequence of the cloned huCD73:*

MCPRAARAPATLLLALGAVLWPAAGAWELTILHTNDVHS
RLEQTSEDSSKCVNASRCMGGVARLFTKVQQIRRAEPNVLLLD
AGDQYQGTIWFTVYKGAEVAHFMNALRYDAMALGNHEFDNGV
EGLIEPLLKEAKFPILSANIKAKGPLASQISGLYLPYKVLPVGDE
VVGIVGYTSKETPFLSNPGTNLVFEDEITALQPEVDKLKTLNVN
KIIALGHSGFEMDKLIAQKVRGVDVVVGGHSNTFLYTGNPPSKE
VPAGKYPFIVTSDDGRKVPVVQAYAFGKYLGYLKIEFDERGNVI
SSHGNPILLNSSIPEDPSIKADINKWRIKLDNYSTQELGKTIVYL

DGSSQSCRFRECNMGNLICDAMINNNLRHTDEMFWNHVSMCIL
NGGGIRSPIDERNNGTITWENLAAVLPFGGTFDLVQLKGSTLKK
AFEHSVHRYGQSTGEFLQVGGIHVVYDLSRKPGDRVVKLDVLC
TKCRVPSYDPLKMDEVYKVILPNFLANGGDGFQMIKDELLRHD
SGDQDINVVSTYISKMKVIYPAVEGRIKHHHHHH (SEQ ID NO: 2)

*Expression and purification of the huCD73 proteins*

After validation of the sequence cloned, cells were nucleofected and the producing pool was then sub-cloned to obtain a cell clone producing the huCD73 protein. Supernatant from the huCD73 clone grown in roller was harvested and purified using Ni-NTA column and eluted using 250 mM imidazole. The purified proteins were then loaded onto a S200 size exclusion chromatography column. The purified protein corresponding to a dimer was formulated in a Tris 20 mM pH 7.5, NaCl 120 mM and CaCl2 4 mM buffer for enzyme activity assays, while formulation buffer is supplemented with 20% glycerol.

**Example 2: Evaluation of soluble CD73 blockade**

The ability of anti-CD73 antibodies to block enzymatic activity of CD73 was evaluated as described in Sachsenmeier et al. (J Biomol Screening, 2012). Briefly, 500 ng/ml of recombinant human CD73-his were incubated in white 96W flat bottom microplates in presence of dose-range of anti-CD73 or isotype control Abs. Plates were incubated for 1h at 37°C. 12.5 $\mu$M ATP and 125$\mu$M AMP were added to each well and plates are incubated at 37°C for 30 supplemental minutes. Luciferase/luciferin-containing Cell Titer Glo (Promega) is added into wells, plates were incubated for 5 minutes at RT in the dark and emitted light is measured using an Enspire apparatus (Perkin Elmer).

Excess of AMP is known to block ATP-dependent luciferase activity. Addition of CD73 that cleaves AMP into adenosine + inorganic phosphate restores luciferase activity and light emission. Thus, antibodies that block enzymatic activity of CD73 will diminish light emission.

The percentage of enzyme inhibition is evaluated as described below:

- Conditions:

  ◦ ATP+AMP: maximal luciferase inhibition (100%)
  ◦ ATP+AMP+CD73: no luciferase inhibition (0%)

- Formula:

$$\text{Residual CD73 activity}: \frac{(CD73+Ab+ATP+AMP)-(ATP+AMP)}{(CD73+ATP+AMP)-(ATP+AMP)} * 100$$

35 antibodies obtained in Example 1, as well as reference mAbs 7G2, 4G4 and 1E9, were all found to inhibit CD73 activity using this experimental setting.

Considering the mixed results reported with reference antibodies, we considered whether CD73 blockade may arise from cross-linking of CD73 dimers by bivalent antibodies rather than true blockade of the enzymatic site. That is, antibodies may be causing oligomeric complexes of the CD73 dimers since bivalently binding mAbs may bind to two different CD73 homodimers, in turn leading to larger protein complexes). We then tested this possibility by performing blocking assays at high ratios of antibody:CD73 dimers. In this setting the mAbs are in large excess and induction of oligomeric complexes may occur, permitting true CD73 functional blockade to be observed. In this setting, practically all antibodies, including the reference mAbs 4G4 and 1E9, did not inhibit the enzymatic activity of CD73.

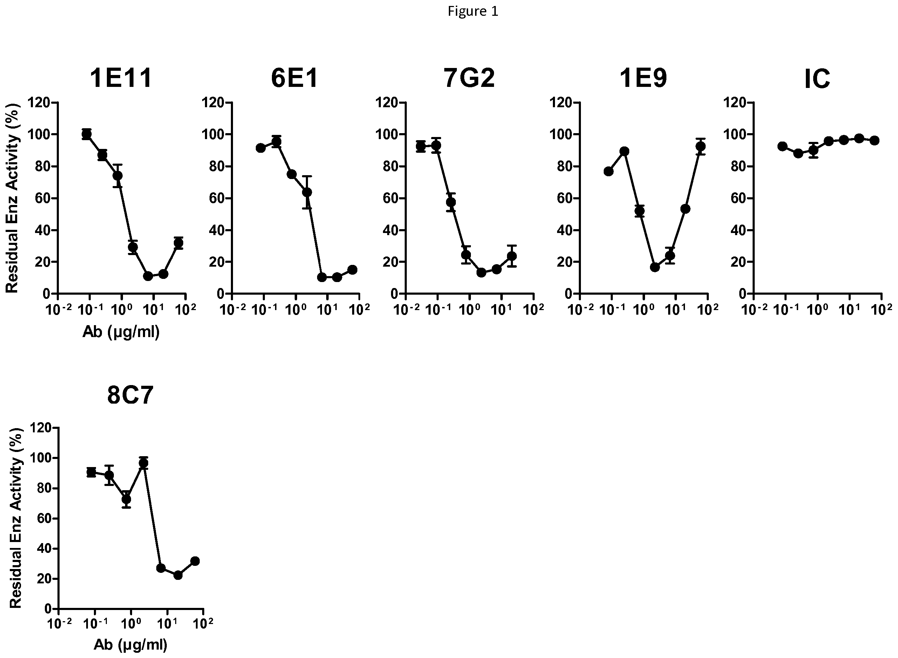

Antibodies 11E1, 8C7, 3C12 and 6E1 functionally blocked CD73 at all concentrations tested. Exemplary results are shown in Figure 1 for mAbs 11E1, 8C7 and 6E1. A further antibody (results not shown) was subsequently found to have low affinity for CD73 expressed on the surface of cells, thus possibly representing an epitope that is not suitable for high affinity binding to cell surface CD73. Available anti-CD73 reference antibodies reported in recent publications were also tested: 7G2, 4G4 and 1E9 were evaluated for CD73 blockade. Results (see Figure 1) showed that 7G2 blocked CD73 whereas 4G4 and 1E9 did not block CD73 activity, as residual enzyme activity rebounded to about the starting level or the negative control. Antibody AD2 was also tested and found not to block CD73 activity at any concentration. 4G4 and 1E9 are thus representative of the class of antibodies that non-specifically inhibit CD73 in this assay, possibly by causing oligomerization in solution. Thus, antibodies 7G2, 11E1, 8C7, 3C12 and 6E1 functionally blocked CD73 in this assay.

The $EC_{50}$ values for CD73 blockade are shown in the table below.

| Ab | EC50 ($\mu$g/ml) |
|---|---|
| 1E11 | 0.41 |
| 6E1 | 0.33 |
| 8C7 | 1.29 |
| 3C12 | 0.41 |

**Example 3: Ab titration on rec CD73 protein by ELISA**

Antibodies that functionally blocked soluble recombinant CD73 were more fully characterized for binding to soluble recombinant human CD73.

5 $\mu$g/ml of recombinant human CD73 protein (IPH, isoform E6) was coated on MaxiSorp ELISA plates (Nunc) in PBS, overnight at 4°C. Plates were washed 5 times in washing buffer (PBS, 0.05%Tween20) and unspecific sites were saturated by adding 200 $\mu$l/w TBS starting block buffer (Thermo Ficher). Dose-range of anti-CD73 antibodies were incubated for 2h at 37°C. Plates were washed 5 times in washing buffer and HRP-coupled goat anti-human or goat anti-mouse IgG Fc fragment secondary antibody (Bethyl, 1/50000) was added for 1hr at RT to detect bound anti-CD73 antibodies. Plates were washed 5 times in washing buffer and bound secondary antibody is revealed by adding TMB (HRP substrate) and incubating plates for 5 to 10 min at RT in the dark. The enzymatic reaction was stopped by adding HCl 1M and O.D. was measured at 450 nm. Optical density vs. anti-CD73 Ab concentration was plotted on graphs and EC50 is calculated using GraphPad Prism software.

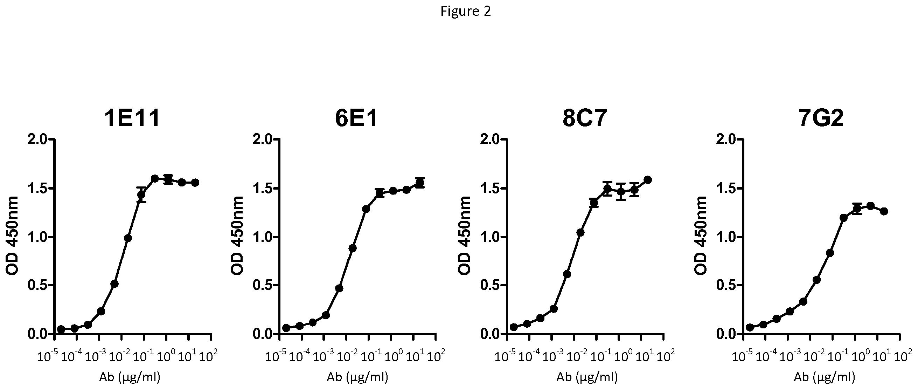

Results are shown in Figure 2. Antibodies, 11E1, 8C7, 6E1 and 7G2 all bound soluble recombinant CD73. $EC_{50}$ values for binding are shown in the table below.

| Antibody | EC50 ($\mu$g/ml) |
|---|---|
| 11E1 | 0.012 |
| 6E1 | 0.014 |
| 8C7 | 0.009 |
| 3C12 | 0.014 |
| 7G2 | 0.037 |

**Example 4: Flow cytometry titration**

Human-, cynomolgus- and mouse-CD73-expressing recombinant host cell lines or human MDA-MB-231 breast adenocarcinoma that endogenously expresses CD73 were used to evaluate ability of anti-CD73 antibodies to bind human CD73 and to cross-react on cynomolgus and/or mouse CD73. MDA-MB-231 cells are available from ATCC (reference HTB-26). $10^5$ cells resuspended in PBS/0.2% BSA/0.02% NaN3 (named "staining buffer") are distributed into round bottom 96W-microplates. Dose-range of anti-CD73 antibodies was added to the plates and cells are incubated for 45 min at 4°C. Cells were washed three times in staining buffer by spinning plates at 400 g for 3 min at 4°C. PE-coupled goat anti-mouse or goat anti-human IgG Fc fragment secondary antibodies (Beckman Coulter) diluted in staining buffer were added to the cells and plates are incubated for 30 additional minutes at 4°C. Cells were washed three times as described above and analyzed on an Accury C6 flow cytometer equipped with an HTFC plate reader.

Median of fluorescence vs. antibodies concentration was plotted on graphs and EC50 is calculated using GraphPad Prism program.

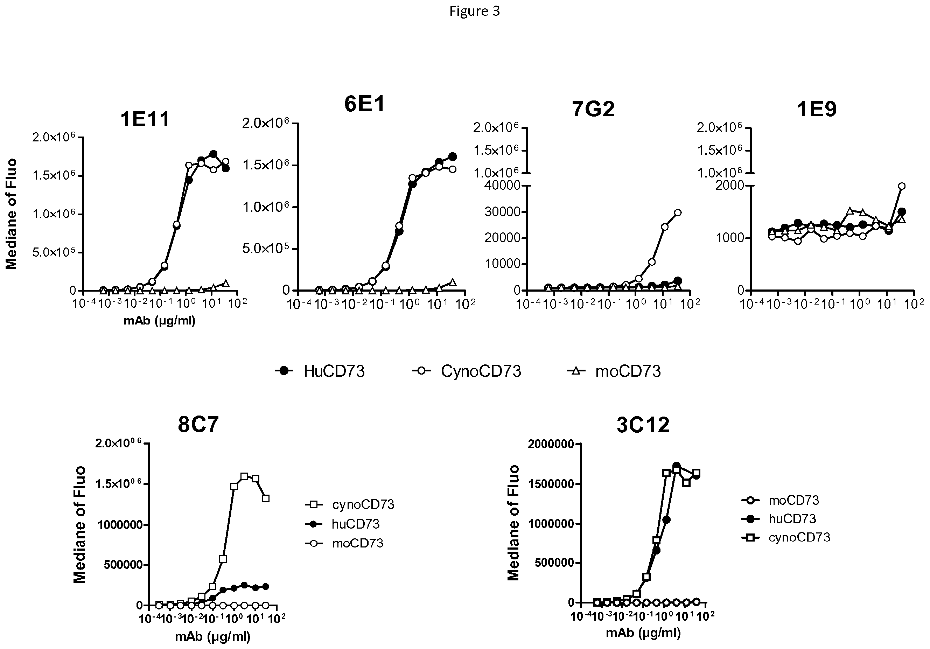

Results are shown in Figure 3 for new mAbs 11E1, 8C7, 3C12 and 6E1, as well as reference mAbs 7G2 and 1E9. mAbs 11E1, 8C7, 3C12 and 6E1 bind to recombinant host cells expressing human or cynomolgus (but not mouse) CD73 with excellent affinity. MAbs 7G2 and 1E9, however, show poor binding to cells expressing human or cynomolgus CD73.

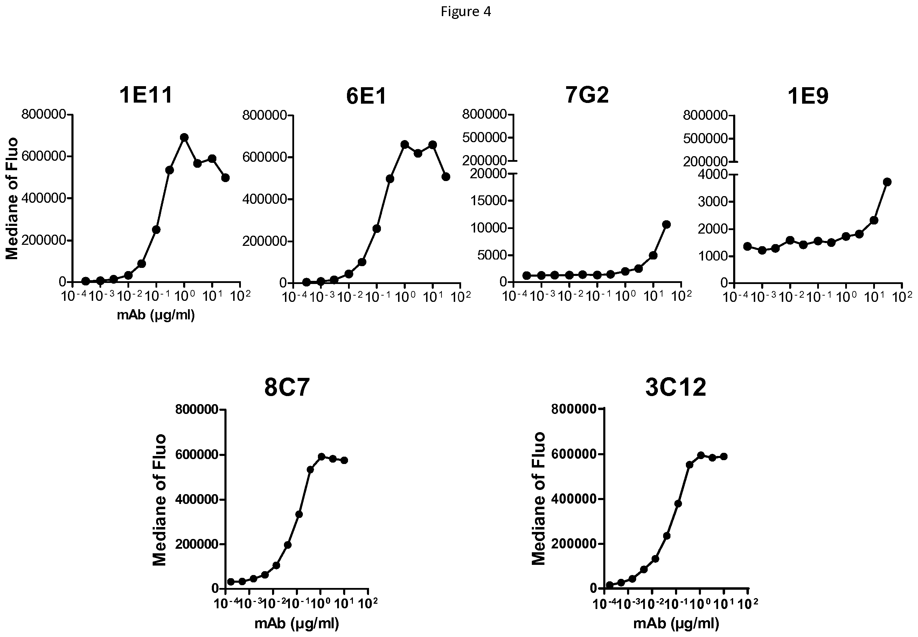

Experiments were repeated using human MDA-MB-231 breast adenocarcinoma cells that endogenously expresses CD73. Again mAbs 11E1, 8C7, 3C12 and 6E1 bind with excellent affinity while mAbs 7G2 and 1E9 show poor binding. Results on MDA-MB-231 cells are shown in Figure 4.

It is possible that 7G2 and 1E9 bind to an epitope on CD73 that is presented differently in recombinant CD73 and CD73 on the surface of the CD73-expressing cells, including notably human tumor cells that endogenously expresses CD73, resulting in mAbs that bind well to recombinant CD73 (e.g. used in immunization) but not to cell surface CD73. The epitopes bound by 11E1, 8C7, 3C12 and 6E1 on the other hand, remains present on cell surface CD73.

Experiments were then repeated using human MDA-MB-231 breast adenocarcinoma cells that endogenously expresses CD73. Cells were pre-incubated at 37 °C for 30 minutes in the presence or not of 200 µM adenosine 5'-($\alpha,\beta$-methylene)diphosphate (APCP), followed by addition of antibodies. APCP is an analog of ADP and binds irreversibly to the active site of CD73. When bound by APCP, CD73 changes conformation from an "open" conformation to a "closed" conformation. mAbs 11E1, 8C7, 3C12 and 6E1 bound MDA-MB-231 cells with good affinity both in the presence and absence of APCP. In the presence of APCP, the EC50 was similar to that observed in the absence of APCP. The plateau for maximal binding of 11E1, 8C7, 3C12 and 6E1 was higher in the absence of APCP than in the presence of APCP, while the inverse was true for AD2. EC50 figures are shown in the table below.

| | EC50 (ng/ml) | |
| --- | --- | --- |
| Antibody | Medium | APCP |
| 11E1 | 61.11 | 37.5 |
| 3C12 | 57.22 | 43.11 |
| 6E1 | 58.39 | 39.48 |
| 8C7 | 90.29 | 143.4 |
| AD2 | 62.89 | 95.92 |

**Example 5: Cellular CD73 activity blockade**

Part A: Blockade in MDA-MB-231 tumor cells

The ability of anti-CD73 antibodies to neutralize the 5'-ectonucletidase enzymatic activity of cell-surface expressed CD73 was evaluated. The MDA-MB-231 tumor cell line was used a model tumor cell line that expresses CD73.

All reagents used in the experiment detailed below were diluted in TBS pH7.5 (Tris 20mM pH7.5, NaCl 150mM). MDA-MB-231 cell line is recovered in PBS-EDTA and washed twice in TBS pH7.5. 0.5 to $1\times10^5$ cells were plated in flat-bottom 96 well plates in presence of dose-range of anti-CD73 antibodies and incubated for 2 hours at 4°C. 200mM AMP was added to the cells for a 30 minutes incubation period at 4°C (to avoid CD73 down-modulation). Plates were then centrifuged and 50 µl supernatant are transferred in flat bottom 96 well culture plate. Free phosphate produced by the hydrolysis of AMP into adenosine was quantified using the Malachite Green Phosphate Detection Kit (R&D Systems) and following TDS provided by the manufacturer. Phosphate concentration vs. anti-CD73 Ab concentration was plotted in graphs and EC50 is calculated using GraphPad Prism software.

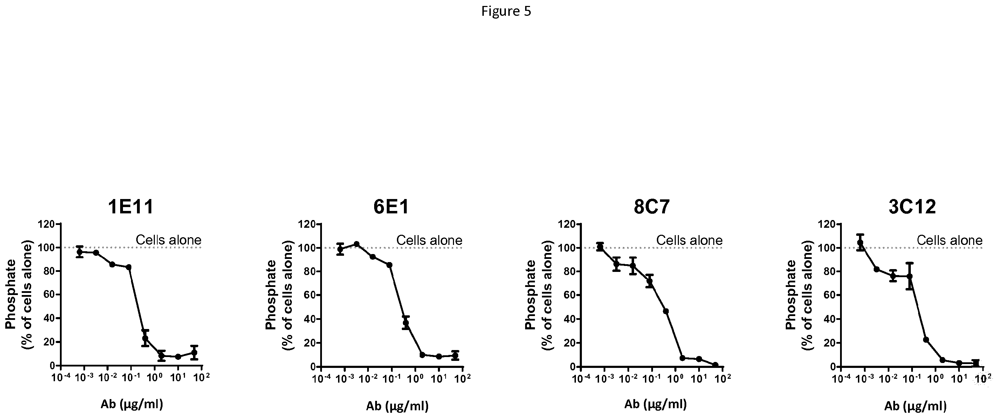

Results are shown in Figure 5. Antibodies 11E1, 8C7, 3C12 and 6E1 neutralized the enzymatic activity of cellular CD73, with $EC_{50}$ values shown in the table below.

| Ab | EC50 ($\mu$g/ml) |
|---|---|
| 1E11 | 0.195 |
| 6E1 | 0.209 |
| 8C7 | 0.319 |
| 3C12 | 0.210 |

Each antibody achieved a decrease of more than 75%, or more than 80% of the enzymatic activity. Antibody 7G2, consistent with poor binding to cellular CD73 (see Example 4), did not neutralize enzymatic activity of cellular CD73. Antibody 7G2, consistent with its limited ability to bind cell surface CD73, did not neutralize CD73 enzymatic activity at any concentration, and only elicited a slight partial inhibition at the highest concentrations tested.

In each case, for the non-internalizing antibodies 1E11, 6E1, 8C7 and 3C12, the $EC_{50}$ values required for neutralization of the enzymatic activity of cellular CD73 in MDA-MB-231 cell were several-fold higher than the $EC_{50}$ values required for binding to cell surface CD73 in MDA-MB-231 cells.

Part B: Blockade in MDA-MB-231, H292 and A375 tumor cells

The ability of anti-CD73 antibodies to neutralize the 5'-ectonucletidase enzymatic activity of cell-surface expressed CD73 was evaluated using the same assay as above, this time in multiple experiments using three tumor cell lines that express CD73lines in parallel: the MDA-MB-231 breast adenocarcinoma tumor cell line, the H292 lung cancer cell line (see, e.g., ATCC reference CRL-1848), and the A375 melanoma cancer cell line (see, e.g., ATCC reference CRL-1619). The effective concentrations required for neutralization (ECs, e.g. EC50, EC70, EC100) shown in the table below were calculated as a mean from repeated experiments on the three different CD73-expressing cell types.

| Antibody | 1E11 | 3C12 | 6E1 | 8C7 |
|---|---|---|---|---|
| N= | 8 | 8 | 8 | 2 |
| $EC_{50}$ ($\mu$g/ml) | 0.10 | 0.14 | 0.13 | 0.21 |
| $EC_{70}$ ($\mu$g/ml) | 0.15 | 0.18 | 0.19 | 0.46 |
| $EC_{100}$ ($\mu$g/ml) | 0.52 | 0.30 | 0.68 | 0.72 |

**Example 6: Flow cytometry competition study**

A human CD73-expressing recombinant host cell line was used to evaluate competition between our candidates and other commercial anti-CD73 antibodies. $10^5$ cells resuspended in staining buffer were distributed into round bottom 96W-microplates. A fixed dose of test antibodies (1$\mu$g/ml) is added to the cells in presence or not of a dose-range of reference mouse anti-human CD73 antibodies. Cells were incubated for 45 minutes at 4°C then washed three times as described above. PE-coupled or goat anti-human IgG Fc fragment secondary antibodies (Beckman Coulter) diluted in staining buffer were added to the cells and plates are incubated for 30 additional minutes at 4°C. Cells were washed three times and analyzed on an Accury C6 flow cytometer equipped with an HTFC plate reader.

Median of fluorescence vs. antibodies concentration was plotted. To study epitopes of CD73-neutralizing antibodies, the ability of known antibodies to block the binding of new antibodies to the cell membrane CD73 was evaluated as competition between antibodies. Reference antibody 7G2, shown in Example 2 to have the ability to neutralize CD73 without dependence upon induction of oligomerization, but which does not bind or neutralize CD73 in cellular assays, was tested with new antibody candidates. Neither antibodies 11E1, 8C7, 3C12 nor 6E1 competed with 7G2 for binding to CD73, showing that 7G2 binds to an area on CD73 distinct from that of the new antibodies.

**Example 7: CD73 down-modulation**

Human MDA-MB-231 breast adenocarcinoma cell line that endogenously expresses CD73 was used to evaluate the capacity of anti-CD73 antibodies to down regulate CD73 expression. $10^5$ cells resuspended in staining buffer were distributed into flat bottom 96W-microplates. 10 $\mu$g/ml of anti-CD73 antibodies were added to the cells and plates are incubated at 4°C or 37°C for a time course. At T=10min, 30min, 1h, 2h, 3h and 4h, cells were recovered using PSB/2mM EDTA, washed three times in staining buffer as prior described and incubated at 4°C until end of the time course. 10 μg/ml of a AlexaFLuor 647-coupled non-competing anti-CD73 antibody were added to the cells and plates are incubated for 30 min at 4°C. Cells were washed three times and analyzed on an Accury C6 flow cytometer equipped with an HTFC plate reader.

Percentage of expression vs. incubation time is plotted on graphs.

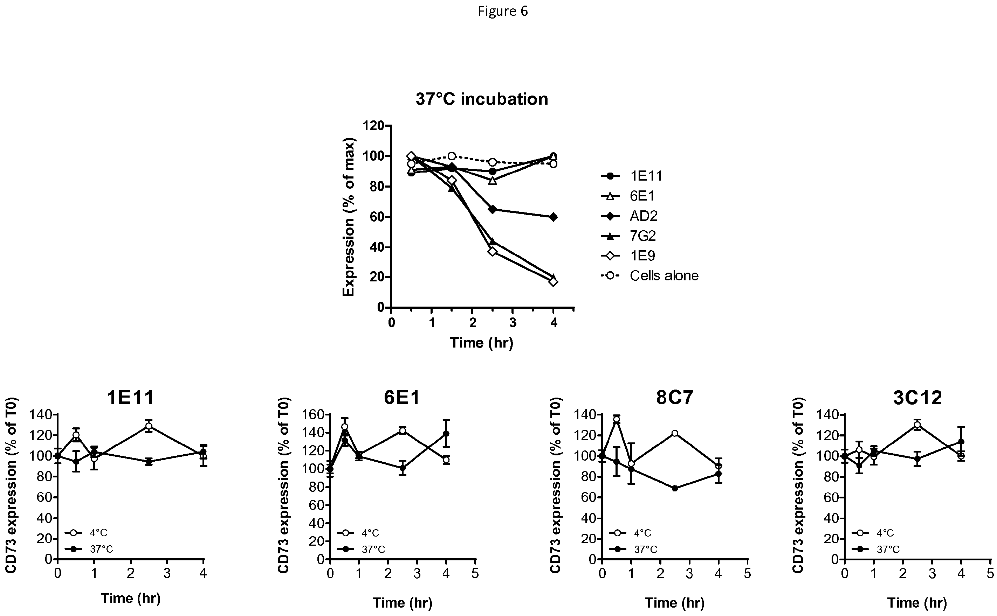

Antibodies were evaluated for their ability to cause down-modulation of CD73 expression on cells, and compared to reference mAbs AD2, 7G2 and 1E9. Each of AD2, 7G2 and 1E9 caused down-modulation of CD73, suggesting that these mAbs may be causing clustering and internalization of CD73. AD2 caused a decrease of well over 20% while 7G2 and 1E9 each caused a decrease in over 50% of receptor at the cell surface. Neither antibody 11E1, 8C7, 3C12 or 6E1 caused a decrease in CD73 at the cell surface. Results are shown in Figure 6.

## Example 8: Epitope mapping

In order to define the epitopes of anti-CD73 antibodies, we designed CD73 mutants defined by substitutions of amino acids exposed at the molecular surface over the surface of CD73. Mutants were transfected in Hek-293T cells, as shown in the table below. The targeted amino acid mutations in the table 1 below are shown using numbering of SEQ ID NO: 1.

Table 1

| Mutant | Substitutions | | | | | | |
|---|---|---|---|---|---|---|---|
| 1 | E46A | S49A | V52A | N53A | R56L | M58V | |
| 2 | Q70S | R73A | A74E | A107I | R109G | | |
| 3 | A99S | E129A | K133A | E134N | A135S | | |
| | K145A | K147A | S152H | S155A | Y161S | E203A | K206A |
| 4 5 | P165S | D168G | N211A | E296A | R297A | | |
| 6 | K179A | E196A | I197S | T198A | E224A | M225S | Q231A |
| 7 | K262A | F265S | I266A | K274Q | I292A | S302A | H304Y |
| 8 | P318A | S319A | K321A | N325A | K326Q | | |
| 9 | Y345A | D347A | S349A | S352A | D399A | R401A | |
| 10 | D460A | L461S | S462A | R463A | G466W | D467N | K471A |
| 11 | D473A | K478A | R480A | S483A | D485A | K488E | E491K |
| 12 | N503A | Q509A | K512S | D513A | | | |
| 13 | R354A | R395A | Q444A | T446A | | | |
| 14 | D332A | N333A | T336A | E409A | | | |
| 15 | H375A | E378A | R517A | S520A | D522A | | |
| 16 | A99S | E129A | K133A | E134N | A135S | K136A | L157S |
| | L159S | K162A | | | | | |
| 17 | A99S | E129A | K133A | E134N | A135S | K136A | |
| 18 | A99S | E129A | K133A | E134N | A135S | K136A | L157S |
| | L159S | K162A | A107S | R109A | | | |
| 19 | A99S | E129A | K133A | E134N | A135S | K136A | L157S |
| | L159S | K162A | S152A | S155A | | | |
| 20 | A107S | R109A | K136A | K162A | P165A | D111A | |
| 21 | A99S | E129A | K133A | E134N | A135S | K97A | E125A |
| | Q153A | | | | | | |
| 22 | A99S | E129A | K133A | E134N | A135S | K97A | E125A |
| | K330A | | | | | | |

Generation of mutants

CD73 mutants were generated by PCR. The sequences amplified were run on agarose gel and purified using the Macherey Nagel PCR Clean-Up Gel Extraction kit (reference 740609). The purified PCR products generated for each mutant were then ligated into an expression vector, with the ClonTech InFusion system. The vectors containing the mutated sequences were prepared as Miniprep and sequenced. After sequencing, the vectors containing the mutated sequences were prepared as Midiprep using the Promega PureYield™ Plasmid Midiprep System. HEK293T cells were grown in DMEM medium (Invitrogen), transfected with vectors using Invitrogen's Lipofectamine 2000 and incubated at 37°C in a CO2 incubator for 24 hours prior to testing for transgene expression.

Flow cytometry analysis of anti-CD73 binding to the HEK293T transfected cells

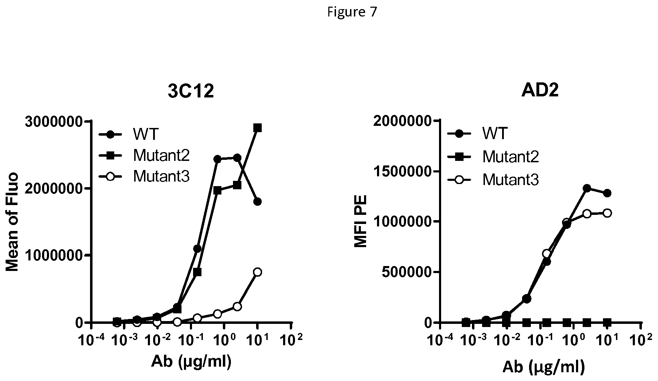

Anti-CD73 antibodies were tested for their binding to each of mutants 1-15 by flow cytometry. A first experiment was performed to determine antibodies that lose their binding to one or several mutants at one concentration. To confirm a loss of binding, titration of antibodies was done on antibodies for which binding seemed to be affected by the CD73 mutations (1 - 0.1 - 0.01 - 0.001 µg/ml). Antibodies 11E1, 8C7, 3C12 and 6E1 all lost binding to mutant 3 of CD73, but not to any other mutant. Mutant 3 contains amino acid substitutions at residues A99, E129, K133, E134, and A135, indicating that one or more, or all of, the residues of the mutant are important to the core epitope of these antibodies. Antibody AD2 that causes clustering and internalization of CD73 did not lose binding to mutant 3; AD2 instead lost binding to mutant 2 having substitutions at residues Q70, R73, A74, A107 and R109. Exemplary results for antibody 3C12 and AD2 are shown in Figure 7. Antibody 7G2 lost binding to mutants 5, 6 and 7 (but not mutants 2 or 3).

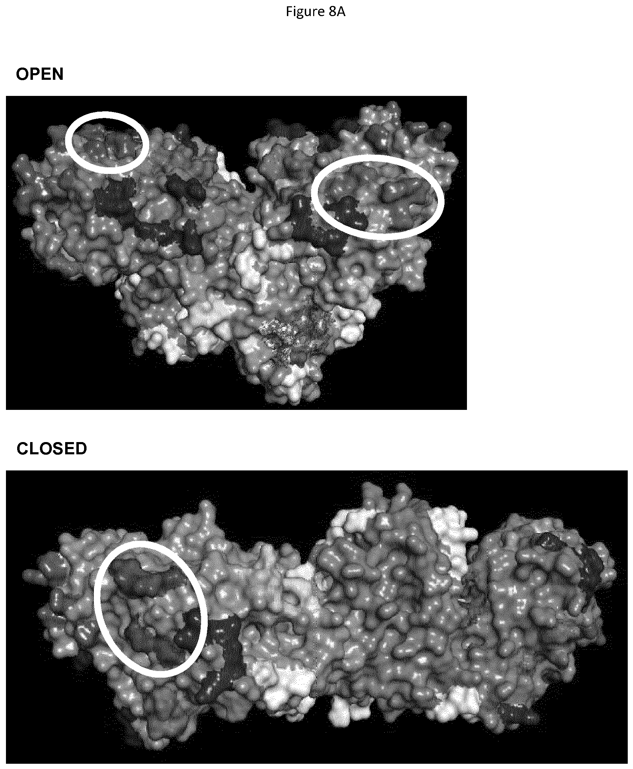

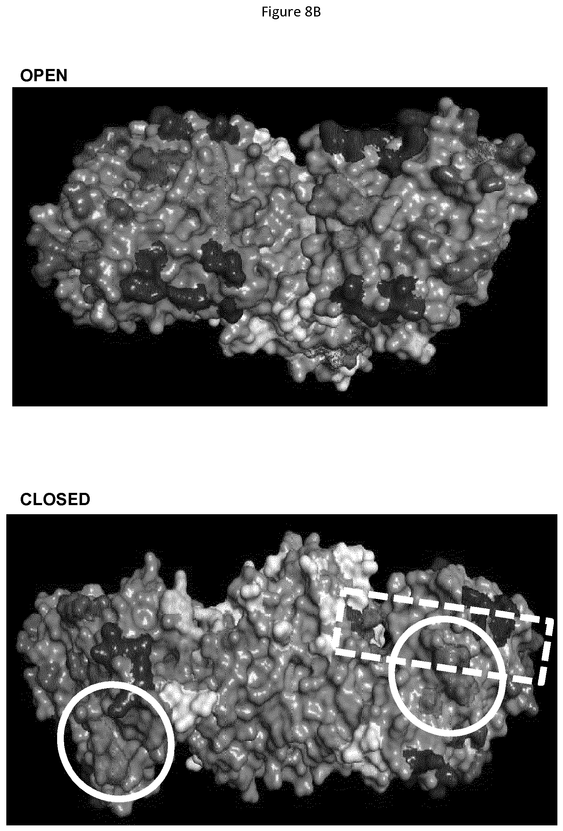

When bound by active site ligands as illustrated by the irreversibly ADP analog binder APCP, CD73 changes conformation from an "open" conformation to a "closed" conformation. As shown in Example 4, mAbs 11E1, 8C7, 3C12 and 6E1 bound cellular CD73 both in the presence and absence of APCP, indicating that the epitope of these antibodies remains present on CD73 when the active site is occupied. Figure 8A shows the molecular structure of the CD73 dimer, with amino acids mutated in mutant 2 (loss of binding by AD2) indicated (white circles) in both "open" or "closed" configurations. Figure 8B shows the molecular structure of the CD73 dimer, with amino acids mutated in mutant 3 (loss of binding by 11E1, 8C7, 3C12 or 6E1) indicated in both "open" or "closed" configurations. The active site is indicated by the box (dashed lines). Interestingly, it can be seen from Figure 8B that when the CD73 assumes a dimeric form, the amino acids mutated in mutant 3 are on a common face of the CD73 dimer, e.g., on or about on a plane (other epitopes of other mutants are not). Finally, from a comparison of the amino acids within mutant 3 one can see that these residues are relatively distant from the enzymatic active site (indicated in Figure 8B), consistent with a mode of action that involves allosteric inhibition of CD73.

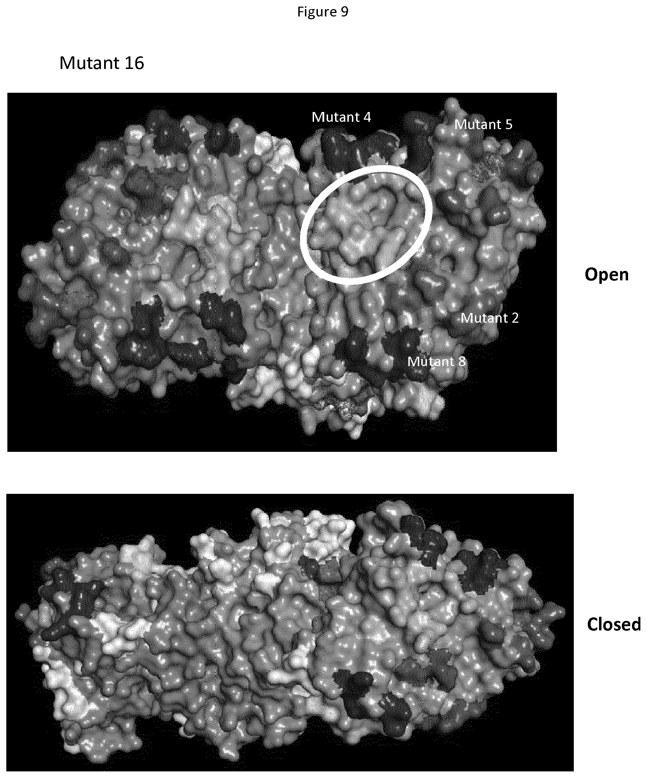

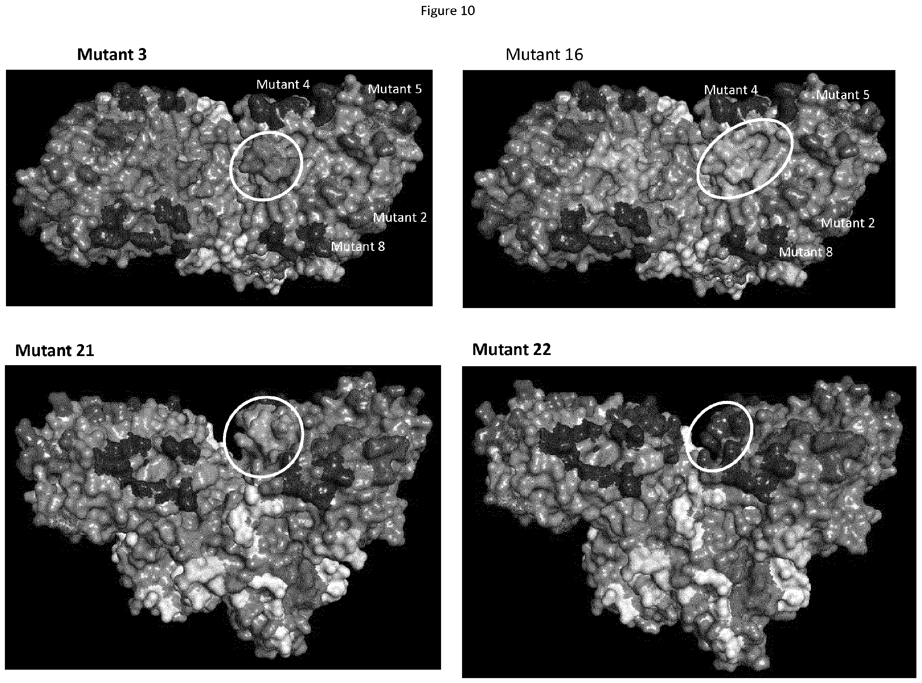

Several additional mutants were constructed with substitutions in the area adjacent to residues mutated in mutant 3. The mutants included notably mutated residues K136, A97, E125, Q153, and K330. A second series of binding assays was then conducted using the same methods as above for mutants 1-15, to assess binding of the antibodies 11E1, 3C12 and 6E1 to mutants 16-22 (see Table 1 above). Each antibody lost binding to mutants 16-22. While mutant 3 had a strong decrease in binding for the antibodies, when K136 was additionally mutated (in mutants 16-20), the resulting mutants had complete loss of binding. Thus, residue K136 is important to the core epitope of the antibodies. Furthermore, mutants 21 and 22 combined the mutations of mutant 3 with additional mutations at A97, E125, Q153, and K330. Mutants 21 and 22 had substitutions at residues A97 and E125, together with substitutions as Q153 or K330, which resulted in complete loss of binding to the antibodies, indicating that these residues are also within or proximal to epitope of these antibodies. Figure 9 shows the molecular structure of the CD73 dimer, with amino acids substituted in mutant 16 (loss of binding by 11E1, 3C12 and 6E1) indicated in both "open" and "closed" configurations. Figure 10 shows the molecular structure of the CD73 dimer in open configuration (from different viewpoints) for mutant 3 and for the additional mutants 16, 21 and 22 that included mutated residues at the border of the area mutated in mutant 3 (mutated area circled).

**Example 9: CD73 binding affinity by Surface Plasmon Resonance (SPR)**

Biacore T100 general procedure and reagents

SPR measurements were performed on a Biacore T100 apparatus (Biacore GE Healthcare) at 25°C. In all Biacore experiments HBS-EP+ (Biacore GE Healthcare) and NaOH 10mM served as running buffer and regeneration buffer respectively. Sensorgrams were analyzed with Biacore T100 Evaluation software. Protein-A was purchase from (GE Healthcare). Human soluble dimeric CD73 proteins were cloned, produced and purified at Innate Pharma.

Immobilization of Protein-A

Protein-A proteins were immobilized covalently to carboxyl groups in the dextran layer on a Sensor Chip CM5. The chip surface was activated with EDC/NHS (N-ethyl-N'-(3-dimethylaminopropyl) carbodiimide hydrochloride and N-hydroxysuccinimide (Biacore GE Healthcare). Protein-A was diluted to 10 µg/ml in coupling buffer (10 mM acetate, pH 5.6) and injected until the appropriate immobilization level was reached (i.e. 2000 RU). Deactivation of the remaining activated groups was performed using 100 mM ethanolamine pH 8 (Biacore GE Healthcare).

Affinity study

Affinity study was carried out according to a standard Capture-Kinetic protocol recommended by the manufacturer (Biacore GE Healthcare kinetic wizard). Serial dilutions of human recombinant soluble dimeric CD73 proteins, ranging from 1.23 to 300 nM were sequentially injected over the captured anti-CD73 antibodies and allowed to dissociate for 10 min before regeneration. The entire sensorgram sets were fitted using the 1:1 kinetic binding model. Bivalent affinities and kinetic association and dissociation rate constants are shown below in Table 2 below.

Table 2

| CD73 Ab | KD (nM) |
| --- | --- |
| 1E11 | 0.822 |
| 3C12 | 0.682 |
| 6E1 | 0.819 |

**Claims**

1. An antibody that specifically binds a human CD73 polypeptide at the surface of a cell and that is capable of neutralizing the 5'-ectonucleotidase activity thereof, as determined by assessing neutralization of 5' ectonucleotidase activity in MDA-MB-231 cells by quantifying hydrolysis of AMP to adenosine, wherein the antibody inhibits the activity of the human CD73 polypeptide without detectably reducing binding between the CD73 polypeptide and a substrate thereof, wherein the antibody is furthermore capable of neutralizing the 5'-ectonucleotidase activity of a soluble human CD73 polypeptide, wherein the antibody has reduced binding to a mutant CD73 polypeptide consisting of the amino acid of SEQ ID NO: 1, wherein the mutations A99S, E129A, K133A, E134N, A135S, and K136A have been introduced, relative to binding between the antibody and a wild-type CD73 polypeptide consisting of the amino acid sequence of SEQ ID NO: 1, wherein the antibody is humanized and comprises framework regions from a human variable region having at least 60% overall sequence identity with the variable regions of an antibody having respectively a VH and VL region of SEQ ID NOS: 3 and 4, or an antibody having respectively a VH and VL region of SEQ ID NOS: 28 and 29, or an antibody having respectively a VH and VL region of SEQ ID NOS: 36 and 37 or an antibody having respectively a VH and VL region of SEQ ID NOS: 21 and 22.

2. The antibody of claim 1, wherein the antibody specifically binds a human CD73 polypeptide without causing intracellular internalization of the CD73 polypeptide.

3. The antibody of any one of the above claims, wherein the antibody lacks binding, via an Fc domain, to the human CD16 polypeptide (FcγIII receptor).

4. The antibody of any one of the above claims, wherein the antibody binds a CD73 polypeptide dimer in bivalent manner.

5. The antibody of any one of the above claims, wherein the substrate is adenosine 5'-($\alpha,\beta$-methylene)diphosphate (APCP) or AMP.

6. The antibody of any one of the above claims, wherein the antibody is capable of neutralizing the 5'-ectonucleotidase activity of a soluble human dimeric CD73 polypeptide when the antibody is provided at a 10-fold or greater molar excess to CD73 polypeptide dimer.

7. The antibody of any one of the above claims, wherein the antibody is capable of causing a decrease in the 5'-ectonucleotidase activity of human cellular CD73 polypeptide by more than 70%.

8. The antibody of any one of the above claims, wherein the antibody is capable of causing a decrease in the 5'-ectonucleotidase activity of a human CD73 polypeptide in solution by more than 70%

9. A pharmaceutical composition comprising an antibody according to any one of the above claims, and a pharmaceutically acceptable carrier.

10. A nucleic acid encoding heavy and light chains of an antibody of any one of claims 1 to 8.

**11.** A hybridoma or recombinant host cell producing the antibody of any one of claims 1 to 8.

**12.** An antibody of any one of claims 1-8 or a composition of claim 9, for use in the treatment or prevention of a cancer in a patient in need thereof.

**Patentansprüche**

**1.** Ein Antikörper, der spezifisch ein humanes CD73-Polypeptid an der Oberfläche einer Zelle bindet und in der Lage ist, dessen 5'-Ectonukleotidase-Aktivität zu neutralisieren, bestimmt durch Bewertung der Neutralisierung der 5'-Ektonukleotidase-Aktivität in MDA-MB-231-Zellen durch Quantifizierung der Hydrolyse von AMP zu Adenosin, wobei der Antikörper die Aktivität des menschlichen CD73-Polypeptids hemmt, ohne die Bindung zwischen dem CD73-Polypeptid und einem Substrat davon nachweisbar zu verringern, wobei der Antikörper ferner in der Lage ist, die 5'-Ektonukleotidase-Aktivität eines löslichen humanen CD73-Polypeptids zu neutralisieren, wobei der Antikörper eine verringerte Bindung an ein mutiertes CD73-Polypeptid aufweist, das aus den Aminosäuren der SEQ ID NO: 1 besteht, wobei die Mutationen A99S, E129A, K133A, E134N, A135S und K136A eingeführt wurden, im Vergleich zur Bindung zwischen dem Antikörper und einem Wildtyp-CD73-Polypeptid, das aus der Aminosäuresequenz von SEQ ID NO: 1 besteht, wobei der Antikörper humanisiert ist und Framework-Regionen aus einer humanen variablen Region umfasst, die mindestens 60 % Gesamtsequenzidentität mit den variablen Regionen eines Antikörpers mit einer VH- und VL-Region der SEQ ID NO: 3 und 4 bzw. eines Antikörpers mit einer VH- und VL-Region der SEQ ID NO: 28 und 29, oder ein Antikörper mit einer VH- und einer VL-Region der SEQ ID NO: 36 und 37 oder ein Antikörper mit einer VH- und einer VL-Region der SEQ ID NO: 21 und 22 aufweisen.

**2.** Der Antikörper nach Anspruch 1, wobei der Antikörper spezifisch ein humanes CD73-Polypeptid bindet ohne intrazelluläre Internalisierung des CD73-Polypeptids zu verursachen.

**3.** Der Antikörper nach einem der obigen Ansprüche, wobei dem Antikörper die Bindung über eine Fc-Domäne and das humane CD16-Polypeptid (FcγIII Rezeptor) fehlt.

**4.** Der Antikörper nach einem der obigen Ansprüche, wobei der Antikörper ein CD73-Polypeptid-Dimer auf bivalente Weise bindet.

**5.** Der Antikörper nach einem der obigen Ansprüche, wobei das Substrat Adenosin 5'-($\alpha,\beta$-Methylen)diphosphat (APCP) oder AMP ist.

**6.** Der Antikörper nach einem der obigen Ansprüche, wobei der Antikörper in der Lage ist, die 5'-Ectonukleotidase-Aktivität eines löslichen, humanen, dimeren CD73-Polypeptids zu neutralisieren, falls der Antikörper in einem 10-fachen oder größeren molaren Überschuss für CD73-Polypeptid-Dimer bereitgestellt wird.

**7.** Der Antikörper nach einem der obigen Ansprüche, wobei der Antikörper in der Lage ist, eine Abnahme in der 5'-Ectonukleotidase-Aktivität des humanen zellulären CD73-Polypeptids um mehr als 70% zu verursachen.

**8.** Der Antikörper nach einem der obigen Ansprüche, wobei der Antikörper in der Lage ist, eine Abnahme in der 5'-Ectonukleotidase-Aktivität des humanen CD73-Polypeptids in Lösung um mehr als 70% zu verursachen.

**9.** Eine pharmazeutische Zusammensetzung umfassend einen Antikörper gemäß einem der obigen Ansprüche und einem pharmazeutisch verträglichen Trägerstoff.

**10.** Eine Nukleinsäure kodierend schwere und leichte Ketten eines Antikörpers nach einem der Ansprüche 1 bis 8.

**11.** Ein Hybridom oder eine rekombinante Wirtszelle, die den Antikörper nach einem der Ansprüche 1 bis 8 produziert.

**12.** Ein Antikörper nach einem der Ansprüche 1-8 oder eine Zusammensetzung nach Anspruch 9 zur Verwendung in the Behandlung oder Prävention eines Krebs in einem Patienten, der dies benötigt.

**Revendications**

1. Anticorps qui se lie spécifiquement à un polypeptide CD73 humain à la surface d'une cellule et qui est capable de neutraliser son activité de 5'-ectonucléotidase, comme déterminé par évaluation de la neutralisation de l'activité de 5' ectonucléotidase dans des cellules MDA-MB-231 par quantification de l'hydrolyse d'AMP en adénosine, lequel anticorps inhibe l'activité du polypeptide CD73 humain sans réduire de manière détectable la liaison entre le polypeptide CD73 et un substrat de celui-ci, lequel anticorps est en outre capable de neutraliser l'activité de 5'-ectonucléotidase d'un polypeptide CD73 humain soluble, lequel anticorps a une liaison réduite à un polypeptide CD73 mutant consistant en les acides aminés de la SEQ ID NO : 1, dans lequel les mutations A99S, E129A, K133A, E134N, A135S et K136A ont été introduites, par rapport à la liaison entre l'anticorps et un polypeptide CD73 de type sauvage consistant en la séquence d'acides aminés de la SEQ ID NO : 1, lequel anticorps est humanisé et comprend des régions de charpente provenant d'une région variable humaine ayant une identité de séquence globale d'au moins 60 % avec les régions variables d'un anticorps ayant respectivement des régions VH et VL des SEQ ID NO : 3 et 4, ou d'un anticorps ayant respectivement des régions VH et VL des SEQ ID NO : 28 et 29, ou d'un anticorps ayant respectivement des régions VH et VL des SEQ ID NO : 36 et 37, ou d'un anticorps ayant respectivement des régions VH et VL des SEQ ID NO : 21 et 22.

2. Anticorps selon la revendication 1, lequel anticorps se lie spécifiquement à un polypeptide CD73 humain sans provoquer d'internalisation intracellulaire du polypeptide CD73.

3. Anticorps selon l'une quelconque des revendications précédentes, lequel anticorps est dépourvu de liaison, via un domaine Fc, au polypeptide CD16 humain (récepteur FcγIII).

4. Anticorps selon l'une quelconque des revendications précédentes, lequel anticorps se lie à un dimère de polypeptide CD73 d'une manière bivalente.

5. Anticorps selon l'une quelconque des revendications précédentes, dans lequel le substrat est l'adénosine 5'-($\alpha$,$\beta$-méthylène)diphosphate (APCP) ou l'AMP.

6. Anticorps selon l'une quelconque des revendications précédentes, lequel anticorps est capable de neutraliser l'activité de 5'-ectonucléotidase d'un polypeptide CD73 dimèrique humain soluble quand l'anticorps est fourni en un excès molaire de 10 fois ou plus par rapport au dimère de polypeptide CD73.

7. Anticorps selon l'une quelconque des revendications précédentes, lequel anticorps est capable de provoquer une diminution de plus de 70 % de l'activité de 5'-ectonucléotidase du polypeptide CD73 cellulaire humain.

8. Anticorps selon l'une quelconque des revendications précédentes, lequel anticorps est capable de provoquer une diminution de plus de 70 % de l'activité de 5'-ectonucléotidase d'un polypeptide CD73 humain en solution.

9. Composition pharmaceutique comprenant un anticorps selon l'une quelconque des revendications précédentes, et un véhicule pharmaceutiquement acceptable.

10. Acide nucléique codant les chaînes lourde et légère d'un anticorps de l'une quelconque des revendications 1 à 8.

11. Hybridome ou cellule hôte recombinante produisant l'anticorps de l'une quelconque des revendications 1 à 8.

12. Anticorps selon l'une quelconque des revendications 1 à 8 ou composition selon la revendication 9, pour une utilisation dans le traitement ou la prévention d'un cancer chez un patient en ayant besoin.

Figure 1

EP 3 259 288 B1

Figure 2

Figure 3

Figure 4

Figure 5

Figure 6

Figure 7

EP 3 259 288 B1

**3C12**

**AD2**

Figure 8A

**OPEN**

**CLOSED**

Figure 8B

**OPEN**

**CLOSED**

Figure 9

Mutant 16

Open

Closed

Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5567610 A
- US 5229275 A
- US 5660827 A
- US 20140235833 A
- WO 03101485 A
- WO 2012065950 A, Heusser
- US 6737056 B
- WO 2011066501 A
- US 20150125444 A
- WO 9958572 A
- WO 2012087746 A
- US 4816567 A

- US 6162963 A
- WO 2006121168 A
- WO 2009101611 A
- WO 2012145493 A
- WO 2011066389 A
- US 2013034559 A
- WO 2010077634 A
- WO 2007005874 A
- WO 2009014708 A
- WO 2009114335 A
- WO 2013019906 A
- WO 2015085847 A

### Non-patent literature cited in the description

- **STAGG et al.** *Proc. Natl. Acad. Sci. USA*, 2010, vol. 104, 1547-1552
- **OHTA et al.** *Proc Natl Acad Sci USA*, 2006, vol. 103, 13132-13137
- **BEAVIS et al.** *Proc. Natl. Acad. Sci. USA*, 2013, vol. 110, 14711-716
- **JIN et al.** *Cancer Res.*, 2010, vol. 70, 2245-55
- **STAGG et al.** *Cancer Res.*, 2010, vol. 71, 2892-2900
- **LOI et al.** *Proc. Natl. Acad. Sci. USA*, 2013, vol. 110, 11091-11096
- **HÄUSLER et al.** *Am L Transl Res*, 2014, vol. 6, 129-139
- **AIRAS.** *Journal of Cell Biology*, 1997, vol. 136, 421-431
- **FLOCKE et al.** *European Journal of Cell Biology*, 1992, vol. 58, 62-70
- **GUTENSOHN et al.** *Cell Immunol.*, 1995, vol. 161, 213-217
- **SACHSENMEIER et al.** *J. Biomed. Screening*, 2012, vol. 17, 993-998
- **RUST et al.** *Mol. Cancer*, 2013, vol. 12, 11
- **HARLOW et al.** Antibodies: A Laboratory Manual. Cold Spring Harbor Laboratory Press, 1988
- Current Protocols in Immunology. Greene Publishing Assoc. and Wiley Interscience, 1992
- **MULLER.** *Meth. Enzymol.*, 1983, vol. 92, 589-601
- **GREEN et al.** *Nature Genet*, 1994, vol. 7, 13
- **LONBERG et al.** *Nature*, 1994, vol. 368, 856
- **TAYLOR et al.** *Int Immun*, 1994, vol. 6, 579

- **MCCAFFERTY et al.** *Nature*, 1990, vol. 348, 552-553
- **CHOTHIA ; LESK.** *J. Mol. Biol*, 1987, vol. 196, 901-917
- **KABAT et al.** Sequences of Protein of Immunological Interest. United States Public Health Service, National Institute of Health, 1991
- Computational Molecular Biology. Oxford University Press, 1988
- Biocomputing: Informatics and Genome Projects. Academic Press, 1993
- Computer Analysis of Sequence Data, Part 1. Humana Press, 1994
- **VON HEINJE, G.** Sequence Analysis in Molecular Biology. Academic Press, 1987
- Sequence Analysis Primer. M. Stockton Press, 1991
- **CARILLO et al.** *SIAM J. Applied Math.*, 1988, vol. 48, 1073
- **DEVEREUX et al.** *Nucl. Acid. Res.*, 1984, vol. 12, 387
- **ALTSCHUL et al.** *J. Mol. Biol.*, 1990, vol. 215, 403-410
- **ALTSCHUL et al.** BLAST Manual. NCB/NLM/NIH Bethesda
- **E. HARLOW ; D. LANE.** Antibodies: A Laboratory Manual.. Cold Spring Harbor Laboratory Press, 1988
- **GODING.** Monoclonal Antibodies: Principles and Practice. Academic Press, 1986, 59-103
- Antibody Purification Handbook. Biosciences
- **WARD et al.** *Nature*, 1989, vol. 341, 544

- **SAUNAL**. *J. Immunol. Methods*, 1995, vol. 183, 33-41
- **EHRING H**. *Analytical Biochemistry*, 1999, vol. 267 (2), 252-259
- **ENGEN, J. R.** ; **SMITH, D. L.** *Anal. Chem.*, 2001, vol. 73, 256A-265A
- **ERNST SCHERING**. *Res Found Workshop.*, 2004, vol. 44, 149-67
- **HUANG et al.** *Journal of Molecular Biology*, 1998, vol. 281 (1), 61-67
- **SAITO** ; **PATTERSON**. *Methods.*, June 1996, vol. 9 (3), 516-24
- **DOWNARD, J**. *Mass Spectrom.*, April 2000, vol. 35 (4), 493-503
- **KISELAR** ; **DOWNARD**. *Anal Chem.*, 01 May 1999, vol. 71 (9), 1792-1801
- **CLACKSON** ; **WELLS**. *Science*, 1995, vol. 267, 383-386
- **WELLS**. *Proc Natl Acad Sci USA*, 1996, vol. 93, 1-6
- **WANG et al.** *Nature*, 1992, vol. 355, 275-278
- **FÄGERSTAM et al.** *Journal Of Molecular Recognition*, 1990, vol. 3, 208-14
- **NICE et al.** *J. Chroma-togr.*, 1993, vol. 646, 159-168
- **LEIPERT et al.** *Angew. Chem. Int. Ed.*, 1998, vol. 37, 3308-3311
- **KRÖGER et al.** *Biosensors and Bioelectronics*, 2002, vol. 17, 937-944
- **SKERRA et al.** *Curr. Opinion in Immunol.*, 1993, vol. 5, 256
- **PLUCKTHUN**. *Immunol.*, 1992, vol. 130, 151
- **HUANG et al.** *AACR Annual meeting*, 2015

- **STROHL, W.** *Curr. Opin. Biotechnol.*, 2009, vol. 20 (6), 685-691
- **BAUDINO et al.** *J. Immunol.*, 2008, vol. 181, 6664-69
- **MORRISON et al.** *PNAS*, 1984, 6851
- **JONES et al.** *Nature*, 1986, vol. 321, 522
- **REICHMANN et al.** *Nature*, 1988, vol. 332, 323
- **PRESTA**. *Curr. Op. Struct. Biol.*, 1992, vol. 2, 593
- **VERHOEYEN**. *Science*, vol. 239, 1534
- **SIMS et al.** *J. Immunol.*, 1993, vol. 151, 2296
- **CHOTHIA** ; **LESK**. *J. Mol.*, 1987, vol. 196, 901
- **CARTER et al.** *PNAS*, 1992, vol. 89, 4285
- **PRESTA et al.** *J. Immunol.*, 1993, vol. 151, 2623
- **JAKOBOVITZ et al.** *Nature*, 1993, vol. 362, 255
- **REMINGTON**. The Science and Practice of Pharmacy. 1995
- **OKAZAKI et al.** *Curr. Opin. Immunol.*, 2002, vol. 14, 391779-82
- **BENNETT et al.** *J Immunol*, 2003, vol. 170, 711-8
- **FREEMAN et al.** *J Exp Med*, 2000, vol. 192, 1027-34
- **LATCHMAN et al.** *Nat Immunol*, 2001, vol. 2, 261-8
- **CARTER et al.** *Eur J Immunol*, 2002, vol. 32, 634-43
- **DONG et al.** *Nat. Med.*, 2002, vol. 8, 787-9
- **SACHSENMEIER et al.** *J Biomol Screening*, 2012